# EUROPEAN PATENT SPECIFICATION

(11) **EP 2 989 121 B1**
(45) Date of publication and mention of the grant of the patent: **07.10.2020**
(21) Application number: 14787690.8
(22) Date of filing: 28.04.2014
(51) Int. Cl.: C07K 14/075, A61K 38/04, A61P 37/02

(54) **METHODS OF PREPARATION AND COMPOSITION OF PEPTIDE CONSTRUCTS USEFUL FOR TREATMENT OF RHEUMATOID ARTHRITIS**
VERFAHREN ZUR HERSTELLUNG UND ZUSAMMENSETZUNG VON PEPTIDKONSTRUKTEN FÜR DIE BEHANDLUNG VON RHEUMATOIDER ARTHRITIS
MÉTHODES DE PRÉPARATION ET COMPOSITION DE CONSTRUCTIONS PEPTIDIQUES UTILES POUR LE TRAITEMENT DE LA POLYARTHRITE RHUMATOÏDE

(30) Priority: 26.04.2013 US 201361854554 P; 14.02.2014 US 201461966018 P
(43) Date of publication of application: 02.03.2016
(73) Proprietor: Cel-Sci Corporation, Vienna, VA 22182 (US); Zimmerman, Daniel, H., Bethesda, MD 20817 (US); Carambula, Roy, Owings Mills, MD 21117 (US); Steiner, Harold, Baltimore, MD 21215 (US); Talor, Eyal, Baltimore MD 21209 (US); Glant, Tibor, Chicago, IL 60610 (US); Mikecz, Katalin, Chicago, IL 60610 (US)
(72) Inventor: ZIMMERMAN, Daniel, H., Bethesda, MD 20817 (US); CARAMBULA, Roy, Owings Mills, MD 21117 (US); STEINER, Harold, Baltimore, MD 21215 (US); TALOR, Eyal, Baltimore, MD 21209 (US); GLANT, Tibor, Chicago, IL 60610 (US); MIKECZ, Katalin, Chicago, IL 60610 (US)
(74) Representative: Dehns
(86) International application number: PCT/US2014/035757
(87) International publication number: WO 2014/176604

(56) References cited:
- WO-A1-99/34827
- WO-A1-2010/120897
- WO-A1-2012/162564
- WO-A2-2009/114869
- WO-A2-2012/162565
- US-A1- 2004 013 676
- US-B1- 6 995 237
- KEN S. ROSENTHAL ET AL: "J-LEAPS peptide and LEAPS dendritic cell vaccines", MICROBIAL BIOTECHNOLOGY, vol. 5, no. 2, 6 September 2011 (2011-09-06), pages 203-213, XP055303324, GB ISSN: 1751-7915, DOI: 10.1111/j.1751-7915.2011.00278.x
- DANIEL H ZIMMERMAN: "LEAPS Therapeutic Vaccines as Antigen Specific Suppressors of Inflammation in Infectious and Autoimmune Diseases", JOURNAL OF VACCINES & VACCINATION, vol. 03, no. 05, 20 September 2012 (2012-09-20), XP055303198, DOI: 10.4172/2157-7560.1000149

### SEQUENCE LISTING

<110> Zimmerman, Daniel Carambula, Roy Steiner, Harold Talor, Eyal Glant, Tibor Mikecz, Katalin
<120> METHODS OF PREPARATION AND COMPOSITION OF PEPTIDE CONSTRUCTS USEFUL FOR TREATMENT OF RHEUMATOID ARTHRITIS
<130> CS-136/PROV2.3/PCT
<160> 30
<170> PatentIn ver. 3.5
<210> 1
   <211> 16
   <212> PRT
   <213> Homo Sapiens
<400> 1
   Gln Pro Ser Pro Leu Arg Val Leu Leu Gly Thr Ser Leu Thr Ile Pro
   1               5                   10                  15
<210> 2
   <211> 32
   <212> PRT
   <213> Artificial Sequence
<220>
   <223> Heteroconjugate Construct
<400> 2
   Asp Leu Leu Lys Asn Gly Glu Arg Ile Glu Lys Val Glu Gly Gly Gly
   1               5                   10                  15
   Gln Pro Ser Pro Leu Arg Val Leu Leu Gly Thr Ser Leu Thr Ile Pro
               20                  25                  30
<210> 3
   <211> 24
   <212> PRT
   <213> Homo Sapiens
<400> 3
   Gln Pro Ser Pro Leu Arg Val Leu Leu Gly Thr Ser Leu Thr Ile Pro
   1               5                   10                  15
   Cys Tyr Phe Ile Asp Pro Met His
               20
<210> 4
   <211> 40
   <212> PRT
   <213> Artificial Sequence
<220>
   <223> Heteroconjugate Construct
<400> 4
   Asp Leu Leu Lys Asn Gly Glu Arg Ile Glu Lys Val Glu Gly Gly Gly
   1               5                   10                  15
   Gln Pro Ser Pro Leu Arg Val Leu Leu Gly Thr Ser Leu Thr Ile Pro
               20                  25                  30
   Cys Tyr Phe Ile Asp Pro Met His
           35                  40
<210> 5
   <211> 15
   <212> PRT
   <213> Homo Sapiens
<400> 5
   Gly Thr Ser Leu Thr Ile Pro Cys Tyr Phe Ile Asp Pro Met His
   1               5                   10                  15
<210> 6
   <211> 30
   <212> PRT
   <213> Artificial Sequence
<220>
   <223> Heteroconjugate Construct
<400> 6
   Asp Leu Leu Lys Asn Gly Glu Arg Ile Glu Lys Val Glu Gly Gly Gly
   1               5                   10                  15
   Thr Ser Leu Thr Ile Pro Cys Tyr Phe Ile Asp Pro Met His
               20                  25                  30
<210> 7
   <211> 21
   <212> PRT
   <213> Homo Sapiens
<400> 7
   Gly Thr Ser Leu Thr Ile Pro Cys Tyr Phe Ile Asp Pro Met His Pro
   1               5                   10                  15
   Val Thr Thr Ala Pro
               20
<210> 8
   <211> 36
   <212> PRT
   <213> Artificial Sequence
<220>
   <223> Heteroconjugate Construct
<400> 8
   Asp Leu Leu Lys Asn Gly Glu Arg Ile Glu Lys Val Glu Gly Gly Gly
   1               5                   10                  15
   Thr Ser Leu Thr Ile Pro Cys Tyr Phe Ile Asp Pro Met His Pro Val
               20                  25                  30
   Thr Thr Ala Pro
           35
<210> 9
   <211> 15
   <212> PRT
   <213> Homo Sapiens
<400> 9
   Pro Leu Ala Pro Arg Ile Lys Trp Ser Arg Val Ser Lys Glu Lys
   1               5                   10                  15
<210> 10
   <211> 31
   <212> PRT
   <213> Artificial Sequence
<220>
   <223> Heteroconjugate Construct
<400> 10
   Asp Leu Leu Lys Asn Gly Glu Arg Ile Glu Lys Val Glu Gly Gly Gly
   1               5                   10                  15
   Pro Leu Ala Pro Arg Ile Lys Trp Ser Arg Val Ser Lys Glu Lys
               20                  25                  30
<210> 11
   <211> 21
   <212> PRT
   <213> Homo Sapiens
<400> 11
   Val Val Leu Leu Val Ala Thr Glu Gly Arg Val Arg Val Asn Ser Ala
   1               5                   10                  15
   Tyr Gln Asp Lys Val
               20
<210> 12
   <211> 37
   <212> PRT
   <213> Artificial Sequence
<220>
   <223> Heteroconjugate Construct
<400> 12
   Asp Leu Leu Lys Asn Gly Glu Arg Ile Glu Lys Val Glu Gly Gly Gly
   1               5                   10                  15
   Val Val Leu Leu Val Ala Thr Glu Gly Arg Val Arg Val Asn Ser Ala
               20                  25                  30
   Tyr Gln Asp Lys Val
           35
<210> 13
   <211> 15
   <212> PRT
   <213> Homo Sapiens
<400> 13
   Ala Thr Glu Gly Arg Val Arg Val Asn Ser Ala Tyr Gln Asp Lys
   1               5                   10                  15
<210> 14
   <211> 31
   <212> PRT
   <213> Artificial Sequence
<220>
   <223> Heteroconjugate Construct
<400> 14
   Asp Leu Leu Lys Asn Gly Glu Arg Ile Glu Lys Val Glu Gly Gly Gly
   1               5                   10                  15
   Ala Thr Glu Gly Arg Val Arg Val Asn Ser Ala Tyr Gln Asp Lys
               20                  25                  30
<210> 15
   <211> 15
   <212> PRT
   <213> Homo Sapiens
<400> 15
   Ala Cys Leu Gln Asn Ser Ala Ile Ile Ala Thr Pro Glu Gln Leu
   1               5                   10                  15
<210> 16
   <211> 31
   <212> PRT
   <213> Artificial Sequence
<220>
   <223> Heteroconjugate Construct
<400> 16
   Asp Leu Leu Lys Asn Gly Glu Arg Ile Glu Lys Val Glu Gly Gly Gly
   1               5                   10                  15
   Ala Cys Leu Gln Asn Ser Ala Ile Ile Ala Thr Pro Glu Gln Leu
               20                  25                  30
<210> 17
   <211> 13
   <212> PRT
   <213> Homo Sapiens
<400> 17
   Thr Arg Tyr Thr Leu Asp Phe Asp Arg Ala Gln Arg Ala
   1               5                   10
<210> 18
   <211> 29
   <212> PRT
   <213> Artificial Sequence
<220>
   <223> Heteroconjugate Construct
<400> 18
   Asp Leu Leu Lys Asn Gly Glu Arg Ile Glu Lys Val Glu Gly Gly Gly
   1 5 10 15
   Thr Arg Tyr Thr Leu Asp Phe Asp Arg Ala Gln Arg Ala
   20 25
<210> 19
   <211> 13
   <212> PRT
   <213> Homo Sapiens
<400> 19
   Ala Gly Trp Leu Ala Asp Gln Thr Val Arg Tyr Pro Ile
   1 5 10
<210> 20
   <211> 29
   <212> PRT
   <213> Artificial Sequence
<220>
   <223> Heteroconjugate Construct
<400> 20
   Asp Leu Leu Lys Asn Gly Glu Arg Ile Glu Lys Val Glu Gly Gly Gly
   1 5 10 15
   Ala Gly Trp Leu Ala Asp Gln Thr Val Arg Tyr Pro Ile
   20 25
<210> 21
   <211> 20
   <212> PRT
   <213> Homo Sapiens
<400> 21
   Thr Thr Gly His Val Tyr Leu Ala Trp Gln Ala Gly Met Asp Met Cys
   1 5 10 15
   Ser Ala Gly Trp
   20
<210> 22
   <211> 36
   <212> PRT
   <213> Artificial Sequence
<220>
   <223> Heteroconjugate Construct
<400> 22
   Asp Leu Leu Lys Asn Gly Glu Arg Ile Glu Lys Val Glu Gly Gly Gly
   1               5                   10                  15
   Thr Thr Gly His Val Tyr Leu Ala Trp Gln Ala Gly Met Asp Met Cys
               20                  25                  30
   Ser Ala Gly Trp
           35
<210> 23
   <211> 16
   <212> PRT
   <213> Homo Sapiens
<400> 23
   Gly His Val Tyr Leu Ala Trp Gln Ala Gly Met Asp Met Cys Ser Ala
   1               5                   10                  15
<210> 24
   <211> 32
   <212> PRT
   <213> Artificial Sequence
<220>
   <223> Heteroconjugate Construct
<400> 24
   Asp Leu Leu Lys Asn Gly Glu Arg Ile Glu Lys Val Glu Gly Gly Gly
   1               5                   10                  15
   Gly His Val Tyr Leu Ala Trp Gln Ala Gly Met Asp Met Cys Ser Ala
               20                  25                  30
<210> 25
   <211> 13
   <212> PRT
   <213> Homo Sapiens
<400> 25
   Ala Gly Trp Leu Ala Asp Arg Ser Val Arg Tyr Pro Ile
   1               5                   10
<210> 26
   <211> 29
   <212> PRT
   <213> Artificial Sequence
<220>
   <223> Heteroconjugate Construct
<400> 26
   Asp Leu Leu Lys Asn Gly Glu Arg Ile Glu Lys Val Glu Gly Gly Gly
   1               5                   10                  15
   Ala Gly Trp Leu Ala Asp Arg Ser Val Arg Tyr Pro Ile
               20                  25
<210> 27
   <211> 15
   <212> PRT
   <213> Homo Sapiens
<400> 27
   Thr Thr Tyr Lys Arg Arg Leu Gln Lys Arg Ser Ser Arg His Pro
   1               5                   10                  15
<210> 28
   <211> 31
   <212> PRT
   <213> Artificial Sequence
<220>
   <223> Heteroconjugate Construct
<400> 28
   Asp Leu Leu Lys Asn Gly Glu Arg Ile Glu Lys Val Glu Gly Gly Gly
   1               5                   10                  15
   Thr Thr Tyr Lys Arg Arg Leu Gln Lys Arg Ser Ser Arg His Pro
               20                  25                  30
<210> 29
   <211> 16
   <212> PRT
   <213> Homo Sapiens
<400> 29
   Leu Gln Lys Arg Ser Ser Arg His Pro Arg Arg Ser Arg Pro Ser Thr
   1               5                   10                  15
<210> 30
   <211> 32
   <212> PRT
   <213> Artificial Sequence
<220>
   <223> Heteroconjugate Construct
<400> 30
   Asp Leu Leu Lys Asn Gly Glu Arg Ile Glu Lys Val Glu Gly Gly Gly
   1 5 10 15
   Leu Gln Lys Arg Ser Ser Arg His Pro Arg Arg Ser Arg Pro Ser Thr
   20 25 30
<210> 31
   <211> 17
   <212> PRT
   <213> Homo Sapiens
<400> 31
   Val Leu Trp Lys Lys Gln Lys Asp Lys Val Ala Glu Leu Glu Asn Ser
   1 5 10 15
   Glu
<210> 32
   <211> 13
   <212> PRT
   <213> Homo Sapiens
<400> 32
   Asp Leu Leu Lys Asn Gly Glu Arg Ile Glu Lys Val Glu
   1 5 10
<210> 33
   <211> 11
   <212> PRT
   <213> Homo Sapiens
<400> 33
   Pro Ser Thr His Val Leu Ile Thr His Thr Ile
   1 5 10
<210> 34
   <211> 15
   <212> PRT
   <213> Homo Sapiens
<400> 34
   Asp Phe Leu Pro His Tyr Lys Asn Thr Ser Leu Gly His Arg Pro
   1 5 10 15
<210> 35
   <211> 15
   <212> PRT
   <213> Homo Sapiens
<400> 35
   Asn Gly Gln Glu Glu Lys Ala Gly Val Val Ser Thr Gly Leu Ile
   1 5 10 15
<210> 36
   <211> 15
   <212> PRT
   <213> Homo Sapiens
<400> 36
   Asp Gly Gln Glu Glu Lys Ala Gly Val Val Ser Thr Gly Leu Ile
   1 5 10 15
<210> 37
   <211> 31
   <212> PRT
   <213> Homo Sapiens
<400> 37
   Asp Asn Gln Leu Leu Glu Thr Cys Lys Gln Asp Arg Leu Arg Asn Arg
   1 5 10 15
   Arg Gly Asn Gly Ser Ser Thr His Phe Glu Gly Asn Leu Pro Cys
   20 25 30
<210> 38
   <211> 9
   <212> PRT
   <213> Homo Sapiens
<400> 38
   Val Gln Gly Glu Glu Ser Asn Asp Lys
   1 5
<210> 39
   <211> 10
   <212> PRT
   <213> Homo Sapiens
<400> 39
   Asp Gln Thr Gln Asp Thr Glu Gly Gly Cys
   1 5 10
<210> 40
   <211> 8
   <212> PRT
   <213> Artificial Sequence
<220>
   <223> Variable Linker
<400> 40
   Leu Arg Gly Gly Gly Gly Ser Ser
   1 5
<210> 41
   <211> 4
   <212> PRT
   <213> Artificial Sequence
<220>
   <223> Variable Linker
<400> 41
   Gly Gly Gly Ser
   1
<210> 42
   <211> 5
   <212> PRT
   <213> Artificial Sequence
<220>
   <223> Variable Linker
<400> 42
   Gly Gly Gly Ser Ser
   1               5
<210> 43
   <211> 5
   <212> PRT
   <213> Artificial Sequence
<220>
   <223> Variable Linker
<400> 43
   Gly Gly Gly Gly Ser
   1               5
<210> 44
   <211> 6
   <212> PRT
   <213> Artificial Sequence
<220>
   <223> Variable Linker
<400> 44
   Gly Gly Gly Gly Ser Ser
   1               5
<210> 45
   <211> 8
   <212> PRT
   <213> Artificial Sequence
<220>
   <223> Variable Linker
<400> 45
   Gly Gly Gly Ser Gly Gly Gly Ser
   1               5
<210> 46
   <211> 33
   <212> PRT
   <213> Artificial Sequence
<220>
   <223> Heteroconjugate Construct
<400> 46
   Asp Gly Gln Glu Glu Lys Ala Gly Val Val Ser Thr Gly Leu Ile Gly
   1               5                   10                  15
   Gly Gly Ala Thr Glu Gly Arg Val Arg Val Asn Ser Ala Tyr Gln Asp
               20                  25                  30
   Lys
<210> 47
   <211> 36
   <212> PRT
   <213> Artificial Sequence
<220>
   <223> Heteroconjugate Construct
<400> 47
   Asp Leu Leu Lys Asn Gly Glu Arg Ile Glu Lys Val Glu Gly Gly Gly
   1               5                   10                  15
   Thr Gly Gly Lys Pro Gly Ile Ala Gly Phe Lys Gly Glu Gln Gly Pro
               20                  25                  30
   Lys Gly Glu Pro
           35
<210> 48
   <211> 46
   <212> PRT
   <213> Artificial Sequence
<220>
   <223> Heteroconjugate Construct
<400> 48
   Asp Leu Leu Lys Asn Gly Glu Arg Ile Glu Lys Val Glu Gly Gly Gly
   1               5                   10                  15
   Tyr Ser Val His Gln Arg Ile Asp Val Lys Asp Thr Lys Glu Ala Leu
               20                  25                  30
   Glu Lys Ile Glu Glu Glu Gln Asn Lys Ser Lys Lys Lys Ala
           35                  40                  45
<210> 49
   <211> 9
   <212> PRT
   <213> Homo Sapiens
<400> 49
   Ala Tyr Met Thr Met Lys Ile Arg Asn
   1               5
<210> 50
   <211> 20
   <212> PRT
   <213> Homo Sapiens
<400> 50
   Thr Gly Gly Lys Pro Gly Ile Ala Gly Phe Lys Gly Glu Gln Gly Pro
                   5                   10                  15
   Lys Gly Glu Pro
               20
<210> 51
   <211> 31
   <212> PRT
   <213> Artificial Sequence
<220>
   <223> Heteroconjugate Construct
<400> 51
<210> 52
   <211> 36
   <212> PRT
   <213> Artificial Sequence
<220>
   <223> Heteroconjugate Construct
<400> 52
<210> 53
   <211> 20
   <212> PRT
   <213> Homo Sapiens
<400> 53
   Pro Gly Leu Pro Gly Ala Arg Gly Leu Thr Gly Arg Pro Gly Asp Ala
                   5                   10                  15
   Gly Pro Gln Gly
               20
<210> 54
   <211> 36
   <212> PRT
   <213> Artificial Sequence
<220>
   <223> Heteroconjugate Construct
<400> 54
   Asp Leu Leu Lys Asn Gly Glu Arg Ile Glu Lys Val Glu Gly Gly Gly
   1               5                   10                  15
   Pro Gly Lys Pro Gly Ala Arg Gly Leu Thr Gly Arg Pro Gly Asp Ala
               20                  25                  30
   Gly Pro Gln Gly
           35
<210> 55
   <211> 36
   <212> PRT
   <213> Artificial Sequence
<220>
   <223> Heteroconjugate Construct
<400> 55
<210> 56
   <211> 36
   <212> PRT
   <213> Homo Sapiens
<400> 56
   Thr Gly Gly Lys Pro Gly Ile Ala Gly Phe Lys Gly Glu Gln Gly Pro
   5 10 15
   Lys Gly Glu Pro Gly Gly Gly Glu Val Lys Glu Ile Arg Glu Gly Asn
   20 25 30
   Lys Leu Leu Asp
   35
<210> 57
   <211> 36
   <212> PRT
   <213> Homo Sapiens
<400> 57
   Pro Glu Gly Lys Pro Gly Gln Glu Gly Lys Phe Gly Ala Ile Gly Pro
   5 10 15
   Lys Gly Gly Thr Gly Gly Gly Glu Val Lys Glu Ile Arg Glu Gly Asn
   20 25 30
   Lys Leu Leu Asp
   35
<210> 58
   <211> 36
   <212> PRT
   <213> Homo Sapiens
<400> 58
   Thr Gly Gly Lys Pro Gly Ile Ala Gly Phe Lys Gly Glu Gln Gly Pro
   5 10 15
   Lys Gly Glu Pro Gly Gly Gly Asp Leu Leu Lys Asn Gly Glu Arg Ile
   20 25 30
   Glu Lys Val Glu
   35
<210> 59
   <211> 36
   <212> PRT
   <213> Homo Sapiens
<400> 59
   Pro Glu Gly Lys Pro Gly Gln Glu Gly Lys Phe Gly Ala Ile Gly Pro
                   5                   10                  15
   Lys Gly Gly Thr Gly Gly Gly Asp Leu Leu Lys Asn Gly Glu Arg Ile
               20                  25                  30
   Glu Lys Val Glu
           35

## Description

### BACKGROUND

Autoimmune conditions are diseases where the body is attacked by its own immune system. This occurs when the adaptive immune system mistakenly identifies molecular aspects of the body as disease antigens. Without being limited to any particular theory, one hypothesis is that autoimmunity develops due to molecular mimicry by certain pathogens. In other words, the pathogens produce antigens that closely resemble certain molecules produced normally by the body. This causes the adaptive immune system to build a memory of these molecules, which is then mistakenly triggered by the body's own, closely mimicking molecules. The innate immune system may also play a role in autoimmunity. Activity of the immune system, especially when chronic, is generally destructive to the body's tissues, which is why autoimmunity is problematic (*see also* Bachmann & Kopf on the role of the innate immunity in autoimmune disease, J. Exp. Med. 193: F47-50(2001)). The goal of most current treatments for autoimmune conditions is to turn off, dampen or redirect the inappropriate immune response regardless of what caused the response (*i.e.* not in an antigen-specific manner). By contrast, the goal of a vaccination approach is to eliminate, reduce or redirect an immune response against self-antigens in an antigen-specific manner. As noted by an Institutes of Medicine report, autoimmune diseases are a major target area proposed for vaccines and are the third major area for expenditure of healthcare in the US behind cancer and cardiovascular diseases.

One of the primary target organs in rheumatoid arthritis (RA) and osteoarthritis is the synovial joints where an inflammatory response takes place against different components of cartilage (de Jong H et al., Cartilage proteoglycan aggrecan epitopes induce proinflammatory autoreactive T-cell responses in rheumatoid arthritis and osteoarthritis 2010, Ann Rheum Dis. 69:255-262). Candidate epitopes in antigens include cartilage proteins, among others, such as collagens, human cartilage glycoprotein-39 (HC-gp39), link protein, and human cartilage proteoglycan (PG) (specifically the protein called aggrecan), several of which might be an autoimmune target especially when treated to remove keratin and chondrotin sulfate blocking chains resulting in the revealing of cryptic or hidden epitopes.

Various antigens, often with defined epitopes recognized by some human leukocyte antigens (HLA) genotypes, have been identified, including those associated with insulin dependent diabetes mellitis (IDDM, also called type I diabetes) and rheumatoid arthritis (RA). Examples of such antigens are proteoglycan aggrecan (PG), in particular the G1 subunit, and specifically the peptide p70-84 (p70-84, which is a peptide starting at position 70 and encompassing residues 70-84 (often abbreviated as p70), amino acids represented as ATEGRVRVNSAYQDK (SEQ ID NO. 13) or other G1 epitopes recognized by cells or circulating antibodies in human patients or murine models (Murad YM et al., Molecular manipulation with the arthritogenic epitopes of the G1 domain of human cartilage proteoglycan aggrecan 2005, Clin. And Exp. Imm. 142: 303-311).

Many of these autoimmune conditions are characterized by elevated levels of one or more different cytokines and other effectors, such as TNFα. Current research focuses on ways to target and attack cells or cellular products of the immune system found in altered amounts in autoimmune diseases and thereby treat autoimmune conditions. Reagents for this include presumptive antigenic peptides or proteins, peptides representing certain T cells, monoclonal antibodies (mAbs) or recombinant proteins binding various effector cells or molecules such as TNFα and IgE (Kleinau S et al., Importance of CD23 for Collagen-Induced Arthritis: Delayed Onset and Reduced Severity in CD23-Deficient Mice 1999, J. Immunol. 162:4266; Preckel T et al., Partial agonism and independent modulation of T cell receptor and CD8 in hapten-specific cytotoxic T cells 1998, Eur. J. Immunol., 28:3706; Wooley PH et al., Influence of a recombinant human soluble tumor necrosis factor receptor FC fusion protein on type II collagen-induced arthritis in mice 1993, J. Immunol., 151:6602).

It should be noted, however, that many of the current approaches, agents or targets (cytokines, cytokine receptor agonists or cell surface markers) are not disease-specific and often recognize and could disadvantageously affect normal cellular and body constituents that have a defined and necessary role in needed normal immune defenses.

One example of an antigen disease-specific approach is to treat multiple sclerosis (MS) patients by oral administration of myelin proteins. These treatments are designed to target the Gut Associated Lymphoid Tissues (GALT) to induce tolerance by antigen-specific suppression of the immune system. It is not known if these treatments would use the intact protein or a hydrolyzate containing smaller peptides (Wucherpfennig KW et al., Shared human T cell receptor V beta usage to immunodominant regions of myelin basic protein. 1990, Science, 248:1016; Ota K et al., T-cell recognition of an immunodominant myelin basic protein epitope in multiple sclerosis 1990, Nature, 346: 183). Collagen type II can also be used for treatment of patients with rheumatoid arthritis.

A related approach to treating autoimmune conditions is the use of an oral formulation of peptide(s) as immunogen given in large quantities. The peptide represents a sequence that is thought to be the autoimmune epitope itself or a modified form which may have altered binding or improved stability properties. By use of the peptide it is believed that either the normal peptide or an Altered Peptide Ligand (APL) will bind to the T cell receptor (TCR) and induce a state of anergy with an antigen presenting cell (APC) (Faith A et al., An Altered Peptide Ligand Specifically Inhibits Th2 Cytokine Synthesis by Abrogating TCR Signaling 1999, J. Immunol., 162:1836; Soares LRB et al., Differential Activation of T Cells by Natural Antigen Peptide Analogues: Influence on Autoimmune and Alloimmune In vivo T Cell Responses 1998, J. Immunol., 160:4768; Croft M et al., Partial activation of naive CD4 T cells and tolerance induction in response to peptide presented by resting B cells 1997, J. Immunol., 159:3257; Ding L et al., Differential Effects of CD28 Engagement and IL-12 on T Cell Activation by Altered Peptide Ligands 1998, J. Immunol., 161:6614; Hin S. et al., Cutting Edge: N-Hydroxy Peptides: A New Class of TCR Antagonists 1999, J. Immunol., 163:2363). Some of the other approaches involving APL include using related amino acids such a D amino acids (Koch U et al., A Synthetic CD4-CDR3 Peptide Analog Enhances Skin Allograft Survival Across a MHC Class II Barrier 1998, J. Immunol., 161:421), amino acids with substituted side chains (De Palma R et al., Use of Antagonist Peptides to Inhibit In vitro T Cell Responses to Par j1, the Major Allergen of Parietaria judaica Pollen 1999, J. Immunol., 162:1982), methylene groups to replace peptide bonds in the peptide backbone (Meda L et al., Beta-amyloid (25-35) peptide and IFNγ synergistically induce the production of the chemotactic cytokine MCP-1/JE in monocytes and microglial cells 1996, J. Immunol., 157:1213) and N-hydroxyl peptides (Hin S *et al.,* 1999 *supra*).

Various substitutions of side chains of the Major Histocompatibility Complex (MHC) and T cell receptor (TCR) molecules have also been contemplated by the prior art (Clay TM et al., Changes in the Fine Specificity of gp100(209-217)-Reactive T Cells in Patients Following Vaccination with a Peptide Modified at an HLA-A2.1 Anchor Residue 1999, J. Imunol., 162:1749). For example, with insulin activity it has been shown that a one-amino acid change on the α-chain can abolish the oral immune tolerance activity in two (2) mechanistically different IDDM murine models (Homann D et al., Insulin in Oral Immune "Tolerance": A One-Amino Acid Change in the B Chain Makes the Difference 1999, J. Immunol., 163:1833). Although not an autoimmune epitope, a single change from threonine to alanine can abolish biological activity (Sutherland CL et al., An 11-Amino Acid Sequence in the Cytoplasmic Domain of CD40 Is Sufficient for Activation of c-Jun N-Terminal Kinase, Activation of MAPKAP Kinase-2, Phosphorylation of I B , and Protection of WEHI-231 Cells from Anti-IgM-Induced Growth Arrest 1999, J. Immunol., 162:4720) while a switch from phenylalanine to alanine converts bee venom phospholipase from an active form to an inactive form (Faith A *et al.,* 1999 *supra*), and a switch from tyrosine to alanine converts from active to inactive for another system (Hausman et al., Peptide Recognition by Two HLA-A2/Tax11-19-Specific T Cell Clones in Relationship to Their MHC/Peptide/TCR Crystal Structures 1999, J. Immunol., 162:5389).

In yet another approach based on peptide materials, truncated peptides of autoimmune-inducing epitope are used as an antagonist in an animal model to treat a particular condition (Karin N et al., Short Peptide-Based Tolerogens Without Self-Antigenic or Pathogenic Activity Reverse Autoimmune Disease 1999, J. Immunol., 160:5188). Synthetic amino acid polymers that are thought to represent epitopes which contain tyrosine (Y), glutamic acid (E), alanine (A) and lysine (K) to target T cells such as copolymer 1 have been contemplated. For example, Copaxone® has been used as an oral tolerance delivery approach to treat MS patients where Copaxone® is believed to be a synthetic copolymer of four amino acids (Hafler DA et al., Anti-CD4 and anti-CD2 monoclonal antibody infusions in subjects with multiple sclerosis. Immunosuppressive effects and human anti-mouse responses 1998, J. Immunol. 141:131). Modified peptides of peptide epitopes are also being studied for treatment of various autoimmune conditions including MS and PV (desmoglein-3) (Hammer J et al., HLA class II peptide binding specificity and autoimmunity 1997, Adv. Immunol., 66:67; Wucherpfennig KW et al., Structural Basis for Major Histocompatibility Complex (MHC)-Linked Susceptibility to Autoimmunity: Charged Residues of a Single MHC Binding Pocket Confer Selective Presentation of Self-Peptides in Pemphigus Vulgaris 1995, PNAS, 92:11935). The use of myelin proteolipid associated peptide epitope, a polymer or derivative of this epitope for MS has been further contemplated (Hammer J *et al.,* 1997 *supra*).

In other approaches, peptides that are unique to the T cell antigen receptor molecule have been contemplated for a psoriasis vaccine, such as IR 502, while others have been contemplated for rheumatoid arthritis. These peptides are found in a particular part of the variable region, usually the third hyper-variable region of the beta chain of the T cell antigen receptor (TCR□V3) (Kotzin BL et al., Preferential T-Cell Receptor β-Chain Variable Gene Use in Myelin Basic Protein-Reactive T-Cell Clones from Patients with Multiple Sclerosis 1991, Proc Nat Acad Sci US, 88:9161; Oksenberg JR et al., Limited heterogeneity of rearranged T-cell receptor V alpha (TCRaV3) transcripts in brains of multiple sclerosis patients 1990, Nature, 345:344). For example, in an immune response to TCRaV3 (Sutherland CL *et al.,* 1999 *supra*) a peptide is generated whose objective is to eliminate the particular T cells responsible for the condition, thus treating the condition. However, this approach has the disadvantageous potential of eliminating other T cells that contain the same αV3 peptide sequence, but which are not responsible for the autoimmune condition.

Still another peptide approach uses complimentary peptide vaccine that induces T cell anergy and prevents experimental autoimmune encephalomyelitis (EAE) in rats by induction of anti-TCR antibodies (similar to anti-idiotype antibodies) and thereby eliminate these cells (Araga S et al., A complementary peptide vaccine that induces T cell anergy and prevents experimental allergic neuritis in Lewis rats 1999, J. Immunol., 163:476).

Small peptides are usually of insufficient size and structure to be antigen-specific and induce an immune response unless altered, and/or conjugated or otherwise made more antigenic. Incorporation into viral proteins has been attempted to address this issue, but this approach suffers from several concerns. First, some viruses are suspected to be causative agents for some autoimmune conditions such as RA, type 1 diabetes, multiple sclerosis (MS), myocarditis, and Graves' disease. Second, it is also known that many viral proteins, including some from human immunodeficiency virus (HIV) and herpes simplex virus (HSV) contain immunosuppressive epitopes.

Similarly, virus-like particles (VLP) suffer from these same disadvantages. These particles incorporate antigenic epitope(s) of the plant, animal or bacterial virus and contain the foreign antigen of interest, *e.g.* in a fusion protein.

Additionally, incorporating genetic materials into a gene for viral proteins, self-heat shock proteins, or autoimmune-related epitopes has been attempted, but similarly presents the disadvantages described above. Another disadvantage in using a DNA-based vaccine for treating autoimmune conditions is the possibility of the vaccine DNA being integrated into the host's genome in 1) an uncontrolled manner, 2 at undesirable sites, 3) in multiple copy numbers, 4) under different regulatory control than desired. Accordingly, an alternative is to conjugate a particular DNA-based epitope to a carrier protein to avoid such incorporation into the genome. It is known within the art to use large carrier proteins such as keyhole limpet hemocyanin (KLH), bovine serum albumin (BSA), or Antigenics Inc.'s heat shock proteins (HSP) conjugated or incorporated with a virus protein.

Other options for delivery of peptide epitopes include the use of synthetic biodegradable microparticles like poly(lactide-co-glycolide) (PLG) with aggregated antigen, Auto Vac™ of Pharmexa, and phage display and multiple antigen presentation (MAPS) technologies (Rosenthal K, Immune peptide enhancement of peptide based vaccines 2005, Frontiers in Bioscience 1:478-482).

WO 2012/162564 discloses peptide constructs, i.e., polypeptides obtained by linking together two or more peptides based on or derived from different molecules, which are useful in the treatment or prevention of influenza virus and other infectious diseases. Compositions containing the same, methods for producing the same, and methods for using the same are also disclosed, wherein the peptide constructs have the formula P1-x-P2, where P2 is a peptide associated with an infectious agent and P1 is a peptide that will bind to a class of immune cells, such as dendritic cells. The peptide construct can cause the maturation of immature dendritic cells to a more mature state. The peptide construct or the more mature dendritic cell can be administered to a subject to modulate or initiate an immune response against an infectious agent. Dyes, radioisotopes, or therapeutic agents conjugated with the dendritic cells can be used for localization of the immune target and/or prophylactic or therapeutic treatment of the disease.

WO 2012/162565 discloses peptide constructs, i.e., polypeptides obtained by linking together two or more peptides based on or derived from different molecules, which are useful in the treatment or prevention of cancer or the treatment of autoimmune diseases and compositions containing same, methods for producing same, and methods for using same; wherein the peptide constructs have the formula Pi-x-P2 where P2 is a peptide associated with forms of cancer or an autoimmune condition and Pi is a peptide which will bind to a class of immune cells such as dendritic cells. The peptide construct can cause the maturation of immature dendritic cells to a more mature state. The peptide construct or the more mature dendritic cells can be administered to a subject to modulate or to initiate an immune response against cancer cells, and can be used with dyes, radioisotopes, or therapeutic agents for detection of the immune target and/or treatment of cancer and autoimmune conditions.

As noted above, however, many of the known approaches present the significant disadvantage of using large, very immunogenic carriers. Moreover, patient populations requiring such therapeutics have usually been exposed to many of these same antigens during their lifetimes. Hence, and similar to vaccine vector delivery of antigens, clearance of the antigen can be so vigorous in the previously exposed host that no response will occur to the new antigen. On the other hand, a strong immune response may occur upon reintroduction of the vector. For example, in the case of the conjugate VP22 containing HSV-1 protein, the response may be undesirable given that a majority of adults have had one or more exposures to HSV-1 (Muranyiova M et al., Immunoprecipitation of herpes simplex virus polypeptides with human sera is related to their ELISA titre 1999, Acta Virol. 35:252-9).

Hence, there is a need for compositions and methods directed to a therapeutic peptide that is a small peptide wherein the peptide can be used as a potential antigen. There is a need for approaches to treating autoimmune diseases with immunotherapeutics requiring more specific and lower doses of an active substance and less frequent dosing. The therapeutics should also allow for the option of subcutaneous, oral intradermal, intranasal or transdermal administration, wherein the active portion of the therapeutics should be comprised of substantially smaller molecules (3-5kDa) that are more likely to be able to cross the blood brain barrier.

Moreover, the need extends from treatments that do not require monoclonal antibodies or receptor agonists, to molecules that are critical to maintaining normal body function, such as TNFα soluble receptor or those that require "humanization."

There is further a need for therapeutics that do not eliminate essential or necessary T cells other than those that cause the autoimmune disease. The therapeutics should only act upon the major abnormal molecule or cell while preventing a high response against the vector or delivery molecule.

There is further a need for a therapeutic that is not integrated into the host's genome wherein the therapeutic may alter the sequence, expression or activation of other normal or oncogenes.

There is still a further need for providing dendritic cells that are isolated from the host and L.E.A.P.S.-activated with specific antigenic peptides *ex vivo,* before being used as a therapeutic or diagnostic agent for autologous cell therapy or diagnostic localization.

There is still further a need for providing specific antigenic peptides associated with disease or the causative organism that can be linked with a T cell binding ligand such as L.E.A.P.S. Moreover, the therapeutics should be manufactured and be cost effective in their production and stable during storage before use.

### BRIEF DESCRIPTION OF THE DRAWINGS

FIG. 1 describes the effects of PG peptides and L.E.A.P.S.-PG peptide conjugates on DC maturation *in vitro.*
FIG. 2 describes the effects of PG peptides and L.E.A.P.S.-PG peptide conjugates on DC IL-12 expression.
FIG. 3 shows the effects of PG peptides and L.E.A.P.S.-PG peptide conjugates on T cell proliferation or Th1:Th2 ratio.
FIG. 4 shows the L.E.A.P.S. peptide vaccinated PGIA mice.
FIG. 5 shows the L.E.A.P.S. peptide vaccinated PGIA mice Histology of ankle joints and shows representative sections of the same groups as in FIG. 4.
FIG. 6 shows the L.E.A.P.S. peptide vaccinated PGIA mice: Effect on PG-specific spleen cell proliferation (*in vitro* assay).
FIG. 7 describes L.E.A.P.S. peptide vaccinated PGIA mice: Effect on secreted cytokines after PG stimulation for the same groups as in FIG. 4.
FIG. 8 describes L.E.A.P.S. peptide vaccinated PGIA mice - Th1- Th2-, Th17-specific intracellular cytokines & regulatory T cells (Tregs) for the same groups as in FIG. 4.

### BRIEF SUMMARY OF THE INVENTION

The present invention provides peptides for use in modulating an autoimmune response in a subject or for maturing dendritic cells, and or activating T cells; matured dendritic cell populations, activated T cell populations; compositions comprising such populations; and ex vivo methods for producing such populations as defined in the appended claims. The invention is related to peptide constructs, *i.e.,* polypeptides obtained by linking together two or more peptides based on or derived from different molecules, which are useful in the treatment or prevention of autoimmune diseases, particularly rheumatoid arthritis, as well as to compositions containing the same, methods for producing the same and methods for using the same.

The invention generally relates to methods and compositions for using these peptide constructs to induce or redirect to a favorable immune response and thereby treat autoimmune conditions such as rheumatoid arthritis or osteoarthritis in a subject.

The invention generally relates to methods and compositions for using these peptide constructs for activating and promoting the maturation of immature dendritic cells or monocytes into matured dendritic cells (DCs), and/or activating or modulating T cells, and eliciting favorable properties in the matured dendritic cells, and/or T cells, for treatment and diagnosis of an autoimmune condition, such as rheumatoid arthritis or osteoarthritis.

The invention generally relates to methods and compositions for using these peptide constructs to activate and promote the maturation of immature dendritic cells or monocytes into matured dendritic cells (DCs), and/or activate or modulate T cells, thus eliciting favorable properties in the matured dendritic cells and/or T cells, and labeling or conjugating these cells with drugs, dyes or radioactive isotopes for treatment and diagnosis of an autoimmune condition such as rheumatoid arthritis or osteoarthritis either directly or indirectly by use of monoclonal antibodies or other cell surface-binding agents.

The present invention relates to a peptide for use in modulating an autoimmune response in a subject or for maturing dendritic cells, and or activating T cells, comprising a peptide construct selected from the group consisting of SEQ ID No.'s 2, 4, 6, 8, 10, 12, 14, 16, 18, 20, 22, 24, 26, 28, 30 and 46.

In any embodiment, the peptide construct is selected from the group consisting of SEQ ID No.'s 2, 4, 6, 8, 10, 12, 14, 16, 18, 20, 22, 24, 26, 28, 30 and 46.

In any embodiment, the peptide construct is composed of two or more peptide constructs selected from the group consisting of SEQ ID No.'s 2, 4, 6, 8, 10, 12, 14, 16, 18, 20, 22, 24, 26, 28, 30 and 46.

Disclosed is a peptide for modulating an autoimmune response in a subject or for maturing dendritic cells, and or activating T cells, comprising a peptide construct having the formula P₁-x-P₂ or P₂-x-P₁, wherein
P₂ represents a specific antigenic peptide competent for recognition by a class or subclass of immune cells or binding to an antibody;
P₁ represents an immunomodulatory peptide which is a portion of an
   immunoprotein capable of promoting binding to a class or subclass of immune cells and modulating a subsequent immune response to the peptide P₂; and
x represents a covalent bond or a divalent linking group.

In any embodiment, the peptide P₂ is one sequence selected from the group consisting of SEQ ID No.'s 1, 3, 5, 7, 9, 11, 13, 15, 17, 19, 21, 23, 25, 27, 29, and variants thereof.

In any embodiment, the peptide P₁ is selected from the group consisting of SEQ ID No.'s 31-39, and variants thereof.

In any embodiment, the peptide P₁ is SEQ ID No. 32.

In any embodiment, the peptide P₂ is a reversal sequence of a sequence selected from the group consisting of SEQ ID No.'s 1, 3, 5, 7, 9, 11, 13, 15, 17, 19, 21, 23, 25, 27, 29, 30, and variants thereof.

In any embodiment, the peptide P₁ is a reversal sequence of a sequence selected from the group consisting of SEQ ID No.'s 31-39 and variants thereof.

In any embodiment, the peptide P₁ is the reversal sequence of SEQ ID No. 32.

In any embodiment, the divalent linker comprises one or more glycine residues.

In any embodiment, the divalent linker is not a peptide.

In any embodiment, the peptide P₂ is one sequence selected from the group consisting of SEQ ID No.'s 1, 3, 5, 7, 9, 11, 13, 15, 17, 19, 21, 23, 25, 27, 29, and variants thereof.

In one embodiment, the peptide P₁ is selected from the group consisting of SEQ ID No.'s 31-39 and variants thereof.

In any embodiment, the peptide P₁ is SEQ ID No. 32.

In any embodiment, the peptide P₂ is a reversal sequence of a sequence selected from the group consisting of SEQ ID No.'s 1, 3, 5, 7, 9, 11, 13, 15, 17, 19, 21, 23, 25, 27, 29, and variants thereof.

In any embodiment, the peptide P₁ is a reversal sequence of a sequence selected from the group consisting of SEQ ID No.'s 31-39 and variants thereof.

In any embodiment, the peptide P₁ is the reversal sequence of SEQ ID No. 32.

In any embodiment, the peptide P₂ is an allowed variant sequence using the allowed substitutions X₁-X₁₇ as taught by the algorithm taught in Table 2 of a sequence selected from the group consisting of SEQ ID No.'s 1, 3, 5, 7, 9, 11, 13, 15, 17, 19, 21, 23, 25, 27, 29, and variants thereof.

In any embodiment, the peptide P₁ using the allowed substitutions X₁-X₁₇ as taught by the algorithm taught in Table 2 of a sequence selected from the group consisting of SEQ ID No.'s 31-39 and variants thereof.

In any embodiment, the peptide P₁ is the using the allowed substitutions X₁-X₁₅ as taught by the algorithm taught in Table 2 sequence of SEQ ID No. 32.

In any embodiment, the matured dendritic cells are isolated away from bone marrow or blood tissues.

In any embodiment, the composition is substantially free of red blood cells.

In any embodiment, the immature dendritic cells or monocytes are derived from one or more selected from the group consisting of blood derived monocytes and bone marrow cells.

In any embodiment, the divalent linker comprises one or more glycine residues.

In any embodiment, the divalent linker is not a peptide.

In any embodiment, the matured dendritic cells are conjugated to a radioisotope.

In any embodiment, the radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In any embodiment, the radioisotope is conjugated to the matured dendritic cells with an antibody.

In any embodiment, the matured dendritic cells are conjugated to a therapeutic agent.

In any embodiment, the therapeutic agent is one or more of a drug selected from the group consisting of auristatin, MMAE, ozogamicin, emtansine, and gelonin; a toxin selected from the group consisting of SEB superantigen or Saporin; or a cytokine.

In any embodiment, the cytokine is interferon-a.

In any embodiment, the matured dendritic cells are conjugated to the therapeutic agent with an antibody.

In any embodiment, the antibody has affinity for MHC II, CD11c, DEC-205, Dectin-1, DC-SIGN, or DC-LAMP.

In any embodiment, the therapeutic agent is conjugated to a lysosomotropic agent.

In any embodiment, the lysosomotropic agent is conjugated to the therapeutic agent through a cleavable linker.

In any embodiment, the therapeutic agent is selected from the group consisting of a mictrotubule inhibitor, an antimitotic agent, a maytansinoid, a receptor tyrosine kinase inhibitor, and phosphinositide 3-kinase inhibitor.

In any embodiment, the therapeutic agent is selected from the group consisting of monomethyl auristatin E, ozogamicin, auristatin, carboplatin, 5-fluorouracil, docetaxel, maytansinoid 1 nomifensine, doxorubicin, irinotecan, interferon-a, staphylococcal enterotoxin A superantigen, and staphylococcal enterotoxin B superantigen.

The present invention also relates to a composition comprising a population of matured dendritic cells, the population of matured dendritic cells formed by contacting immature dendritic cells or monocytes with an effective amount of a peptide construct selected from the group consisting of SEQ ID No.'s 2, 4, 6, 8, 10, 12, 14, 16, 18, 20, 22, 24, 26, 28 and 30 under conditions suitable for maturation of the dendritic cells or monocytes.

The present invention further relates to a composition comprising a population of matured dendritic cells, the population of matured dendritic cells formed by contacting immature dendritic cells or monocytes with an effective amount of a peptide mixture of two or more peptide constructs selected from the group consisting of SEQ ID No.'s 2, 4, 6, 8, 10 ,12, 14, 16, 18, 20, 22, 24, 26, 28 and 30 and variants thereof under conditions suitable for maturation of the dendritic cells or monocytes.

In any embodiment, the population of matured dendritic cells produces an increased amount of Interleukin 12p70 (IL-12p70) compared to immature dendritic cells or monocytes not contacted with the peptide construct.

In any embodiment, the immature dendritic cells or monocytes originate from blood derived monocytes or cells derived from bone marrow cells.

In any embodiment, the matured dendritic cells are conjugated to a radioisotope.

In any embodiment, the radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In any embodiment, the radioisotope is conjugated to the matured dendritic cells with an antibody.

In any embodiment, the antibody has affinity for MHC II, CD11c, DEC-205, Dectin-1, DC-SIGN, or DC-LAMP.

In any embodiment, the matured dendritic cells are conjugated to a therapeutic agent.

In any embodiment, the therapeutic agent is one or more of a drug selected from the group consisting of auristatin, MMAE, ozogamicin, emtansine, and gelonin; a toxin selected from the group consisting of SEB superantigen or Saporin; or a cytokine.

Disclosed is a method for producing a matured dendritic cell population, comprising contacting or treating immature dendritic cells or monocytes with an effective amount of a peptide construct having the formula P₁-X-P₂ or P₂-X-P₁ under conditions suitable for maturation of dendritic cells or monocytes to form matured dendritic cells, wherein
P₂ represents a specific antigenic peptide competent for recognition by a class or subclass of immune cells or binding to an antibody;
P₁ represents an immunomodulatory peptide which is a portion of an
   immunoprotein capable of promoting binding to a class or subclass of dendritic cells; and
x represents a covalent bond or a divalent peptide linking group.

In one embodiment, the matured dendritic cells produce an immunomodulatory effect to an autoimmune disease when administered to a patient.

In one embodiment, the matured dendritic cells produce an increased amount of Interleukin 12p70 (IL-12p70) compared to immature dendritic cells or monocytes not contacted with the peptide construct.

In one embodiment, the divalent linker comprises one or more glycine residues.

In one embodiment, the peptide construct has the formula P₁-x-P₂ or P₂-x-P₁, wherein P₂ represents a specific antigenic peptide competent for recognition by a class or subclass of immune cells or binding to an antibody, P₁ represents an immunomodulatory peptide which is a portion of an immunoprotein capable of promoting binding to a class or subclass of dendritic cells, and x represents a covalent bond or a divalent peptide linking group.

In one embodiment, the peptide P₁ is the reversal sequence of SEQ ID No. 32.

In one embodiment, the immature dendritic cells or monocytes are collected from the subject, and the cells after maturation are introduced back into the subject.

In one embodiment, the immature dendritic cells or monocytes comprise one or more selected from the group consisting of blood derived monocytes and bone marrow cells.

In one embodiment, the subject is human.

In one embodiment, the divalent linker comprises one or more glycine residues.

In one embodiment, the matured dendritic cells or the peptide construct are conjugated to a radioisotope.

In one embodiment, the radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In one embodiment, the radioisotope is conjugated to the matured dendritic cells with an antibody.

In one embodiment, the antibody has affinity for MHC II, CD11c, DEC-205, Dectin-1, DC-SIGN, or DC-LAMP.

In one embodiment, the matured dendritic cells or the peptide construct are conjugated to a therapeutic agent.

In one embodiment, the therapeutic agent is one or more of a drug selected from the group consisting of auristatin, MMAE, ozogamicin, emtansine, and gelonin; a toxin selected from the group consisting of SEB superantigen or Saporin; or a cytokine.

In one embodiment, the cytokine is interferon-a.

In one embodiment, the matured dendritic cells are conjugated to the therapeutic agent with an antibody.

In one embodiment, the antibody has affinity for MHC II, CD11c, DEC-205, Dectin-1, DC-SIGN, or DC-LAMP.

In one embodiment, the therapeutic agent is conjugated to a lysosomotropic agent.

In one embodiment, the lysosomotropic agent is conjugated to the therapeutic agent through a cleavable linker.

In one embodiment, the peptide construct is conjugated to a therapeutic agent by a cathepsin cleavable valine-citrulline dipeptide linker or by linking with a cysteine or lysine residue of the peptide construct or by conjugation to a group selected from OH groups, COOH groups, amine groups, and amide groups of the peptide construct.

Also disclosed is a method for targeting matured dendritic cells to a site of an autoimmune condition in a subject, comprising:
treating immature dendritic cells or monocytes with a peptide construct *ex vivo* under conditions suitable for maturation of the cells to form matured dendritic cells, the peptide construct having a structure formula P₁-x-P₂ or P₂-x-P₁; and
administering an effective amount of the matured dendritic cells to the subject,
wherein a majority of the matured dendritic cells locate to the site of the autoimmune condition, and wherein
P₂ represents a specific antigenic peptide competent for recognition by a class or subclass of immune cells or binding to an antibody;
P₁ represents an immunomodulatory peptide which is a portion of an immunoprotein capable of promoting binding to a class or subclass of dendritic cells; and
x represents a covalent bond or a divalent peptide linking group.

In one embodiment, the immature dendritic cells or monocytes are collected from the subject, and the cells after maturation are introduced back into the subject.

In one embodiment, the immature dendritic cells or monocytes comprise one or more selected

In one embodiment, the peptide P₁ is derived using the allowed substitutions X₁-X₁₅ as taught by the algorithm taught in Table 2 of a sequence selected of a sequence selected from the group consisting of SEQ ID No.'s 31-39 and variants thereof.

In one embodiment, the peptide P₁ is derived using the allowed substitutions X₁-X₁₅ as taught by the algorithm taught in Table 2 of a sequence selected sequence of SEQ ID No. 32.

In one embodiment, the peptide P₂ is derived using the allowed substitutions X₁-X₁₅ as taught by the algorithm taught in Table 2 of a sequence selected sequence of a sequence selected from the group consisting of SEQ ID No.'s 1, 3, 5, 7, 9, 11, 13, 15, 17, 19, 21, 23, 25, 27, 29 and variants thereof.

In one embodiment, the peptide P₁ is derived using the allowed substitutions X₁-X₁₄ as taught by the algorithm taught in Table 2 of a sequence selected sequence of SEQ ID No. 32.

In one embodiment, the immature dendritic cells or monocytes are collected from a donor compatible with the subject.

In one embodiment, the subject is human.

In one embodiment, the divalent linker comprises one or more glycine residues.

Also disclosed is a method for targeting matured dendritic cells to a site of an autoimmune condition in a subject, comprising: collecting immature dendritic cells or monocytes from a subject or donor, treating the immature dendritic cells or monocytes with a peptide construct *ex vivo* under conditions suitable for maturation of the cells to form matured dendritic cells, the peptide construct having a structure of formula P₁-x-P₂ or P₂-x-P₁; and administering an effective amount of the matured dendritic cells to the subject, wherein a majority of the matured dendritic cells locate to the site of an autoimmune condition, P₂ represents a specific antigenic peptide competent for recognition by a class or subclass of immune cells or binding to an antibody; P₁ represents an immunomodulatory peptide which is a portion of an immunoprotein capable of promoting binding to a class or subclass of dendritic cells; and x represents a covalent bond or a divalent peptide linking group.

In one embodiment, the immature dendritic cells or monocytes comprise one or more selected from the group consisting of blood derived monocytes and bone marrow cells.

In one embodiment, the cytokine is interferon-a.

The composition of claim 120, wherein the antibody has affinity for MHC II, CD11c, DEC-205, Dectin-1, DC-SIGN, or DC-LAMP.

In one embodiment, the therapeutic agent is conjugated to a lysosomotropic agent.

In one embodiment, the lysosomotropic agent is conjugated to the therapeutic agent through a cleavable linker.

In one embodiment, the peptide construct is conjugated to the therapeutic agent by a cathepsin cleavable valine-citrulline dipeptide linker or by linking with a cysteine or lysine residue of the peptide construct or by conjugation to a group selected from OH groups, COOH groups, amine groups, and amide groups of the peptide construct.

Also disclosed is a method for delivering a therapeutic or a radiation source to the site of an autoimmune condition in a subject, comprising: administering matured dendritic cells to the subject, wherein the matured dendritic cells are conjugated to the therapeutic agent or the radiation source, wherein the matured dendritic cells are formed by: contacting or treating immature dendritic cells or monocytes with an effective amount of a peptide construct having the formula P₁-X-P₂ or P₂-X-P₁ under conditions suitable for maturation of dendritic cells or monocytes to form the matured dendritic cells, wherein P₂ represents a specific antigenic peptide competent for recognition by a class or subclass of immune cells or binding to an antibody, P₁ represents an immunomodulatory peptide which is a portion of an immunoprotein capable of promoting binding to a class or subclass of dendritic cells, and x represents a covalent bond or a divalent peptide linking group; and conjugating the matured dendritic cells to the therapeutic or to the radiation source.

Also disclosed is a method for detecting the presence or location of an autoimmune condition in a subject, comprising: administering matured dendritic cells to the subject, wherein the matured dendritic cells are labeled with a tracking marker allowing for detection of the matured dendritic cells; observing the location of the tracking marker in the body of the subject, wherein the tracking marker is concentrated in a location, tissue type or organ structure of the subject's body after an elapse of a period of time from administration of the matured dendritic cells to the subject.

In one embodiment, the matured dendritic cells are formed by: contacting or treating immature dendritic cells or monocytes with an effective amount of a peptide construct having the formula P₁-X-P₂ or P₂-X-P₁ under conditions suitable for maturation of dendritic cells or monocytes to form the matured dendritic cells, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of dendritic cells; and x represents a covalent bond or a divalent peptide linking group.

In one embodiment, the peptide P₂ is one sequence selected from the group consisting of SEQ ID No.'s 1, 3, 5, 7, 9, 11, 13, 15, 17, 19, 21, 23, 25, 27, 29, and variants thereof.

In one embodiment, the peptide P₁ is selected from the group consisting of SEQ ID No.'s 31-39 and variants thereof.

In one embodiment, the peptide P₁ is SEQ ID No. 32.

In one embodiment, the peptide P₂ is a reversal sequence of a sequence selected from the group consisting of SEQ ID No.'s 1, 3, 5, 7, 9, 11, 13, 15, 17, 19, 21, 23, 25, 27, 29, and variants thereof.

In one embodiment, the peptide P₁ is a reversal sequence of a sequence selected from the group consisting of SEQ ID No.'s 31-39 and variants thereof.

In one embodiment, the peptide P₁ is the reversal sequence of SEQ ID No. 32.

In one embodiment, the peptide P₂ is a peptide P₁ is derived using the allowed substitutions X₁-X₁₇ as taught by the algorithm taught in Table 2 sequence of a sequence selected from the group consisting of SEQ ID No.'s 1, 3, 5, 7, 9, 11, 13, 15, 17, 19, 21, 23, 25, 27, 29, and variants thereof.

In one embodiment, the peptide P₁ is a peptide P₁ is derived using the allowed substitutions X₁-X₁₇ as taught by the algorithm taught in Table 2 sequence of a sequence selected from the group consisting of SEQ ID No.'s 31-39 and variants thereof.

In one embodiment, the divalent linker comprises one or more glycine residues.

In one embodiment, the location of the autoimmune condition is detected by observing at least a majority of the tracking marker present in a location, tissue type or organ structure of the subject's body.

In one embodiment, the tracking marker is selected from the group consisting of a radionuclide, a fluorescent dye and a luminescent dye.

In one embodiment, observing the location the tracking marker is done using a radiation detector.

In one embodiment, observing the location the tracking marker is done by observing the presence of fluorescence or radiation in a tissue sample taken from the subject.

In one embodiment, fluorescence is detected by flow cytometry.

In one embodiment, the immature dendritic cells or monocytes are collected from the subject, and where the cells after maturation are introduced back into the subject in an autologous fashion.

In one embodiment, immature dendritic cells or monocytes are collected from a donor compatible with the subject.

In one embodiment, the population of matured dendritic cells exhibits an upregulation of one or more of CD80, CD86 and major histocompatibility complex II relative to immature dendritic cells or monocytes not contacted with the peptide construct.

In one embodiment, the immature dendritic cells or monocytes comprise one or more selected from the group consisting of blood derived monocytes and bone marrow cells.

In one embodiment, the subject is human.

In one embodiment, the matured dendritic cells or the peptide construct are conjugated to a radioisotope.

In one embodiment, the tracking marker is conjugated to the matured dendritic cells with an antibody.

In one embodiment, the antibody has affinity for MHC II, CD11c, DEC-205, Dectin-1, DC-SIGN, or DC-LAMP.

In one embodiment, the tracking marker is conjugated to the peptide construct.

In one embodiment, the tracking marker is a radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In one embodiment, the tracking marker is the tracking marker is a fluorescent dye.

In one embodiment, the dye is selected from the group consisting of Cy5.5, Alexa Fluor (Alexa), CSFE, CMTPX, CMFDA and other fluorescent luminescent or near-infrared probes.

In one embodiment, the fluorescent dye is selected from the group consisting of N, N'-di-carboxypentyl-indodicarbocyanine-5,5'-disulfonic acid (Cy5.5), carboxyfluorescein succinimidyl ester, 4-N(S-glutathionylacetylaminophenyl)arsenoxide-Cy5.5, 2,3-dicyanonaphthalene-Cy5.5 and near-infrared probes, Alexa Fluor (Alexa) probes, 4-N(S-glutathionylacetylaminophenyl)arsenoxide-Cy5.5, 2,3-dicyanonaphthalene-Cy5.5, 4-N(S-glutathionylacetylaminophenyl)arsenoxide-Alexa, 2,3-dicyanonaphthalene-Alexa, 4-N(S-glutathionylacetylaminophenyl)arsenoxide-CSFE, 2,3-dicyanonaphthalene-CSFE and other near-infrared probes.

Also disclosed is a method for modulating an autoimmune condition in a subject, comprising: contacting immature dendritic cells or monocytes with a peptide construct having the formula P₁-x-P₂ or P₂-x-P₁ under conditions suitable for maturation of the cells to form matured dendritic cells; and administering an effective amount of the matured dendritic cells to the subject, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of dendritic cells; and x represents a covalent bond or a divalent peptide linking group.

Also disclosed is a composition comprising activated T cells, the activated T cells formed by contacting T cells with an effective amount of a peptide construct having the formula P₁-x-P₂ under conditions suitable for activation of the T cells to form the activate T cells, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent linking group.

In one embodiment, the T cells are isolated away from blood tissues or lymphatic tissues.

In one embodiment, the composition is substantially free of red blood cells.

In one embodiment, the population of activated T cells produces an increased amount of Interleukin 4 (IL-4) compared to unactivated T cells not contacted with the peptide construct.

In one embodiment, the divalent linker comprises one or more glycine residues.

In one embodiment, the divalent linker is not a peptide.

In one embodiment, the activated T cells are conjugated to a radioisotope.

In one embodiment, the radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In one embodiment, the radioisotope is conjugated to the activated T cells with an antibody.

In one embodiment, the activated T cells are conjugated to a therapeutic agent.

In one embodiment, the antibody has affinity for TCRα, TCRβ, CD3, CD4, CD8, or OX40.

In one embodiment, the therapeutic agent is conjugated to a lysosomotropic agent.

In one embodiment, the lysosomotropic agent is conjugated to the therapeutic agent through a cleavable linker.

The present invention is further related to a composition comprising a population of activated T cells, the population of activated T cells formed by contacting T cells with an effective amount of a peptide construct selected from the group consisting of SEQ ID No.'s 2, 4, 6, 8, 10, 12, 14, 16, 18, 20, 22, 24, 26, 28 and 30 under conditions suitable for activation of T cells.

The present invention is also directed to a composition comprising a population of activated T cells, the population of activated T cells formed by contacting T cells with an effective amount of a peptide mixture of two or more peptide constructs selected from the group consisting of SEQ ID No.'s 2, 4, 6, 8, 10, 12, 14, 16, 18, 20, 22, 24, 26, 28 and 30 under conditions suitable for activation of T cells.

In one embodiment, the population of activated T cells produces an increased amount of Interleukin 4 (IL-4) compared to unactivated T cells not contacted with the peptide construct.

In one embodiment, the activated T cells are conjugated to a radioisotope.

In one embodiment, the radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In one embodiment, the radioisotope is conjugated to the matured dendritic cells with an antibody.

In one embodiment, the antibody has affinity for TCRα, TCRβ, CD3, CD4, CD8, or OX40.

In one embodiment, the activated T cells are conjugated to a therapeutic agent.

In one embodiment, the therapeutic agent is one or more of a drug selected from the group consisting of auristatin, MMAE, ozogamicin, emtansine, and gelonin; a toxin selected from the group consisting of SEB superantigen or Saporin; or a cytokine.

In one embodiment, the cytokine is interferon-a.

In one embodiment, the activated T cells are conjugated to the therapeutic agent with an antibody.

In one embodiment, the antibody has affinity for TCRα, TCRβ, CD3, CD4, CD8, or OX40.

In one embodiment, the therapeutic agent is conjugated to a lysosomotropic agent.

In one embodiment, the lysosomotropic agent is conjugated to the therapeutic agent through a cleavable linker.

Disclosed is a method for producing an activated T cell population, comprising contacting or treating T cells or monocytes with an effective amount of a peptide construct having the formula P₁-X-P₂ or P₂-X-P₁ under conditions suitable for activation of T cells to form activated T cells, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent peptide linking group.

Disclosed is a method for targeting activated T cells to a site of an autoimmune condition in a subject, comprising: contacting T cells with a peptide construct *ex vivo* under conditions suitable for activation of the cells to form the activate T cells, and administering an effective amount of the activated T cells to the subject, wherein a majority of the activated T cells administered to the subject locate to the site of the autoimmune condition.

In one embodiment, the peptide construct has the formula P₁-x-P₂ or P₂-x-P₁, wherein P₂ represents a specific antigenic peptide competent for recognition by a class or subclass of immune cells or binding to an antibody, P₁ represents an immunomodulatory peptide which is a portion of an immunoprotein capable of promoting binding to a class or subclass of T cells, and x represents a covalent bond or a divalent peptide linking group.

In one embodiment, the therapeutic agent is one or more of a drug selected from the group consisting of auristatin, MMAE, ozogamicin, emtansine, and gelonin; a toxin selected from the group consisting of SEB superantigen or Saporin; or a cytokine

In one embodiment, the therapeutic agent is selected from the group consisting of a mictrotubule inhibitor, an antimitotic agent, a maytansinoid, a receptor tyrosine kinase inhibitor, and phosphinositide 3-kinase inhibitor.

In one embodiment, the therapeutic agent is selected from the group consisting of monomethyl auristatin E, ozogamicin, auristatin, carboplatin, 5-fluorouracil, docetaxel, maytansinoid 1 nomifensine, doxorubicin, irinotecan, interferon-a, staphylococcal enterotoxin A superantigen, and staphylococcal enterotoxin B superantigen.

In one embodiment, the peptide construct is conjugated to a therapeutic agent by a cathepsin cleavable valine-citrulline dipeptide linker or by linking with a cysteine or lysine residue of the peptide construct or by conjugation to a group selected from OH groups, COOH groups, amine groups, and amide groups of the peptide construct.

Disclosed is a method for targeting activated T cells to a site of an autoimmune condition in a subject, comprising: treating T cells with a peptide construct *ex vivo* under conditions suitable for activation of the cells to form activate T cells, the peptide construct having a structure formula P₁-x-P₂ or P₂-x-P₁; and administering an effective amount of the activated T cells to the subject, wherein a majority of the activated T cells locate to the site of the autoimmune condition, and wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent peptide linking group.

Disclosed is a method for targeting activated T cells to a site of an autoimmune condition in a subject, comprising: collecting T cells from a subject or donor, treating the T cells with a peptide construct *ex vivo* under conditions suitable for activation of the cells to form activated T cells, the peptide construct having a structure of formula P₁-x-P₂ or P₂-x-P₁; and administering an effective amount of the activated T cells to the subject, wherein a majority of the activated T cells locate to the site of an autoimmune condition, P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent peptide linking group.

In one embodiment, the peptide P₁ is derived using the allowed substitutions X₁-X₁₇ as taught by the algorithm taught in Table 2 of a sequence selected of a sequence selected from the group consisting of SEQ ID No.'s 31-39 and variants thereof.

In one embodiment, the therapeutic agent is selected from the group consisting of monomethyl auristatin E, ozogamicin, auristatin, carboplatin, 5-fluorouracil, docetaxel, maytansinoid 1 nomifensine, doxorubicin, irinotecan, interferon-a, staphylococcal enterotoxin A superantigen, and staphylococcal enterotoxin B superantigen.

In one embodiment, the peptide construct is conjugated to the therapeutic agent by a cathepsin cleavable valine-citrulline dipeptide linker or by linking with a cysteine or lysine residue of the peptide construct or by conjugation to a group selected from OH groups, COOH groups, amine groups, and amide groups of the peptide construct.

Disclosed is a method for delivering a therapeutic or a radiation source to the site of an autoimmune condition in a subject, comprising: administering activated T cells to the subject, wherein the activated T cells are conjugated to the therapeutic agent or the radiation source, wherein the activated T cells are formed by: contacting or treating T cells with an effective amount of a peptide construct having the formula P₁-X-P₂ or P₂-X-P₁under conditions suitable for activation of T cells to form the activated T cells, wherein P₂ represents a specific antigenic peptide competent for recognition by a class or subclass of immune cells or binding to an antibody, P₁ represents an immunomodulatory peptide which is a portion of an immunoprotein capable of promoting binding to a class or subclass of T cells, and x represents a covalent bond or a divalent peptide linking group; and conjugating the activated T cells to the therapeutic or to the radiation source.

In one embodiment, the therapeutic agent is selected from the group consisting of a mictrotubule inhibitor, an antimitotic agent, a maytansinoid, a receptor tyrosine kinase inhibitor, and phosphinositide 3-kinase inhibitor.

In one embodiment, the therapeutic agent is selected from the group consisting of monomethyl auristatin E, ozogamicin, auristatin, carboplatin, 5-fluorouracil, docetaxel, maytansinoid 1 nomifensine, doxorubicin, irinotecan, interferon-a, staphylococcal enterotoxin A superantigen, and staphylococcal enterotoxin B superantigen.

In one embodiment, the peptide construct is conjugated to a therapeutic agent by a cathepsin cleavable valine-citrulline dipeptide linker by linking with a cysteine or lysine residue of the peptide construct or by conjugation to a group selected from OH groups, COOH groups, amine groups, and amide groups of the peptide construct.

Disclosed is a method for detecting the presence or location of an autoimmune condition in a subject, comprising: administering activated T cells to the subject, wherein the activated T cells are labeled with a tracking marker allowing for detection of the activated T cells; observing the location of the tracking marker in the body of the subject, wherein the tracking marker is concentrated in a location, tissue type or organ structure of the subject's body after an elapse of a period of time from administration of the activated T cells to the subject.

In one embodiment, the activated T cells are formed by: contacting or treating T cells with an effective amount of a peptide construct having the formula P₁-X-P₂ or P₂-X-P₁ under conditions suitable for activation of T cells to form the activated T cells, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent peptide linking group.

In one embodiment, the location of the autoimmune condition is detected by observing at least a majority of the tracking marker present in a location, tissue type or organ structure of the subject's body.

In one embodiment, the tracking marker is selected from the group consisting of a radionuclide, a fluorescent dye and a luminescent dye.

In one embodiment, observing the location the tracking marker is done using a radiation detector.

In one embodiment, observing the location the tracking marker is done by observing the presence of fluorescence or radiation in a tissue sample taken from the subject.

In one embodiment, fluorescence is detected by flow cytometry.

In one embodiment, the T cells are collected from the subject, and where the cells after activation are introduced back into the subject in an autologous fashion.

In one embodiment, T cells are collected from a donor compatible with the subject.

In one embodiment, the subject is human.

In one embodiment, the activated T cells or the peptide construct are conjugated to a radioisotope.

In one embodiment, the tracking marker is conjugated to the activated T cells with an antibody.

In one embodiment, the antibody has affinity for TCRα, TCRβ, CD3, CD4, CD8, or OX40.

In one embodiment, the tracking marker is conjugated to the peptide construct.

In one embodiment, the tracking marker is a radioisotope is selected from the group consisting of ¹⁸F, ³²P, ⁶⁴Cu, ⁹⁰Y, ⁹⁹Tc, ¹³¹I, ¹²⁵I, ¹²⁴I, ⁸⁹Zr, ¹¹¹In, ¹⁸⁸Re, and ¹⁷⁷Lu.

In one embodiment, the tracking marker is the tracking marker is a fluorescent dye.

In one embodiment, the fluorescent dye is selected from the group consisting of N, N'-di-carboxypentyl-indodicarbocyanine-5,5'-disulfonic acid (Cy5.5), carboxyfluorescein succinimidyl ester, 4-N(S-glutathionylacetylaminophenyl)arsenoxide-Cy5.5, 2,3-dicyanonaphthalene-Cy5.5 and near-infrared probes, Alexa Fluor (Alexa) probes, 4-N(S-glutathionylacetylaminophenyl)arsenoxide-Cy5.5, 2,3-dicyanonaphthalene-Cy5.5, 4-N(S-glutathionylacetylaminophenyl)arsenoxide-Alexa, 2,3-dicyanonaphthalene-Alexa, 4-N(S-glutathionylacetylaminophenyl)arsenoxide-CSFE, 2,3-dicyanonaphthalene-CSFE and other near-infrared probes.

Disclosed is a method for modulating an autoimmune condition in a subject, comprising: contacting T cells with a peptide construct having the formula P₁-x-P₂ or P₂-x-P₁ under conditions suitable for maturation of the cells to form activated T cells; and administering an effective amount of the activated T cells to the subject, wherein P₂ represents a specific antigenic peptide competent for recognition by a class or subclass of immune cells or binding to an antibody; P₁ represents an immunomodulatory peptide which is a portion of an immunoprotein capable of promoting binding to a class or subclass of T cells; and x represents a covalent bond or a divalent peptide linking group.

In one embodiment, the immune response is modulated without administering supplementary immunomodulators to the subject.

In one embodiment, the divalent linker comprises one or more glycine residues.

Disclosed is a method for treating or preventing inappropriate autoimmune response in individuals at risk for autoimmune disease, wherein a pharmacologically effective amount of a peptide construct having the formula P₁-x-P₂ or P₂-x-P₁ is administered to the individual to effectively eliminate the set or subset of T cells involved in the autoimmune response.

Disclosed is a method for modulating an inappropriate autoimmune response in individuals at risk for autoimmune disease, wherein a pharmacologically effective amount of a peptide construct having the formula P₁-x-P₂ or P₂-x-P₁ is administered to the individual to redirect the autoimmune response from a Th1 to a Th2 immune response, or from a Th2 to a Th1 response, whereby the inappropriate autoimmune response is modulated to decrease or eliminate the adverse effects associated with the inappropriate autoimmune response.

Disclosed is a first method for treating or preventing inappropriate autoimmune response in individuals at risk for autoimmune disease, wherein a pharmacologically effective amount of a peptide construct of formula (I) is administered to the individual to effectively eliminate the set or subset of T cells involved in the autoimmune response.

Disclosed is a method for modulating an inappropriate autoimmune response in individuals at risk for autoimmune disease, wherein a pharmacologically effective amount of a peptide construct having the formula P₁-x-P₂ or P₂-x-P₁ is administered to the individual to redirect the autoimmune response from a Th1 to a Th2 immune response, or from a Th2 to a Th1 immune response, whereby the inappropriate autoimmune response is modulated to decrease or eliminate the adverse effects associated with the inappropriate autoimmune response.

Disclosed is a composition comprising a population of matured dendritic cells. The population of matured dendritic cells is formed by treating immature dendritic cells or monocytes with an effective amount of a peptide construct serving as an immunomodulator having the formula P₁-x-P₂ or P₂-x-P₁ under conditions suitable for maturation of the immature dendritic cells to form the matured or effective dendritic cells which interact with T cells, where P₂ represents a specific antigenic peptide derived from an autoimmune antigen, P₁ represents an immunomodulatory peptide which is a portion of an immunoprotein capable of promoting binding to a class or subclass of DC cells, and -x- represents a covalent bond or a divalent linking group.

Disclosed is a method for targeting matured or effective dendritic cells to a site of an autoimmune condition in a subject. Immature dendritic cells or monocytes are treated with a peptide construct immunomodulator *ex vivo* under conditions suitable for maturation of the cells to form more matured or activated dendritic cells, and an effective amount of these matured dendritic cells are administered to the subject, wherein a majority of the dendritic cells administered to the subject locate to the site of an autoimmune or other undesirable immune response.

### DEFINITIONS

Unless defined otherwise, all technical and scientific terms used herein generally have the same meaning as commonly understood by one of ordinary skill in the relevant art.

The articles "a" and "an" are used herein to refer to one or to more than one (*i.e.,* to at least one) of the grammatical object of the article. By way of example, "an element" means one element or more than one element.

The trademarked term "L.E.A.P.S.™" (or "L.E.A.P.S.") refers to "Ligand Epitope Antigen Presentation System" and stands for a peptide based antigen delivery technology for directing immune responses toward a desired outcome.

The term "adjuvant" refers to substance that accelerates, prolongs or enhances antigen-specific immune responses when used in combination with vaccine antigens.

The terms "administering," "administer," "delivering," "deliver," "introducing," and "introduce" can be used interchangeably to indicate the introduction of a therapeutic or diagnostic agent into the body of a patient in need thereof to treat a disease or condition, and can further mean the introduction of any agent into the body for any purpose.

The term "antigen" refers to a substance or molecule that generates an immune response when introduced to the body or any molecule or fragment thereof. The term additionally refers to any molecule or molecular fragment that can be bound by a major histocompatibility complex (MHC).

The term "autologous" refers to a situation wherein the donor and recipient of cells, fluids or other biological sample is the same person or is a genetically identical individual or person.

The term "autoimmune disease" refers to a condition wherein a subject's immune system directs an immune response against the subject's own cells and tissues. Autoimmune diseases include, but are not limited to, rheumatoid arthritis (RA) and osteoarthritis.

The term "blood tissue" refers to cells suspended in or in contact with plasma.

The term "bone marrow cell" or "BMC" refers to any cell originating from the interior of bones.

The terms "CD80," "CD86," "CD11c," "CD85", "CD4", "CD8" and similar terms refer to cell surface molecules present on leukocyte cells through a nomenclature protocol maintained by Human Cell Differentiation Molecules (www.hcdm.org; Paris, France).

The term "citrulline" indicates an α-amino acid. The name is derived from *citrullus.* Citrulline has the idealized chemical formula H₂NC(O)NH(CH₂)₃CH(NH₂)CO₂H, and is a key intermediate in the urea cycle, the pathway by which mammals excrete ammonia. Citrulline is made from ornithine and carbamoyl phosphate in one of the central reactions in the urea cycle. It is also produced from arginine as a by-product of the reaction catalyzed by NOS family (NOS; EC 1.14.13.39). Arginine is first oxidized into N-hydroxyl-arginine, which is then further oxidized to citrulline concomitant with release of nitric oxide. Citrulline is formed in the protein, after protein synthesis, by enzymatic or chemical conversion of arginine to citrulline.

The term "collagen" indicates a group of naturally occurring proteins found in animals, especially in the flesh and connective tissues of vertebrates. Collagens can be divided into several different groups, with and type II collagens being a more implicated group than other collagen groups in some RA models and patients. Collagen is the main component of connective tissue, and is the most abundant protein in mammals, making up about 25% to 35% of the whole-body protein content. Collagen, in the form of elongated fibrils, is mostly found in fibrous tissues such as tendon, ligament and skin, and is also abundant in cornea, cartilage, bone, blood vessels, the gut, and intervertebral disc. The fibroblast is the most common cell which manufactures and secretes collagen.

The terms "conjugate," "conjugation" and similar terms refer to two molecules being spatially associated with each other by covalent linkage, non-covalent binding or by a combination of covalent linkage and non-covalent binding. For example, an antibody can be conjugated to an epitope through non-covalent binding to the epitope, as well as the antibody serving to conjugate the epitope (such as a cell surface marker) to a compound that is linked to the antibody.

The term "comprising" includes the recited steps, elements, structures or compositions of matter and does not exclude any un-recited elements, structures or compositions of matter.

The term "consisting of' includes and is limited to whatever follows the phrase "consisting of." Thus, the phrase indicates that the limited elements are required or mandatory and that no other elements may be present.

The phrase "consisting essentially of' includes any elements listed after the phrase and is limited to other elements that do not interfere with or contribute to the activity or action specified in the disclosure for the listed elements. Thus, the phrase indicates that the listed elements are required or mandatory but that other elements are optional and may or may not be present, depending upon whether or not they affect the activity or action of the listed elements.

The term "cytokine(s)" refers to a group of small molecules (5-20 kDa) secreted by a broad group of cells, including cells of the immune system (such as T, B and dendritic cells) and non-immune cells (such as endothelial cells and fibroblasts).Although they have additional functions, cytokines are especially important in regulation of the immune system. Cytokines generally affect the behavior of other cells or the releasing cell itself, which behavior can include growth, movement, secretion or action of other molecules, including other cytokines. Cytokines fulfill their cell signaling role through binding to a cell receptor. Examples of cytokines include interferon gamma (IFNγ), tumor necrosis factor alpha (TNFα), IL-1, and IL-17.

A "dendritic cell" or "DC" refers to an antigen-presenting leukocyte that is found in the skin, mucosa, and lymphoid tissues having a capability under appropriate conditions to initiate a primary immune response by activating T cells, lymphocytes and/or secreting cytokines. DCs can be further subdivided into immature DC (iDCs) and mature DC (mDCs), and by a plasma or myleoid cell origin, respectively, as "DCp" or "DCm."

The term "diagnostic" or "diagnosis" refers to any technique for determining the presence of any disease condition such as cancer, or in certain embodiments a particular autoimmune condition or antigen, in a subject.

The term "divalent linker" refers to any moiety having a structure forming a peptide bond to a first peptide moiety and forming a second bond to a second peptide moiety.

The term "effective amount" is an amount of a therapeutic which produces a therapeutic response, including an immune response, in the subject to which the therapeutic is administered.

A "heteroconjugate" refers to a protein or peptide or other entity containing at least two amino acid sequences covalently linked to form a single molecule, wherein two sequences originate or are homologous to proteins expressed at least in part by different genes.

The term "homocitrulline" is derived from the amino acid lysine, where the epsilon terminal amino function is replaced by a carbamylated functional group (the same functional group as is present in citrulline, which is derived from arginine).Homocitrulline is very similar to citrulline, with the two entities having an alpha carbon amino group and the same functional groups at their two ends, with the distinction that homocitrulline is one carbon atom shorter than citrulline.

The term "infection" refers to the colonization of a host organism by a pathogen that can include parasites, viruses, and bacteria.

An "immature dendritic cell" or "iDC" is a dendritic cell in a state characteristic of immune cells prior to contact with an antigen and having a limited present ability to activate T cells, lymphocytes and/or to secrete cytokines. However, "immature dendritic cells" may acquire the ability to activate T cells, lymphocytes and secrete cytokines upon contact with an antigen.

The terms "immunomodulatory" and "immunoprotein" refer to a protein, peptide or cell having the ability to bind or interact with an immune cell to alter or to regulate one or more immune functions.

The terms "interferon gamma," "IFNγ," or "type II interferon" refer to a dimerized soluble cytokine. The IFNγ monomer consists of a core of six α-helices and an extended unfolded sequence in the C-terminal region. It is a cytokine that is critical for innate and adaptive immunity against viral and intracellular bacterial infections and for tumor control. Aberrant IFNγ expression is associated with a number of autoinflammatory and autoimmune diseases. The importance of IFNγ in the immune system stems in part from its ability to inhibit viral replication directly, and most importantly from its immunostimulatory and immunomodulatory effects. IFNγ is produced predominantly by natural killer (NK) and natural killer T (NKT) cells as part of the innate immune response, and by CD4 Th1 and CD8 cytotoxic T lymphocyte (CTL) effector T cells once antigen-specific immunity develops.

The term "immune response" refers to the response by an animal's immune system to invasion by a pathogen or foreign object. As such, the immune system is a collection of tissues, cells, molecules and processes that protect against disease. The immune system and associated responses may be subdivided, for example into innate and adaptive immune systems and responses. The innate immune system consists mainly of fast and broadly-targeted defenses such as structural barriers (*e.g.,* the skin), the inflammatory response, the so-called complement system, and certain immune cells, but lacks a memory component. The adaptive immune system mounts a fast and targeted response and consists mainly of specialized immune cells (lymphocytes), and contains an immunological memory, allowing the system to adapt so as to better respond to pathogens previously encountered.

The terms "Th1," "Th2" and "Th17" refer to specific branches of the adaptive immune system, characterized by activity of two different subclasses of T helper cells, and activity against different types of pathogens. The Th1 response leads to "cell-mediated immunity," is primarily directed against intracellular pathogens (mainly viruses and bacteria), and is characterized by the release of IFNγ and activation of macrophages, and release of opsonizing (coating) antibodies by B cells. The Th2 response leads to "humoral immunity," is primarily directed against extracellular pathogens (mainly bacteria, helminths (worms) and toxins), and is characterized by the release of IL4 and of neutralizing antibodies by B cells. Generally, activation of a Th1 response will inhibit a Th2 response, and vice versa. The Th17 response is associated with a third distinct subset of T helper cells, which produce IL17, and generally has an antimicrobial role.

The term "interleukin 12p70" refers to a cytokine (IL-12), and in particular the p70 subunit. This subunit is composed of two parts, a p40 shared by many other cytokines and a p35 unique to this molecule, and is produced by dendritic cells capable of directing the development of lymphocytes in a Th1 immune response.

The terms "isolated matured dendritic cells" or "isolated dendritic cells" refer to dendritic cells suspended in a liquid medium, a cell culture or a composition wherein at least 50% of the viable cells present in the liquid medium, the cell culture or the composition are dendritic cells or monocytes.

An "isotype control" is usually a fluorescent conjugate antibody having the same serological structure (usually referring to having the same class of heavy and light chains) and optionally the same fluorescent conjugate dye as an antibody conjugate having affinity for a cellular surface or cytokine marker, with the exception that the isotype control does not have affinity for the cellular surface or cytokine marker.

The term "magnetic resonance imaging" refers to any technique where information is collected from the exposure of a subject or sample to a magnetic field.

The term "maturation" refers to a process of signal-regulated adjustments to immature immune cells that lead to cellular specialization. For example, an "immature dendritic cell" matures into a "matured dendritic cell." Maturation is evidenced by changes in kind and amount of cell surface activation markers such as, but not restricted to, CD11c, CD80, CD86, MHC-II, and CD25.

The terms "matured dendritic cell," "maturated dendritic cell," "activated dendritic cell" or "effective dendritic cell" refer to a dendritic cell in a state characteristic of cells after contact with an antigen and having a present ability to initiate a primary immune response by activating T cells, lymphocytes and/or secreting cytokines.

The terms "activated T cell" or "effective T cell" refer to a T cell in a state characteristic of cells after contact with an antigen-presenting cell and having an ability to initiate a primary immune response by activating B cells, other T lymphocytes and/or secreting cytokines.

The term "monocyte" refers to immune cells produced by bone marrow and haematopoietic stem cells (*i.e.,* stem cells that can produce the cellular components of blood), having the ability to differentiate into macrophages or dendritic cells.

The terms "originating" and "derived" as related to peptide sequences refers to an organism or cell type that produces a protein containing the peptide sequence.

The term "paramagnetic contrast agent" refers to any agent having paramagnetic behavior in an applied magnetic field indicated by a positive magnetic susceptibility, *i.e.,* attraction to the magnetic field. This is opposed to diamagnetic agents, which are repelled by an external magnetic field.

The terms "peptide" and "peptide construct" refer to a molecule including two or more amino acid residues linked by a peptide bond. The term "peptide" includes molecular species where only part of the molecule has peptide character and/or where two parts of the molecular species formed of peptide bonds are covalently linked by a divalent linker. A "polypeptide" is a long, continuous and unbranched peptide chain.

The term "phenotype" as relating to the phenotype of immune cells refers to any observable characteristic or trait of a cell such as its morphology, development, biochemical or physiological properties, including the expression or presence of specific cell surface proteins or markers.

The term "proteoglycans" (PG) indicates proteins that are heavily glycosylated. The basic proteoglycan unit consists of a "core protein" with one or more covalently attached glycosaminoglycan (GAG) chains. The point of attachment is a serine (Ser) residue to which the glycosaminoglycan is joined through a tetrasaccharide bridge (for example: chondroitin sulfate-GlcA-Gal-Gal-Xyl-PROTEIN). The serine residue is generally in the sequence -Ser-Gly-X-Gly-(where X can be any amino acid residue), although not every protein with this sequence has an attached glycosaminoglycan. The chains are long, linear carbohydrate polymers that are negatively charged under physiological conditions, due to the occurrence of sulfate and uronic acid groups. Proteoglycans occur in the connective tissue. Aggrecan is one specific PG molecule composed of several domains, the first domain of which, designated G1, is a major source of antigen found in RA reactive to PG.

The term "red blood cells" or "RBC" refers to erythrocytes, the blood cells responsible for transporting oxygen

The term "rheumatoid arthritis" (RA) is a chronic, systemic inflammatory disorder that may affect many tissues and/organs, but principally attacks flexible (synovial) joints. Rheumatoid arthritis involves an inflammatory response of the capsule around the joints (synovium) secondary to swelling (hyperplasia) of synovial cells, excess synovial fluid, and the development of fibrous tissue (pannus) in the synovium. The pathology of the disease process often leads to the destruction of articular cartilage and ankylosis (fusion) of the joints. Rheumatoid arthritis can also produce diffuse inflammation in the lungs, membrane around the heart (pericardium), membranes of the lung (pleura), and white of the eye (sclera), and also nodular lesions, most common in subcutaneous tissue. Although the cause of rheumatoid arthritis is unknown, autoimmunity plays a pivotal role in both its chronicity and progression, and RA is considered a systemic autoimmune disease. RA patients often have antibodies and/or T cells reactive to one or more of the common proteins found in joints and connective tissue such as collagen, aggrecan, and fibrin, either as the native form or as post translationally-modified (PTM) forms such as citrullinated (Cit) or carbamylated (Hct). RA patients often have rheumatoid factor (Rf) which is an immunoglobulin molecule. Rf and anti-Cit antibodies are not found in CIA models but are found in the PGIA model (described in detail below). About 1% of the world's population is afflicted by rheumatoid arthritis, women three times more often than men. Onset is most frequent between the ages of 40 and 50, but people of any age can be affected. In addition, individuals with the HLA-DR1 or HLA-DR4 serotypes have an increased risk for developing the disorder. It can be a disabling and painful condition, which can lead to substantial loss of functioning and mobility if not adequately treated. It is a clinical diagnosis made on the basis of symptoms, physical examination, radiographs (X-rays) and laboratory results. Both the American College of Rheumatology (ACR) and the European League Against Rheumatism (EULAR) publish classification criteria for the purpose of research. Diagnosis and long-term management are typically performed by a rheumatologist, an expert in joint, muscle and bone diseases. Based on different immunological reactivities, tissue involvement, joints involved and symptoms it is likely there are several different categories of RA, each involving different proteins (see Carrasco, R. & Barton, A. Biomarkers of outcome in rheumatoid arthritis. *Rheumatology Reports* 2, 776 (2010)) such as collagen, PG, vimentin and keratin, as well as some other proteins, perhaps different PTM, and different immune pathways irregularities of either the Th1 or Th2 pathway. Therefore, each category likely requires different therapeutic proteins or vaccines.

The term "subject" or "patient" refers to an animal, including mice and humans, to which a therapeutic agent is administered.

The term "systemic immune response" refers to an immune response where antibodies, cytokines or immune cells generated by the immune response are detectable throughout the circulatory and lymph systems of the body.

The term "T cell" refers to a lymphocyte having T cell receptor (TCR) proteins, CD4 or CD8 and CD3, on the surface of the cell.

The term "transfection" refers to the process of introducing nucleic acids into nucleated cells.

The terms "treating" and "treatment," as related to treating or treatment of immune cells, refers to bringing an immune cell into contact with a substance or composition for a time period sufficient to cause a change in phenotype.

The term "vaccine" refers to a composition containing one or more antigens that stimulate an immune response when administered to an organism *in vivo.*

The term "vimentin" refers to a type III intermediate filament (IF) protein that is expressed in mesenchymal cells. IF, along with tubulin-based microtubules and actin-based microfilaments, comprises the cytoskeleton (the protein scaffolding that determines cellular shape). All IF proteins are expressed in a highly developmentally-regulated fashion; vimentin is the major cytoskeletal component of mesenchymal cells. Because of this, vimentin is often used as a marker of mesenchymally-derived cells or cells undergoing an epithelial-to-mesenchymal transition (EMT) during both normal development and metastatic progression. Vimentin as a PTM citrullinated form is found in some RA patients and is recognized by anti-Cit antibodies.

### L.E.A.P.S. summary

The peptide constructs disclosed herein are based on L.E.A.P.S. technology and are heteroconjugates of two peptides which are linked together covalently. The peptide heteroconjugates or constructs can be synthesized artificially using solid-phase synthesis or another synthetic technique, or can be expressed using recombinant DNA technology. The two peptides can be synthesized separately and joined covalently or can be synthesized or expressed as a single construct.

L.E.A.P.S. uses small peptides referred to as T cell binding ligands (TCBLs) as a peptide antigen delivery technology wherein the TCBLs are peptide sequences derived from human immune system molecules known or suspected to bind to human T cells. L.E.A.P.S. includes a first peptide which is an antigenic peptide associated with disease or the causative organism of disease, covalently bonded to a second peptide which is a T cell binding ligand. The hetero-functional cellular immunological reagents taught by U.S. 5,652,342 are antigen- (disease-) specific and T cell binding ligands, which include portions of MHC Classes I and II or accessory molecules such as β-2-microglobulin, portions of LFA-3, or portions of the Fc region of the heavy chain of immunoglobulins. The therapeutic peptides can be a small peptide wherein the peptide can be used as a potential antigen, as in Epimmune's Padre™, Antigen Express "Ii-Key" system or CEL-SCI's Ligand Epitope Antigen Presentation System (L.E.A.P.S.) technology as described in U.S. 5,652,342, 6,096,315, 6,995,237 and 7,256,254.

A first peptide (hereinafter referred to as Peptide P2) of the heteroconjugate is a portion of an immunoprotein capable of promoting binding to a class or subclass of dendritic cells (DCs) or T cells and is referred to as an immune cell binding ligand (ICBL or TCBL). Peptide P2 may have a structure for promoting interaction and/or binding with specific surface receptors present on DCs and subsequently on T cells. Peptide P2 can be a peptide sequence derived from major histocompatibility complex (MHC) I or II. A more detailed discussion of Peptide P2 and the peptide heteroconjugates involved with L.E.A.P.S. technology can be found in U.S. Pat. No. 5,652,342. As the examples herein demonstrate, the L.E.A.P.S. heteroconjugates disclosed herein have the capability to modulate an undesirable autoimmune response and cause a reduction in symptoms and/or disease severity. The exact mechanism for immune modulation and/or a decrease in immune response in an antigen-specific matter is not fully known, and Applicants do not wish be bound by any particular theory regarding the mechanism of operation of the L.E.A.P.S. heteroconjugates disclosed herein.

Regardless of the theoretical mechanism for the action of L.E.A.P.S heteroconjugate vaccines in modulating immune response, the present disclosure describes making and using effective L.E.A.P.S. heteroconjugates of human aggrecan peptides in subjects or with subjects DCs maturing these DCs and using in the subjects after *ex vivo* activation.

A second peptide (hereinafter referred to as Peptide PI) is a specific antigen peptide derived from and/or associated with an autoimmune condition. Peptide P1 being covalently linked to the ICBL Peptide P2 is thought to allow for a more effective recognition of the antigen Peptide P1 by the immune system and specific immune cells, thus allowing for antigen-specific immunomodulation. Peptide epitopes that have a limited number of amino acid residues have sufficient structure to be bound by an antibody or an MHC molecule with a high degree of specificity. However, peptide epitopes of limited size are less competent to cross-link immunoglobulins and cause lymphocyte activation and/or are capable of inducing an immune reaction or immunomodulation. As such, small peptide epitopes introduced into a subject may produce a poor immune response and need assistance.

In the L.E.A.P.S. heteroconjugates disclosed herein, the antigen Peptide P1 is covalently bound to ICBL/TCBL Peptide P2 or another immunomodulatory peptide having the capability to bind to molecules present on the surface of dendritic cells or monocytes. Once bound to the surface of a dendritic cell, the antigen Peptide P1 can then be recognized by local T cell receptor (TCR) or major histocompatibility complex (MHC) molecules to trigger a corresponding immune response and/or immunomodulation through an immune recognition of the antigen Peptide P1. In certain embodiments, DCs are treated or contacted with a L.E.A.P.S. heteroconjugate *ex vivo* (away from a subject's body) and then administered to the subject. Through such a mechanism, the immune system of a subject receiving the L.E.A.P.S. heteroconjugate-activated DCs can have a modulated response to a source of antigen within the body that triggers the autoimmune condition. In certain other embodiments, the L.E.A.P.S. heteroconjugate-activated DCs, when administered to a subject, locate to the site of a source of the antigen, whether such antigen source is a self-antigen involved in an autoimmune condition or not. The L.E.A.P.S. heteroconjugate-activated DCs can be associated or conjugated with a radio label, dye, therapeutic compound or source of ionizing radiation to assist with the detection or imaging of the antigen source or to deliver the therapeutic compound or ionizing radiation to the site of the antigen source.

In any embodiment, the L.E.A.P.S. heteroconjugates disclosed have a result wherein the extent of pro-inflammatory or inflammatory cytokines produced during the immune response to the peptide constructs can be reduced relative to levels typically associated with larger antigen proteins containing many different epitope sequences. Further, a Th1 type of immune response or a Th2 type of immune response may be promoted based upon the identity of the ICBL Peptide P2 conjugated with the antigen Peptide P1.

A still further embodiment of the L.E.A.P.S. heteroconjugates disclosed herein is that the heteroconjugates can be treated or contacted with dendritic cells isolated from a subject or donor under conditions where the dendritic cells differentiate into more matured immune cells capable of directing immunity toward the antigen peptide sequence contained within the L.E.A.P.S. heteroconjugates. The matured DCs can modulate an immune response to sources of the antigen within the body of a subject to whom the matured DCs are administered.

### Direct use of L.E.A.P.S. conjugates in vivo

Disclosed is *in vivo* immunization with a L.E.A.P.S. heteroconjugate vaccine containing the appropriate epitopes, which is protective in several autoimmune and infectious disease challenge models (Rosenthal K et al., Immunization with a L.E.A.P.S. heteroconjugate containing a CTL epitope and a peptide from beta-2-microglobulin elicits a protective and DTH response to herpes simplex virus type 1 1999, Vaccine 17: 535-542; Goel, N., Zimmerman, D., & Rosenthal, K. 2003. A L.E.A.P.S. Heteroconjugate Vaccine Containing a T Cell Epitope From HSV-1 Glycoprotein D Elicits Th1 Responses and Protection. Vaccine 21:4410-4420; Goel N et al., A L.E.A.P.S. Heteroconjugate Vaccine Containing a T Cell Epitope From HSV-1 Glycoprotein D Elicits Th1 Responses and Protection 2003, Vaccine 21:4410-4420; Cihakova D et al., L.E.A.P.S. heteroconjugate is able to prevent and treat experimental autoimmune myocarditis by altering trafficking of autoaggressive cells to the heart 2008, Int. Immunopharmacol. 8:624-633; Zimmerman DH et al., CEL-2000: A Therapeutic Vaccine for Rheumatoid Arthritis Arrests Disease Development and Alters Serum Cytokine / Chemokine Patterns in the Bovine Collagen Type II Induced arthritis in the DBA Mouse Model, 2010 Int. Immunopharmacol. 10:412).Unpublished studies for other infectious diseases such as H1N1 influenza in various animal models including mouse are demonstrated in U.S. Provisional App. Nos. 61/490,050 and 61/538,427.

### Use of L.E.A.P.S. activated dendritic cells and/or T cells directly or after further processing

The invention contemplates that the same protective conjugates are also effective in an antigen-specific manner based on a HSV challenge study using an *ex vivo* adoptive transfer model *et al.* (Taylor PR et al., J-L.E.A.P.S. Vaccines Initiate Murine Th1 Responses By Activating Dendritic Cells 2010, Vaccine 28:5533-42). Murine and human DCs respond similarly to treatment with L.E.A.P.S. heteroconjugates (Taylor PR *et al.,* 2010 *supra*). Boonnak et al., 2013 (Antigen-activated dendritic cells ameliorate influenza A infections, J. Clin. Invest. 2013 Jul 1;123(7):2850-61) showed that these antigen-specific J-L.E.A.P.S.-activated DCs are effective in treatment even after infection has been initiated for influenza in mice. Without being limited to any theory, just as the murine L.E.A.P.S.-activated DC are protective in an antigen-specific mode in a HSV-1 murine challenge model, they are also protective for other infectious diseases such as H1N1 influenza in various animal models including mouse (U.S. Provisional App. Nos. 61/490,050 and 61/538,427).

L.E.A.P.S.-specific antigenic peptides can be manufactured and stored in a cost effective manner, and are sufficiently stable during storage before use to *ex vivo* activate DC or T cells for therapeutic and diagnostic use. Lyophilized L.E.A.P.S. peptides can be stored at -70°C for several years as a desiccated dry powder in a darkened container without degradation. In other embodiments, the peptides can be stored at room temperature as a desiccated powder under nitrogen in a darkened container and can show little-to-no degradation after 12 months. Degradation and purity can be determined by reverse phase (RP) and ion exchange (IEC) HPLC.

### Direct use of L.E.A.P.S. conjugates in vivo

Disclosed are peptide constructs useful for treatment of autoimmune disease, particularly for rheumatoid arthritis, which differ from the above approaches used with antigenic peptide alone. The novel constructs bind in an antigen-specific manner and redirect the T cell in the direction of a non-deleterious autoimmune response, primarily from a Th1 to a Th2 immune response, but where advantageous, from a Th2 to a Th1 immune response. Alternatively, the novel constructs include one peptide component which will bind to T cells associated with autoimmune disease, while a second peptide component will bind to sites on the T cells which will preclude the normal sequence of events required for cell activation thereby initiating an abortive T cell modulation resulting in cell anergy and apoptosis.

The peptide constructs disclosed herein are based on L.E.A.P.S., which can also convert small peptides, which typically do not exhibit a strong effect on the immune system, into antigen-specific immunomodulators.

The immunomodulators disclosed herein can have the ability to promote the differentiation of immature dendritic cells (DCs) to mature DCs that are able to affect other components of the immune system with respect to specific antigens. The immunogens disclosed herein can promote the upregulation of CD11c, CD80, CD86 and major histocompatibility complex class II (MHC II) in immature DCs isolated or generated from bone marrow cells, which are phenotypical indications of matured DCs. The matured DCs have an increased production of IL-12, particularly IL-12p70, indicating matured DCs that are competent to signal a Th1-type immune response.

### Use of L.E.A.P.S. activated DCs to treat a subject

Upon administration to a subject, DCs matured with the immunomodulator disclosed herein can have the capability to locate to a site in the subject's body harboring a source of the antigen to which an autoimmune response is directed. In certain embodiments, the administered matured DCs can have the ability to modulate an immune response to an autoimmune antigen in the recipient.

### Use of L.E.A.P.S. activated T cells to treat a subject

Upon administration to a subject, T cells treated with the immunomodulator disclosed herein can have the capability to locate to a site in the subject's body harboring a source of the antigen to which an autoimmune response is directed. In certain embodiments, the administered activated T cells can have the ability to modulate an immune response to an autoimmune antigen in the recipient.

### Use of multiple L.E.A.P.S conjugates to treat a subject

The peptide constructs described herein may be therapeutically useful in varying degrees depending on individual patient parameters. Given that arthritic conditions may have varying origins in patients, particularly patients with different genetic backgrounds, different epitopes of the P₁ portion of the conjugate may have varying efficacies in patient treatment. The conjugates may be used in a modular way, either as combinations, sequential or alternating administrations.

### Use of L.E.A.P.S. treated DCs and/or T cells upon further processing to have improved therapeutic benefit or additional diagnostic properties

In certain embodiments, DCs matured with, and/or T cells treated with the immunomodulators disclosed herein can have an ability to locate or "target" to a site in the subject's body harboring a source of the antigen and or the site of an autoimmune condition. The matured DCs, and/or activated T cells, with the immunomodulators can be used to diagnose or determine the presence or location in the body of an autoimmune response, allergic response or asthma in a subject to which the matured DCs, and/or activated T cells, are administered. The immunomodulators can be conjugated with a drug, dye or radionuclide (including ¹⁸F, ³²P, ⁶⁴Cu, ⁹⁰Y, ^{99m}Tc, ¹³¹I, ¹²⁵I, ¹²⁴I, ⁸⁹Zr, ¹¹¹In, ¹⁸⁸Re, or ¹⁷⁷Lu) that co-locate with the matured DCs, and/or activated T cells. The location of the matured DCs, and/or activated T cells can then be determined through appropriate radiation-detection techniques to diagnose the presence and/or location of an autoimmune condition in the body of the subject.

In certain embodiments, the matured DCs, and/or activated T cells, can be secondarily conjugated or associated with a radionuclide (including ¹⁸F, ³²P, ⁶⁴Cu, ⁹⁰Y, ^{99m}Tc, ¹³¹I, ¹²⁵I, ¹²⁴I, ⁸⁹Zr, ¹¹¹In ¹⁸⁸Re, or ¹⁷⁷Lu) that locates to the location of an autoimmune or other undesirable immune response in the body of a subject to which the matured DCs, and/or activated T cells, are administered. The radionuclide can be conjugated to an antibody (mAb or mAbs for plural) to CDllc or MHC II located on the surface of the matured DCs. In the alternative, an antibody can have specificity to other cell surface markers including DEC-205, Dectin-1, DC-SIGN, and DC-LAMP for DCs, or TCRα, TCRβ, CD3, CD4, CD8, and OX-40 for T cells. In certain embodiments, and mAb binds to markers on the surface of DCs and/or T cells such that the binding of the mAb to the DCs does not alter the activation or changes induced by the peptide construct heteroconjugates described herein.

In certain embodiments, radiation from radioisotopes can be used for detection by X-ray sensitive films or instruments or related technologies. ¹¹¹In can be detected by single-photon emission tomography/computed tomography (SPECT/CT), and ⁹⁹Te, ²⁰¹Ti, and ⁸⁹Zr can be used to generate photon emission tomography (PET) images. Sources of radiation can be conjugated to the immunomodulator peptide used to mature the DCs, and/or activate the T cells, or can be conjugated to an mAb having specificity for the matured DCs, and/or for the activated T cells.

In certain embodiments, the matured DCs, and/or activated T cells, can be conjugated or associated with a therapeutic agent or drug. The therapeutic agent can co-locate to the site of an autoimmune response or other undesirable immune response together with matured DCs, and/or activated T cells, administered to a subject.

In certain embodiments, the matured DCs, and/or activated T cells, can be conjugated or associated with a dye agent. The dye agents such carboxyfluorescein succinimidyl ester (CFSE), Cy 5.5 or Alexa Fluor®, CMTPX (a red fluorescent dye), CMFDA (a green fluorescent dye) L.E.A.P.S. activated DCs, and/or activated T cells, can co-locate to the site of an autoimmune or other undesirable immune response to allow for the diagnostic detection or imaging of the site.

Examples of therapeutic compounds include monomethyl auristatin E, saporin, maytansinoid 1, ozogamicin, doxorubicin, emtansine, carboplatin, 5-fluorouracil, docetaxel, receptor tyrosine kinase inhibitors, phosphatidylinositol 3- kinase inhibitors, nomifensine, and irinotecan (CPT-11) as well as larger molecules such as interferon-alpha (IFNγ), staphylococcal enterotoxin A superantigen (SE-A), and staphylococcal enterotoxin B superantigen (SE-B) (using the chemical conjugating reagent N-succinimidyl 3-(2 pyridyldithio)propionate) [SPDP]. The use of L.E.A.P.S. heteroconjugate-treated DCs, and/or T cells, to deliver therapeutic agents can be particularly effective for addressing toxicity. For example, the antimitotic agent auristatin is 100-1000 times more potent than doxorubicin making auristatin highly toxic and not well-tolerated at therapeutic doses. By targeting such cytotoxic agents by conjugation with L.E.A.P.S. heteroconjugate-treated DCs, and/or T cells, the delivery of the cytotoxic agents to target cells is more specific, and requires lower drug amounts, thereby reducing toxicity and safety concerns.

The therapeutic agent, dye or radioisotope can also be conjugated to a L.E.A.P.S. heteroconjugate or to an mAb, or can be conjugated or linked to a lysosomatropic agent. A lysosomatropic agent is a weak organic base that can diffuse through membranes but will become protonated in the lysosome of a cell, where the protonated lysosomatropic agent is unable to diffuse through membranes and will, therefore, be trapped within the cell. Hydrophobic amines, including butylamine, spermidine, spermine, methylamine, and cyanine dyes (including those used for studying membrane potential or that are used as tracers in neurobiology) are examples of lysosomatropic agents. These lysosomatropic agents can be modified to be conjugated to a radioisotope or to a therapeutic compound (*e.g.* cytokines such as IFNγ, SE-B, SE-A or other molecules) by a cleavable linkage.

The failure of mature CD8 cells to simultaneously engage their TCR and CD8 co-receptor triggers an activation process that can begin with inhibition of CD8 gene expression through remethylation, and that concludes with up-regulation of surface Fas and Fas ligand and cellular apoptosis (Pestano GA et al., Inactivation of Misselected CD8 T Cells by CD8 Gene Methylation and Cell Death 1999, Science, 284:1187). If full engagement of certain coreceptors are not effected then an activation process occurs which can lead to apoptosis (*see also* Gogolak P et al., Collaboration of TCR-, CD4- and CD28-mediated signaling in antigen-specific MHC class II-restricted T-cells 1996, Immunol. Let. 54:135; Grakoui A et al., The Immunological Synapse: A Molecular Machine Controlling T Cell Activation 1999, Science, 285:221; Malissen B, Dancing the Immunological Two-Step 1999, Science, 285:207; Redpath S et al., Cutting Edge: Trimolecular Interaction of TCR with MHC Class II and Bacterial Superantigen Shows a Similar Affinity to MHC:Peptide Ligands 1999, J. Immunol., 163:6; Preckel T *et al.,* 1998 *supra*; Sambhara SR et al., Programmed cell death of T cells signaled by the T cell receptor and the alpha 3 domain of class I MHC 1991, Science, 252:1424; Kishimoto H et al., Strong TCR Ligation Without Costimulation Causes Rapid Onset of Fas-Dependent Apoptosis of Naive Murine CD4+ T Cells 1999, J. Immunol., 163:1817; Kubo M et al., CD28 Costimulation Accelerates IL-4 Receptor Sensitivity and IL- 4-Mediated Th2 Differentiation 1999, J. Immunol., 163:2432).

Therefore, a different approach can have a modulation not having a full sequence of events that causes disease: the construct binding in an antigen- specific manner with the antigenic epitope, but the TCBL binding to a site on another molecule associated with certain early events in the pathway that is responsible for full expression. The latter will occupy the space and cause an early event in the process of activation (such as Ca⁺⁺ flux, activation of various phosphatases, membrane migration events, such as "patching" or "capping," changes in RNA metabolism) but will not support the complete activation process (the ultimate tertiary complex of binding events (MHC, antigen TCR and CD4 [or CD8]) necessary for full activation). The complete activation process can be culminating in antigen-specific antibody or non antibody-mediated specific cytotoxic T lymphocyte activity, such as killing of infected or tumor cells, by acting on DNA synthesis, cell division, or cytokine secretion. This early binding can be of such strength that it does not disassociate to allow the cell surface rearrangement necessary for the full and normal sequence of modulatory events, such as proliferation or secretion of late cytokines (*e.g.,* Fas, TNFα or IFNγ) and thereby prohibiting events found in an autoimmune disease-associated pathway with complete T cell activation.

### L.E.A.P.S. peptide basic constructs

Disclosed are peptide constructs of the following formula (I):

P₁-x-P₂ (I)

where P₁ is a peptide associated with autoimmune disease and which will bind to an antigen receptor on a set or subset of T cells; P₂ is an immune response modifying peptide which will (i) cause a directed immune response by said set or subset of T cells to which the peptide P₁ is attached or (ii) bind to a T cell receptor which will cause said set or subset of T cells to which the peptide P₁ is attached to initiate, but not complete, an immune response causing said set or subset of T cells to undergo anergy and apoptosis; and x is a direct bond or linker covalently bonding P₁ and P₂.

Although not strictly limited, the peptide constructs may have as many as about 200 amino acids in sequence, preferably up to about 150 amino acids, and especially, up to about 100 amino acids. The minimum number of amino acids is also not strictly limited but usually each of the peptide components P₁ and P₂ will have at least about 4, preferably at least about 6, and more preferably at least about 8 or 9 amino acids in order to provide the appropriate epitope configuration for effectively binding to the appropriate site on the T cells of interest. Thus, the peptide constructs of this invention will usually contain from about 20 to about 100 or more amino acids.

The peptide constructs may be prepared using conventional solution phase condensation chemistry of smaller peptides prepared by solid phase peptide synthesis, provided however, that for constructs having more than about 40 amino acids, especially more than about 50 amino acids, it is usually convenient and preferred to prepare shorter segments and then link the shorter segments using well known techniques in solid phase peptide synthesis.

Alternatively, the peptide constructs of this invention may be prepared using well known genetic engineering methods. Further details on methods for producing the instant peptide constructs can be found in U.S. 5,652,342.

For example, it is known that amino acids can be nonpolar, hydrophobic while another group of amino acids are polar, uncharged amino acids that are hydrophilic. Still further, it is known that amino acids can be grouped as those that are polar, charged, hydrophilic amino acids further subdivided between acidic and basic amino acids. Table 1 describes the chemical and structural features along with the corresponding amino acids.

**Table 1**

| Chemical or Structural Feature | Amino Acid |
|---|---|
| Polar / hydrophilic | N, Q, S, T, K, R, H, D, E, (C, Y)* Non- |
| polar / hydrophobic | (G), A, V, L, I, P, Y, F, W, M, C |
| H-bonding | C, W, N, Q, S, T, Y, K, R, H, D, E |
| Sulfur containing | C, M |
| Charged at Neutral pH Negative/acidic at Neutral pH Positive/basic | D, E, Charged |
| | K, R, (H) |
| Ionizable | D, E, H, C, Y, K, R |
| Aromatic | F, W, Y, (H, no absorption) |
| Aliphatic | G, A, V, L, I, P |
| Forms covalent cross-link (disulfide bond) | C |
| Cyclic P | |

| | |
|---|---|
| *Note: Amino acids in parentheses have the indicated character to a limited extent. | |

### Reversal Sequences for L.E.A.P.S. Constructs

Embodiments also contemplate reversal sequences where the order of amino acids in Peptides P1, P2 as disclosed herein is reversed from N-terminus to C-terminus. For example, peptide J has the sequence DLLKNGERIEKVE (SEQ ID No. 32). The reversal sequence of SEQ ID No. 32 has the sequence from N-terminus to C-terminus of EVKEIREGNKLLD. The reversal sequence for any ICBL disclosed herein is envisioned for inclusion in a L.E.A.P.S. heteroconjugate as described herein. Further, the non-reversal sequence for an ICBL can be conjugated with an antigen sequence from Table 3 or with a reversal antigen sequence of those antigens.

Examples are DLLKNGERIEKVEGGGPEGKPGQEGKFGAIGPKGGT and DLLKNGERIEKVEGGGTGGKPGIAGFKGEQGPKGEP, where Peptide J (SEQ ID No. 32) is conjugated with the reversal sequence of SEQ ID No. 50 or the non-reversal sequence of SEQ ID No. 50, respectively. For example, the following sequences are disclosed: SEQ ID No. 50 can be TGGKPGIAGFKGEQGPKGEP, SEQ ID No. 56 can be TGGKPGIAGFKGEQGPKGEPGGGEVKEIREGNKLLD and SEQ ID No. 57 can be PEGKPGQEGKFGAIGPKGGTGGGEVKEIREGNKLLD. Further, the reversal sequence for an ICBL can be conjugated with an antigen sequence from Table 3 or with a reversal antigen sequence. Such L.E.A.P.S. heteroconjugates contemplated in certain embodiments include EVKEIREGNKLLDGGGPEGKPGQEGKFGAIGPKGGT and EVKEIREGNKLLDGGGTGGKPGIAGFKGEQGPKGEP, where the reversal sequence for Peptide J (SEQ ID No. 32) is conjugated with the reversal sequence of SEQ ID No. 50 or the non-reversal sequence of SEQ ID No. 50, respectively. For example, the following sequences are disclosed: SEQ ID No. 58 can be TGGKPGIAGFKGEQGPKGEPGGGDLLKNGERIEKVE and SEQ ID No. 59 can be PEGKPGQEGKFGAIGPKGGTGGGDLLKNGERIEKVE.

### Variants of L.E.A.P.S. peptide sequences by substitutions of homologous residues, structures or molecules

Improved versions of the peptide constructs are comprised of variables X₁ to X₁₇ substitutions where each of X₁ to X₁₇ describe a group of particular types of amino acids based on their features. Table 2 describes the amino acids for variables X₁ to X₁₇ wherein the amino acids for each have been selected based on their common chemical and structural features as shown in Table 1 for inclusion in the improved variants. Instances of X₂X₃ or X₃X₂ or X₂X₃ or X₃X₂ or X₃X₃ or X₂X₂ can be replaced by X₁₁ or by gamma amino butyric acid (GABA) wherein the common feature is that the amino function is on the γ-carbon and not the a-carbon. Instances of X₁X₁ or X₁X₃ or X₃X₁ or X₁X₂ or X₂X₁ can be replaced by X₁₅. Modifications for increased stability at the amino terminus by use of an acetyl or propionyl group, D glycine or D alanine or use of cyclohexylalanine at the amino terminus to reduce proteolysis are contemplated by the substitution of X₁₂. As shown in the improved variants of the sequence, X₁₂ can be present or not present on the sequence. It is noted that the sequences in computer readable form do not include X₁₂. However, the modification is present for all sequences where disclosed in the instant specification. Similarly, modifications by use of 5-aminopentanoic acid for replacement of lengths of 3 or 4 amino acids of X₂ and X₃ are also disclosed and represented as X₁₃ wherein the lengths of 3 or 4 amino acids of X₂ and X₃ can be replaced by 5-aminopentanoic acid. Although X₁₃ is not properly an amino acid, it is used as a place holder to indicate 5-aminopentanoic acid bonded to the adjacent or underlying amino acid for improved stability. X₁₄ is a variable selected from any of X₁, X₂, X₃, X₄, X₅, X₆, X₇₅ X₈, X₉, or X₁₀. Substitutions of Cit (citrulline) for R (arginine), or Hct (homocitrulline) for K (lysine) are respectively represented as X₁₆ and X₁₇. These substitutions are seen *in vivo* as post-translational modifications leading to autoimmune disease. The chemical properties of Cit and Hct can be sufficiently similar to be interchangeable in making substitutions. The X₁₆ and X₁₇ substitutions are defined to maintain coherence with the changes seen *in vivo.*

The conversion of arginine into citrulline is catalyzed by a family of proteins known as peptidyl arginine deaminases (PADs). Their role in the initiation of autoimmune diseases is not fully understood, but it is hypothesized that aberrant deamination of proteins leads to immune exposure to citrullinated epitopes, initiating the autoimmune response against self proteins. (Uysal, H. et al. Antibodies to citrullinated proteins: molecular interactions and arthritogenicity. Immunological reviews 233, 9-33 2010). The conversion of lysine to homocitrulline is a biochemical reaction of lysine with cyanate, a side-product of the urea cycle (Wang, Z. et al. Protein carbamylation links inflammation, smoking, uremia and atherogenesis. Nature medicine 13, 1176-84 2007). Although this reaction is not as common as the PAD catalysis, epitopes containing homocitrulline appear to be similarly implicated in autoimmune diseases (Mydel, P. et al. Carbamylation-dependent activation of T cells: a novel mechanism in the pathogenesis of autoimmune arthritis. Journal of Immunology 184, 6882-90 2010).

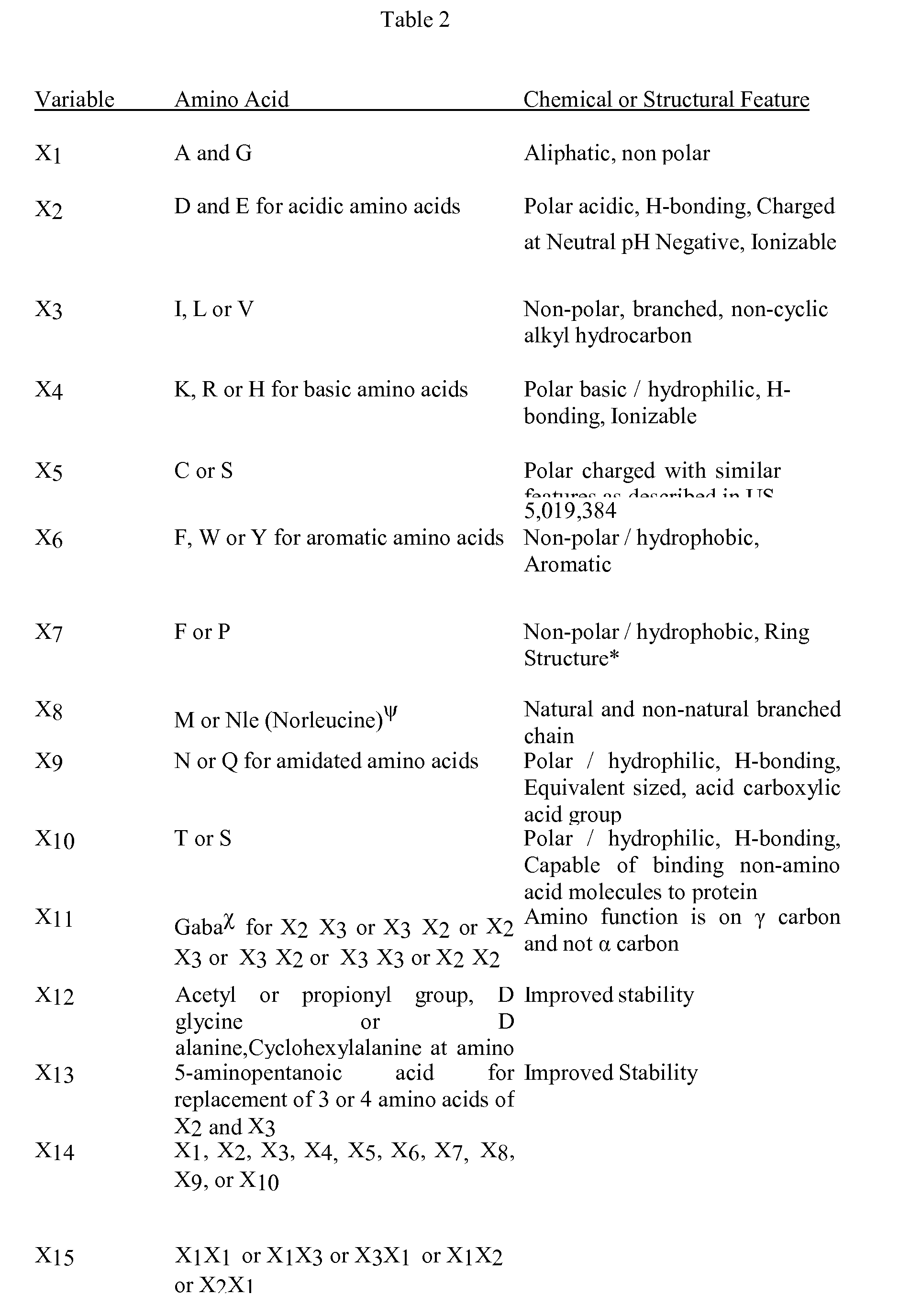

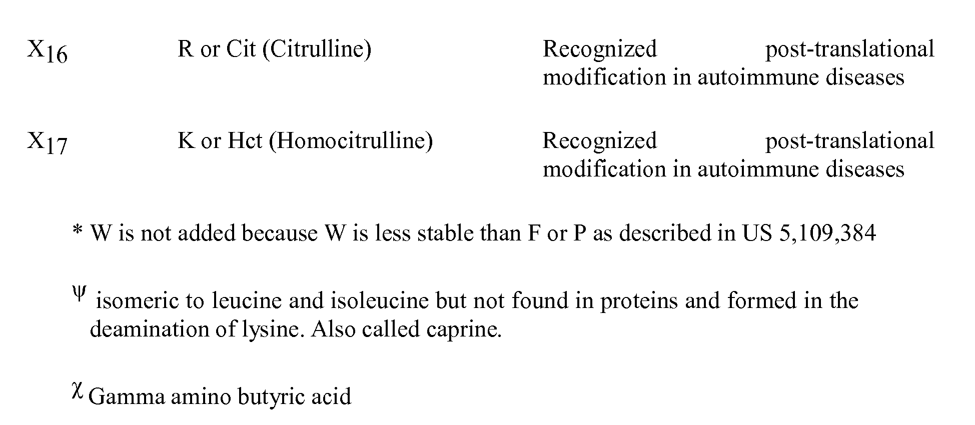

The novel peptide constructs of this invention can also be used to treat autoimmune disease such as rheumatoid arthritis (RA). In this case, the peptide antigen(s) used to make the construct would be antigens with defined epitopes recognized for human leukocyte antigens (HLA) genotypes associated with for example, RA, combined with a TCBL as described above to either suppress or redirect the immune response.

The peptide constructs of this invention may be used as activators of dendritic cells *ex vivo* for autologous cell therapy, directly or after further processing of the activated DCs for diagnosis/localization. They may also be used as therapy for patients with arthritis, formulated for *in vivo* use in prophylactic vaccines, or as therapeutic agents for treatment of autoimmune disease (e.g., rheumatoid arthritis or osteoarthritis). The vaccines may be administered with an adjuvant on a regular regimen such as weekly, biweekly, monthly, quarterly, semi-annually or annually by one of the following routes: intradermal (ID), intramuscular (IM) or subcutaneous (Sub-cu) and perhaps also as a cutaneous transdermal or nasal delivery vehicle in amounts of from 1-100, usually 10-50, micrograms per kilogram of body weight.

### Basic elements of L.E.A.P.S. constructs

### P₁ for rheumatoid arthritis

Proteoglycan PG molecules and other joint or tendon-related proteins, especially the molecules aggregcan and collagen type II, are thought to be major sources of human antigens associated with rheumatoid arthritis (RA) and osteoarthritis (OA).

The PG molecules, which include aggrecan (the sequence of which has been stripped of side chains of keratin and chondrotin sulfates) and those mentioned elsewhere, are shown in the following, non-limiting, representative examples, as well as in the literature references cited herein.

Table 3 shows exemplary antigens that can be employed as Peptide P₁ in certain embodiments. L.E.A.P.S. heteroconjugates consistent with Formula (I) can be formed by combining any permutation of ICBL peptide P₂ (*e.g.,* CEL-1000, Peptide J and/or Peptide G, IL-1β, etc.) with an antigen (Peptides P₁), as presented in Table 3. Table 3 lists antigen sequences implicated in RA or OA or disease models, including transgenic animals having human MHC sequences (REFERENCES FOR TABLE 3: (a) Brintnell, W et al. 2007 The influence of MHC class II molecules containing the rheumatoid arthritis shared epitope on the Immune response to aggrecan G1 and its peptides. Sc J Imm 65: 444-452; (b) de Jong, H et al. 2010 Cartilage proteoglycan aggrecan epitopes induce proinflammatory autoreactive T-cell responses in rheumatoid arthritis and osteoarthritis. Ann Rheum Dis. 69: 255-262; (c) Hanyecz, A et al. 2003 Induction of arthritis in SCID mice by T cells specific for the "shared epitope" sequence in the G3 domain of human cartilage proteoglycan. Arth & Rheum 48: 2959-2973; (d) Murad, YM et al. 2005 Molecular manipulation with the arthritogenic epitopes of the G1 domain of human cartilage proteoglycan aggrecan. Clin. And Exp. Imm. 142: 303-311; (e) Giant, TT et al. 2011 Proteoglycan-Induced Arthritis and Recombinant Human Proteoglycan Aggrecan G1 Domain-Induced Arthritis in BALB/c Mice Resembling Two subtypes of Rheumatoid Arthritis. Arthritis & Rheumatism 63: 1312-1321; (f) Protein Sequence Identification Number GI: 256017257, "The NCBI handbook [Internet]. Bethesda (MD): National Library of Medicine (US), National Center for Biotechnology Information; 2002 Oct. Chapter 18, The Reference Sequence (RefSeq) Project. Available from http://www.ncbi.nlm.nih.gov/protein/; (1) CDC Arthritis-Related Statistics (2011) at http://www.cdc.gov/arthritis/data_statistics/arthritis_related_stats.htm; (2) Van Vollenhoven, R. F. (2007). Switching between anti-tumour necrosis factors: trying to get a handle on a complex issue. Annals of the rheumatic diseases, 66(7), 849-51. doi:10.1136/ard.2007.0698721; (3) McInnes, I. B., & Schett, G. (2007). Cytokines in the pathogenesis of rheumatoid arthritis. Nature reviews. Immunology, 7(6), 429-42. doi:10.1038/nri2094; (4) Giant, T.T., & Mikecz, K. (2004). Proteoglycan Aggrecan-Induced Arthritis. Autoimmunity: methods and protocols, 102, 313. Retrieved from http://www.springerlink.com/index/r87547677026100u.pdf; (5) Giant, Tibor T, Radacs, M., Nagyeri, G., Olasz, K., Laszlo, A., Boldizsar, F., Hegyi, A., et al. (2011). Proteoglycan-induced arthritis and recombinant human proteoglycan aggrecan G1 domain-induced arthritis in BALB/c mice resembling two subtypes of rheumatoid arthritis. Arthritis and rheumatism, 63(5), 1312-21. doi:10.1002/art.30261; (6) Von Delwig, A., Locke, J., Robinson, J. H., & Ng, W.-F. (2010). Response of Th17 cells to a citrullinated arthritogenic aggrecan peptide in patients with rheumatoid arthritis. Arthritis and rheumatism, 62(1), 143-9. doi:10.1002/art.25064; (7) Zimmerman, D. H., Steiner, H., Carambula, R., Talor, E., & Rosenthal, K. S. (2012). L.E.A.P.S. Therapeutic Vaccines as Antigen Specific Suppressors of Inflammation in Infectious and Autoimmune Diseases. Journal of Vaccines & Vaccination, 03(05). doi:10.4172/2157-7560.1000149; (8) Zimmerman, D. H., Taylor, P., Bendele, A., Carambula, R., Duzant, Y., Lowe, V., O'Neill, S. P., et al. (2010). CEL-2000: A therapeutic vaccine for rheumatoid arthritis arrests disease development and alters serum cytokine/chemokine patterns in the bovine collagen type II induced arthritis in the DBA mouse model. International immunopharmacology, 10(4), 412-21. doi:10.1016/j.intimp.2009.12.016; and (9) Rodeghero R, Cao Y, Olalekan SA, Iwakua Y, Giant TT, et al. (2013) Location of CD4+ T Cell Priming Regulates the Differentiation of Th1 and Th17 Cells and Their Contribution to Arthritis. J Immunol. Available: http://www.ncbi.nlm.nih.gov/pubmed/23630349).

Specifically, the first column of Table 3 lists the SEQ ID No. for the sequence presented in each row. The second column specifies the protein from which individual amino acid sequences are derived. The third column gives the abbreviation to which the sequence presented in each row can be referred. For example, PG70 stands for proteoglycan aggrecan (G1) residues 70-84, whereas J-PG70 indicates a L.E.A.P.S. heteroconjugate having the ICBL Peptide J linked to PG70 (residues 70-84). The fourth column specifies the core epitope sequence, if any, for the protein described in each row. The fifth column indicates the range of amino acids from the described protein corresponding to the epitope sequence. The sixth column specifies an exemplary L.E.A.P.S. heteroconjugate with the ICBL Peptide J (DLLKNGERIEKVE [SEQ ID No. 32]) linked to one of the described antigens through a triglycine linker. Those skilled in the art will recognize that other L.E.A.P.S. heteroconjugate constructs can be formed substituting for peptide P₁ and peptide P₂, where the examples presented in Table 3 are merely illustrative and are not limiting. The last column lists references describing the epitope sequence. References are specified by a number corresponding to the list found at the end of this disclosure.

**Table 3: PG-Related Antigens and Example L.E.A.P.S. Heteroconjugates with Cit/Hct Variants**

| **Seq ID) No.** | **Protein Candidates** | **abbreviation** | **core epitope** | **amino acid position** | **J LEAPS conjugate** | Refer ence |
|---|---|---|---|---|---|---|
| | | | | | | |
| 1 | Proteoglycan Aggrecan (G1) | PG16 | QPSPLRVLLGTSLTIP | 16-31 | NA | 2,6 |
| 2 | | J-PG16 | NA | NA | DLLKNGERIEKVEGGGQPSPLRVLLGTSLTIP | |
| | | | | | | |
| 3 | Proteoglycan Aggrecan (G1) | PG16A | QPSPLRVLLGTSLTIPCYFIDPMH | 16-39 | NA | 2,6 |
| 4 | | J-PG16A | NA | NA | DLLKNGERIEKVEGGGQPSPLRVLLGTSLTIPCYFIDPMH | |
| | | | | | | |
| 5 | Proteoglycan Aggrecan (G1) | PG25 | GTSLTIPCYFIDPMH | 25-39 | NA | 2,6 |
| 6* | | J-PG25 | NA | NA | DLLKNGERIEKVEGGGTSLTIPCYFIDPMH | |
| | | | | | | |
| 7 | Proteoglycan Aggrecan (G1) | PG25A | GTSLTIPCYFIDPMHPVTTAP | 25-45 | NA | 2,6 |
| 8* | | J-PG25A | NA | NA | DLLKNGERIEKVEGGGTSLTIPCYFIDPMHPVTTAP | |
| | | | | | | |
| 9 | Proteoglycan Aggrecan (G1) | PG49 | PLAPRIKWSRVSKEK | 49-63 | NA | 4,6 |
| 10 | | J-PG49 | NA | NA | DLLKNGERIEKVEGGGPLAPRIKWSRVSKEK | |
| | | | | | | |
| 11 | Proteoglycan Aggrecan (G1) | PG65 | VVLLVATEGRVRVNSAYQDKV | 65-85 | NA | 2,6 |
| 12 | | J-PG65 | NA | NA | DLLKNGERIEKVEGGGWLLVATEGRVRVNSAYQDKV | |
| | | | | | | |
| 13 | Proteoglycan Aggrecan (G1) | PG70 | ATEGRVRVNSAYQDK | 70-84 | NA | 4,6 |
| 14 | | J-PG70 | NA | NA | DLLKNGERIEKVEGGGATEGRVRVNSAYQDK | |
| | | | | | | |
| 15 | Proteoglycan Aggrecan (G1) | PG155 | ACLQNSAIIATPEQL | 155-169 | NA | 4,6 |
| 16 | | J-PG155 | NA | NA | DLLKNGERIEKVEGGGACLQNSAIIATPEQL | |
| | | | | | | |
| 17 | Proteoglycan Aggrecan (G1) | PG143 | TRYTLDFDRAQRA | 143-155 | NA | 1,6 |
| 18 | | J-PG143 | NA | NA | DLLKNGERIEKVEGGGTRYTLDFDRAQRA | |
| | | | | | | |
| 19 | Proteoglycan Aggrecan (G1) | PG182 | AGWLADQTVRYPI | 182-194 | NA | 1,6 |
| 20 | | J-PG182 | NA | NA | DLLKNGERIEKVEGGGAGWLADQTVRYPI | |
| | | | | | | |
| 21 | Proteoglycan Aggrecan (G1) | PG263 | TTGHVYLAWQAGMDMCSAGW | 263-282 | NA | 2,6 |
| 22 | | J-PG263 | NA | NA | DLLKNGERIEKVEGGGTTGHVYLAWQAGMDMCSAGW | |
| | | | | | | |
| 23 | Proteoglycan Aggrecan (G1) | PG265 | GHVYLAWQAGMDMCSA | 265-280 | NA | 2,6 |
| 24 | | J-PG265 | NA | NA | DLLKNGERIEKVEGGGGHVYLAWQAGMDMCSA | |
| | | | | | | |
| 25 | Proteoglycan Aggrecan (G1) | PG280 | AGWLADRSVRYPI | 280-292 | NA | 1,6 |
| 26 | | J-PG280 | NA | NA | DLLKNGERIEKVEGGGAGWLADRSVRYPI | |
| | | | | | | |
| 27 | Proteoglycan Aggrecan (G3) | PG2373 | TTYKRRLQKRSSRHP | 2373-2387 | NA | 3,6 |
| 28 | | J-PG2373 | NA | NA | DLLKNGERIEKVEGGGTTYKRRLQKRSSRHP | |
| | | | | | | |
| 29 | Proteoglycan Aggrecan (G3) | PG2379 | LQKRSSRHPRRSRPST | 2379-2394 | NA | 2,6 |
| 30 | | J-PG2379 | NA | NA | DLLKNGERIEKVEGGGLQKRSSRHPRRSRPST | |
| | | | | | | |
| 50 | Type II Collagen | CII254 | TGGKPGIAGFKGEQGPKGEP | 254-273 | NA | |
| 47 | | J-CII254 | NA | NA | DLLKNGERIEKVEGGGTGGKPGIAGFKGEQGPKGEP | |
| 52 | | J-CII254-Hct | NA | NA | DLLKNGERIEKVEGGGTGGKPGIAGF(Hct)GEQGPKGEP | |
| | | | | | | |
| 53 | Type II Collagen | CII350 | PGLPGARGLTGRPGDAGPQG | 350-369 | NA | |
| 54 | | J-CII350 | NA | | DLLKNGERIEKVEGGGPGLPGARGLTGRPGDAGPQG | |
| 55 | | J-CII350-Cit | NA | NA | DLLKNGERIEKVEGGGPGLPGA(Cit)GLTGRPGDAGPQG | |
| | | | | | | |
| *As peptide 5 and 7 start with a G residue the common practice of having 3 G on the TCBL before the antigenic peptide could result in a 4 G sequence. In several instances in synthesis and purification this has resulted in problems, so the practice has been to delete and only have 3 G in tandem sequence before another amino acid residue is used. We show the conjugates 6 and 8 respectively with only 3 G and not 4 G at this juncture | | | | | | |

As mentioned, one antigen with defined epitopes recognized for rheumatoid arthritis (RA) is the proteoglycan aggrecan (G1) including the peptide from position 70-84 (Murad, YM *et al.,* 2005 *supra*).

For example, the proteoglycan aggrecan (G1) 70-84 (PG70) is shown below.
ATEGRVRVNSAYQDK (SEQ ID NO. 13)

The improved variants of PG70 can be generated using the substitutions indicated in Table 2 and elsewhere in the text of this document.

Other various antigens, often with defined epitopes recognized for rheumatoid arthritis (RA), include proteoglycan aggrecan (G1) 263-282 (PG263), shown below.
TTGHVYLAWQAGMDMCSAGW (SEQ ID NO. 21)

The improved variants of PG70 can be generated using the substitutions indicated in Table 2 and elsewhere as described herein.

Another antigen for HIV is shown below.
YSVHQRIDVKDTKEALEKIEEEQNKSKKKA (SEQ ID NO. 48)

The improved variants of SEQ ID NO. 48 can be generated using the substitutions indicated in Table 2 and elsewhere as described herein.

### P₂ or TCBL epitopes

One TCBL known to re-direct the Th2 to Th1 pathway is peptide "J." The peptide "J" can be conjugated to a specific antigen wherein the conjugated construct can direct the immune response to the peptide antigen toward the Th1 pathway. One example of such a construct is the L.E.A.P.S. construct.

Similarly, the TCBL peptide "G" or the improved version "derG" can direct the immune response down the Th2 pathway. The directed response is based upon data from several systems using peptide antigens from HSV, HIV, malaria, TB and murine myosin for antigen-specific induction of antibody isotypes, DTH, and cytokine secretion (IL2, IL4 and IFNγ). The basis for the directed response can be also founded on observed protection upon pathogen challenge as well as immune cell types evaluated to demonstrate effects of treatment with L.E.A.P.S. constructs.

As shown previously, one TCBL sequence contemplated in the disclosed L.E.A.P.S. construct is the peptide "G" NGQEEKAGVVSTGLI shown by SEQ ID NO. 35.

The improved variants of peptide "G" can be generated using the substitutions indicated in Table 2 and elsewhere in the text of this document.

One variant of the peptide "G" is the compound known as "derG" or CEL-1000, and has the sequence DGQEEKAGVVSTGLI (SEQ ID NO. 36).

The improved variants of peptide "derG" can be generated using the substitutions indicated in Table 2 and elsewhere in the text of this document.

Other TCBLs and spacers are also generated using the substitutions indicated in Table 2 and elsewhere in the text of this document.

The peptide J is shown by SEQ ID NO. 32.
DLLKNGERIEKVE (SEQ ID NO. 32)

The improved variants of peptide "J" can be generated using the substitutions indicated in Table 2 and elsewhere in the text of this document.

TCBLs can be selected from, but are not restricted to, LFA-3 or FasL described above (Lie BL et al., Synthesis and biological activity of four kinds of reversed peptides 1996, Biol. Pharma. Bul. 19:1602; or Tomita Y et al., Tetrapeptide DEVD-aldehyde or YVAD chloromethylketone inhibits Fas/Apo-1(CD95)-mediated apoptosis in renal-cell-cancer cells 1996, Int. J. Can. 68:132), or Hu IL-10 DNQLLETCKQDRLRNRRGNGSSTHFEGNLPC (SEQ ID NO. 37) (Gesser B et al., Identification of functional domains on human interleukin 10 1997, PNAS 94:14620; Lie BL *et al.,* 1996 *supra;* and Tomita Y *et al.,* 1996 *supra),* and may be selected for redirection of immune responses.

As activated T cells normally express MHC molecules, another method of immunomodulation is to take advantage of the programmed pathway established by antigen addition. T cells which receive a signal from both their TCR and MHC I (via CD-8⁺ cells) will undergo apoptosis without other costimulatory signals (Sambhara SR *et al.,* 1991 *supra).* Therefore, the TCBL, peptide E DQTQDTEGGC (SEQ ID 39), the α3 domain (amino acids 223-229) of the human MHC I conserved region, can be used along with the autoimmune epitope to form a peptide construct according to another embodiment.

If a binding site is known on the target T cell with a defined amino acid sequence and the TCBL amino acid sequence on the ligand is not known, then determination of the DNA encoding for the peptide can allow for determination of the cDNA and in turn the complementary peptide sequence using the technique of Lie BL *et al.,* 1996 *supra,* for example. In general, the TCBLs (P₂) used to form the disclosed peptide constructs will be selected from those that normally induce and/or modulate immune responses. This includes those selected to effect redirection from Th1 or Th2, for example peptides that are known to be related and involved in the normal events of activation, namely: IL-1β, IL-2, IL-10, IL-12, IL-4. IL-1β has been extensively studied as such a peptide (VQGEESNDK (SEQ ID NO. 38) (Bajpai K et al., Immunomodulating activity of analogs of noninflammatory fragment 163-171 of human interleukin-1beta 1998 Immunopharmacology 38:237; Beckers W et al., Increasing the immunogenicity of protein antigens through the genetic insertion of nucleotides coding for VQGEESNDK sequence of human IL-1 beta into their sequence 1993, J. Immunol. 151:1757; Fresca D et al., In vivo restoration of T cell functions by human IL-1 beta or its 163- 171 non-apeptide in immunodepressed mice 1988, J. Immunol. 141:2651; Antoni G et al., A short synthetic peptide fragment of human interleukin 1 with immunostimulatory but not inflammatory activity 1986, J. Immunol. 137:3201). Other examples of TCBLs are associated with earlier activation events include, for example, Fas and FasL, TNFα and TNFαR and those for formation of early intermediate complexes and LFA-3 and CD2.

Examples of TNF peptides known to activate macrophages are amino acids 70-80, as shown in SEQ ID NO. 33 (Britton WJ *et al.,* 1998 *supra*).
PSTHVLITHTI (SEQ ID NO. 33)

The improved variants of SEQ ID NO. 33 above can be generated using the substitutions indicated in Table 2 and elsewhere in the text of this document.

The antagonist peptide of another region of TNF peptides is shown by SEQ ID NO. 34 (Chirinos-Rojas CL *et al.,* 1998 *supra*).
DFLPHYKNTSLGHRP (SEQ ID NO. 34)

The improved variants of SEQ ID NO. 34 above can be generated using the substitutions indicated in Table 2 and elsewhere in the text of this document.

Still further, the TCBLs disclosed can be selected from any of the sequences above but are not restricted to just the LFA-3 or FasL sequences described above (Lie BL *et al.,* 1996 *supra;* or Tomita Y *et al.,* 1996 *supra*).

For example, another TCBL can be the Hu IL-10 aa₁₅₂₋₁₆₀ shown by SEQ ID NO. 49.
AYMTMKIRN (SEQ ID NO. 49)

The TCBL portion of the disclosed immunomodulatory peptide construct, *i.e.,* P2, may comprise a discontinuous epitope composed of two small regions separated by a loop or by a single chain short peptide in place of the loop (Shan D et al., Characterization of scFv-Ig Constructs Generated from the Anti-CD20 mAb 1F5 Using Linker Peptides of Varying Lengths 1999, J. Immunol. 162:6589). For example, an eight-amino acid group, LRGGGGSS (SEQ ID NO. 40), of 11.2 ångströms (11.2 x 10⁻¹⁰m) in length (Reineke U et al., A synthetic mimic of a discontinuous binding site on interleukin-10 1999, Nature Biotechnology 17:271) has been used to form a single peptide from two smaller discontinuous peptides of IL-10, thereby forming a TCBL which could be used for redirection from a Th1 to a Th2, in combination with, for example, the IDDM, PV or MS inducing epitopes (Hammer J *et al.,* 1997 *supra;* Tisch R *et al.,* 1999 *supra*).

### Variable linker spacer sequences

The improved versions of many of the sequences can contain multiple glycine chains, 3 to 6 glycines in length. In particular, if coupling two peptides, carbodiimides are commonly used to modify proteins' carboxylate groups. They react with proteins at a pH of around 5 to 6 and can be used either alone or in combination with amines to create new amide bonds off the carboxyl. The reaction producing various new adducts are well known within the art.

For example, a triglycine with or without one or two serines to separate two portions of the conjugate can be made by carbodiimide modification. The triglycine with a single serine is shown by the SEQ ID NO. 41.
GGGS (SEQ ID NO. 41)

For a trigylcine with two serines, the spacer is shown by SEQ ID NO. 42.
GGGSS (SEQ ID NO. 42)

For four glycines with a single serine, the spacer is shown by SEQ ID NO. 43.
GGGGS (SEQ ID NO. 43)

Sequences containing 4G without other modifications, such as addition of a serine or other amino acid can influence peptide folding. In several instances in synthesis and purification this 4G sequence has resulted in undesired folding, and so one of ordinary skill will understand to first examine the proposed two peptide ends to be linked and if using a 3G linker to delete 1G and only have 3G in a tandem sequence before another amino acid residue is used. Accordingly conjugates with only 3G and not 4G are disclosed herein.

An example of four glycines with two serines is shown by SEQ ID NO. 10.
GGGGSS (SEQ ID NO. 44)

An example of a spacer having three glycines interspaced with a single serine followed by three glycines and a serine is shown by SEQ ID NO. 11.
GGGSGGGS (SEQ ID NO. 45)

Other conventional linkages disclosed are direct linkages taught by the aforementioned U.S. 5,652,342. Examples of bifunctional linkers which can be employed to covalently link the T cell-specific binding ligand and antigen associated with disease, a causative agent of disease, or epitope thereof, include: N-succinimidyl-3-(2-pyridyldthio) propionate (hereinafter "SPDP") (Pharmacia, Piscataway, N.J.), which activates and allows formation of a bridge between two sulfhydryl groups of cysteines or a bridge between a derivatized (propinated-thiolyated) primary amino group and a cysteine; m-maleimidobenzoyl-N-hydroxy-succimide ester (hereinafter "MBS") (Pierce Chemical, Rockford, Ill.), which activates an amino group and then couples by a sulfhydryl group to a cysteine sulfydryl so as to form a disulfide bond between the two polypeptides; and 1-ethyl-3-(3- dimethylaminopropyl)carbodiimide (hereinafter "EDC") (Pierce Chemical, Rockford, Ill.), which can cross-link two polypeptides by sequentially activating the carboxyl group of one polypeptide and then adding such to an amino group of another polypeptide. N-isocyano-ethylmorphlin, bis-diazotized-benzidine, benzoquone and glutaraldehyde, which are other reagents commonly employed to link polypeptides, can be employed and are available from Pierce Chemical, Rockford, Ill.; Eastman Kodak Chemicals, Rochester, N.Y.; Serva, Westbury, N.Y.; Sigma Chemical Co., St. Louis, Mo.; and E. Merck, Darnstadt, West Germany (Briand JP et al., Synthetic peptides as antigens: pitfalls of conjugation methods 1985, J. Immunol. Meth. 78: 59; Kitagawa T et al., Enzyme coupled immunoassay of insulin using a novel coupling reagent 1976, J. Biochem. 79: 233; Liu FT et al., New procedures for preparation and isolation of conjugates of proteins and a synthetic copolymer of D-amino acids and immunochemical characterization of such conjugates 1979, Biochem. 18: 690; Ternynck T et al., Conjugation of p-benzoquinone treated enzymes with antibodies and Fab fragments 1977, Immunochem. 14: 767; and Drevin H et al., Covalent coupling of proteins to erythrocytes by isocyanide. A new, sensitive and mild technique for identification and estimation of antibodies by passive hemagglutination 1985, J. Immunol. Meth. 77: 9).

One example of a conventional hetero functional linker is shown by an eight-amino acid group:
LRGGGGSS (SEQ ID NO. 40)

Disclosed is formation of a single peptide from two smaller discontinuous peptides of IL-10, 11.2 ångströms in length, to form a TCBL to redirect from a Th1 to a Th2 response in combination with, for example, the IDDM, PV or MS-inducing epitopes (Reineke U *et al.,* 1999 *supra*).

It is also noted that any of the linkers disclosed herein may result in a tertiary structure which might be of use to form a more avid TCBL.

### Description of proteoglycan (PG) induced arthritis (PGIA) animal model

There are various rodent models of arthritis which replicate several clinical, immunological, or histopathological features of the disease in humans. In selecting the model for study and evaluation of therapies, it is important to select a model having as many features in common with the human condition as possible, including target organs and tissues, such as limb joints (feet, hands ankles, wrists, long bones of the arms and legs), and whether these features are bilaterally symmetrically distributed should also be taken into account. Natural history can also important such as sexual proclivity, maturity level (*e.g*., adolescent, mature adult or post mature), proteins implicated, and aggressiveness of disease progression. One of ordinary skill will understand that no single model fits all these criteria in mimicking the human condition. Furthermore, it will be understood that several models sacrifice some of these criteria in order to have a more homogenous population response and more rapid disease progression in order to conduct more studies with smaller group sizes. Rat models (CIA, AA, AIA, SCW) tend to be characterized as more aggressive (faster developing), requiring a single inducing event and agent (either antigen of complete Freund's adjuvant (CFA)), bilateral symmetrical in distribution (on both sides) and more distinctive in histology from human disease (rheumatoid arthritis) than mouse (murine) CIA and PGIA models (Bolon B et al., Rodent preclinical models for developing novel anti-arthritic molecules: Comparative biology and preferred methods for evaluating efficacy 2011, J. Biomed. & Biotech).

Several proteins, including some found in cartilage and joints, have been implicated in the disease process, whether as initiators or co-initiators, or as early targets including collagen type IV and various proteoglycans, perhaps in a masked form. Other proteins, such as derivatized albumin or (self or foreign) heat shock proteins have been used to induce arthritic conditions but are less likely to be the primary initiator of disease.

First described in 1980, the proteoglycan-induced arthritis (PGIA) model is similar to several other antigen-induced arthritis models in that immunization with a molecular component of tissue cartilage, such as type II collagen or proteoglycan, is the method of disease induction, and that development of disease is based upon cross-reactive immune responses between the immunizing non-self and self antigens (Mikecz K et al., Immunity to cartilage proteoglycans in BALB/c mice with progressive polyarthritis and ankylosing spondylitis induced by injection of human cartilage proteoglycan 1987, Arthritis Rheum. 30: 306; Giant TT et al., Variations in susceptibility to proteoglycan-induced arthritis and spondylitis among C3H substrains of mice: evidence of genetically acquired resistance to autoimmune disease 2001 Arthritis Rheum. 44: 682; Giant TT et al., Critical role of glycosaminoglycan side chains of cartilage proteoglycan (aggrecan) in antigen recognition and presentation 1998 J. Immunol. 160: 3812).

In the PGIA model, the aggrecan protein core of human cartilage proteoglycan is stripped of its molecular side-chains and used to induce disease; aggrecan from other species may be used, but may not produce optimal results (Giant TT et al., Experimental arthritis produced by proteoglycan antigens in rabbits 1980 Scand. J. Rheumatol. 9:271-27; Brand DD, Rodent Models of Rheumatoid Arthritis 2005 Comparative Medicine 55: 114-122.; Giant TT et al., Proteoglycan Aggrecan-Induced Arthritis 2004 Methods in Molecular Medicine 102: 313-338). The aggrecan molecule has several domains, including three globular domains. The most antigenic components of the aggrecan protein have been found in the G1 domain, although some have been described in G3 (*see* Murad YM *et al.,* 2005 *supra;* de Jong H *et al.,* 2010).

Without being limited to any particular theory, the PGIA disease in BALB/c mice is a Th1 disease (*i.e.,* a disease involving a Th1 immune response), characterized by high levels of IFNγ and IL-12, no elevation of IL17 or role for Th17, and is ameliorated by the Th2 cytokine IL-4. This is in contrast to CIA in the DBA mouse model, which by these same criteria is a Th2 condition. However, it is unclear from the published literature whether the IL-12 is IL-12p40 or IL-12p70. This distinction can be important for the testing of L.E.A.P.S. conjugates, as prior work suggests that the ICBL peptide derG induces a Th2 response, while the peptide J induces a Th1 response by inducing DCs to produce IL-12p70. Further evidence suggests that BALB/c mice do not regulate IL-12 properly due to the IL-12 receptor beta 2 chain (IL12Rβ2) because of genetic differences in the sequence of the chains between different strains of mice (Gorham, J. D., Güler, M. L., Fenoglio, D., Gubler, U. & Murphy, K. M. Low dose TGF-beta attenuates IL-12 responsiveness in murine Th cells. Journal of Immunology 161, 1664-70 1998. Gorham, J. D. et al. Genetic mapping of a murine locus controlling development of T helper 1/T helper 2 type responses. Proceedings of the National Academy of Sciences of the United States of America 93, 12467-72 1996. Himmelrich, H. et al. In BALB/c mice, IL-4 production during the initial phase of infection with Leishmania major is necessary and sufficient to instruct Th2 cell development resulting in progressive disease. Journal of Immunology 164, 4819-25 2000. Peng, Y., Falck-Pedersen, E. & Elkon, K. B. Variation in adenovirus transgene expression between BALB/c and C57BL/6 mice is associated with differences in interleukin-12 and IFNγ production and NK cell activation. Journal of virology 75, 4540-50 2001.). However, prior work with J-ICBL conjugates has shown efficacy upon challenge in multiple mouse strains, including influenza and HSV in BALB/c mice. Initial studies may examine whether J-PG70 (Seq ID No. 14) or derG-PG70 (Seq ID No. 46) peptides display the most efficacy and longevity of effect in the PGIA model. Further distinctions may be made on the basis of manufacturing efficiencies and stability arising from chemical and physical characteristics of the J or derG-ICBL peptides.

The PGIA model differs from collagen-induced arthritis (CIA) in several key respects. Mouse strains vulnerable to CIA are not similarly susceptible to PGIA, and the reverse holds true also. PGIA also features some amount of spondylitis (inflammation of the vertebrae), which is not commonly seen in CIA but which is relevant to the human condition which may also involve spondylitis. When considering the animals' sex and maturity level, one of ordinary skill will understand that in humans, RA is most commonly found in females, more often in more mature or even post-menopausal women, which is analogous to the murine PGIA model which is most readily induced in mature BALB/c females (retired breeders). This is in opposition to the CIA model which is more readily induced in young male DBA mice (Giant *et al.,* 2004 *supra*). Also, as noted in the definitions, PGIA and RA are characterized by Rf and anti citrulline antibodies, whereas CIA is not.

The PGIA model can be divided into either a Th1 or Th17 disease phenotype depending on whether the disease is induced intraperitoneally (for a Th1 phenotype) or subcutaneously (for a Th17 phenotype) (Rodeghero, R. et al. Location of CD4+ T Cell Priming Regulates the Differentiation of Th1 and Th17 Cells and Their Contribution to Arthritis. J. Immunol. (2013). published online at http://www.ncbi.nlm.nih.gov/pubmed/23630349).

Both the CIA and PGIA mouse models can be relatively slow to develop and require multiple induction events (immunizations), with CIA requiring at least 2 immunizations (day 0 and 21) with use of adjuvant (Complete Freunds on day 0 and incomplete Freunds on subsequent immunizations (day 21 and occasionally day 42 or later in young (6-10 week old) male DBA/1J mice and the PGIA requiring 3 or 4 immunizations, Days 0, 21 and 42 with DDA adjuvant using BALB/c retired females). These multiple steps could be considered more like the inductive process in human RA, which develops most likely over many years in the older, more immunosuppressed individual, than the faster-developing (in days) rat arthritic diseases with a single induction event (Giant *et al.* 2004, *supra;* Brand *et al.* 2005, *supra*).

Another important distinction between the two models is the cytokines which characterize the immunological response to disease. At present, the CIA model is defined by a response involving increased TNFα, IL-1, and IL-17, while the PGIA model involves increased IFNγ, IL-4, and IL-12. These differences, if confirmed, may be important, as the variation in human arthritis, including response to treatment, may be reflected in the different aspects of these models (*see* Bevaart L et al., Evaluation of therapeutic targets in animal models of arthritis 2010, Arthritis & Rheumatism 62:2192).

The advantage of using several different antigen-induced arthritis models derives not just from the different clinical and immunological features, but also from the variation in human rheumatoid arthritis, osteoarthritis and spondylitis. Rheumatoid arthritis may be characterized by overproduction of pro-inflammatory and pro-erosive cytokines, either TNFα or IL-1, and abnormal recognition of self-antigens as non-self, reflecting a difference in the autoimmune response and requiring an adjustment of treatment (Van den Berg WB, Lessons from animal models of arthritis over the last decade 2009, Arthritis Research & Therapy 11: 250; Bolon B *et al.,* 2011 *supra*).

Giant also reports a model using recombinant human G1 domain as the inducing antigen in mice (GIA). This model features several advantages over the PGIA model, the most prominent being the acquisition of the immunizing antigen used in each model. The PGIA model requires human tissue cartilage, which must be heavily processed before the aggrecan can be used for animal studies. The GIA model utilizes recombinant protein obtained from a mammalian-expression system transfected into CHO-K1 cells, thus featuring a simpler purification scheme and fewer regulatory requirements. In addition, the GIA model features higher severity and incidence, while also progressing faster than the PGIA model. Most other features of the models remain similar, although autoantibody ratios to several RA factors were observed to be different between the two models (*see* Giant TT et al., Proteoglycan-induced arthritis and recombinant human proteoglycan aggrecan G1 domain-induced arthritis in BALB/c mice resembling two subtypes of rheumatoid arthritis 2011 Arthritis & Rheumatism 63: 1312-1321).

Evaluation of L.E.A.P.S. conjugates in multiple rodent models of arthritis is therefore necessary to best determine efficacy. Several studies have been done in a CIA model using a collagen conjugate, CEL-2000 (Seq. ID No. 47) (Zimmerman D *et al.,* 2010, *supra*). Evaluating the efficacy of candidate conjugates in the PGIA model should thus be done with both a collagen conjugate and proteoglycan conjugate (see table 3).

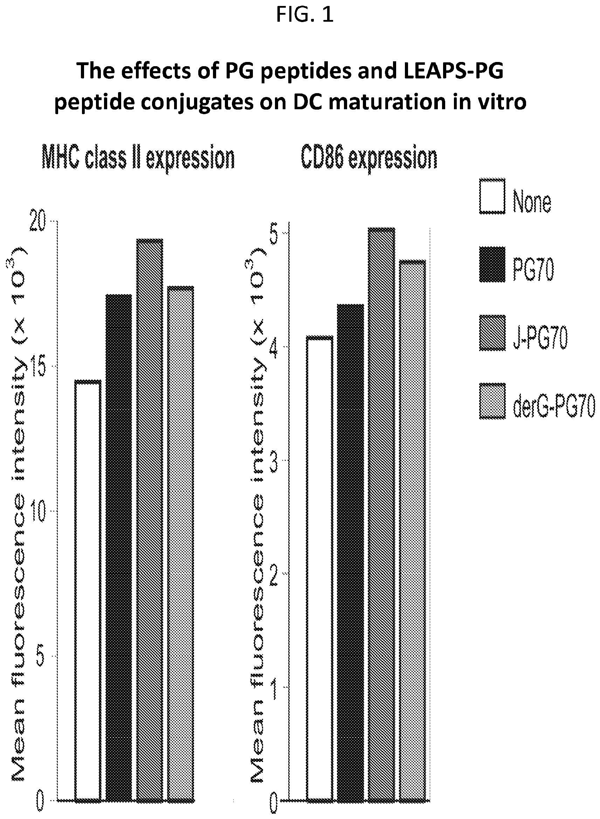

FIG. 1 illustrates the effects of PG and L.E.A.P.S.-PG conjugates on DC maturation as indicated by MHC class II and CD86 expression (left and right panels respectively) following treatment (24 hours). Bone marrow-derived DCs were treated with PG70, J-PG70, derG-PG70, PG70Cit (5 µM each, or 10-20ug/mL, based on previous work) (Murad YM, Szabó Z, Ludányi K, Giant TT (2005) Molecular manipulation with the arthritogenic epitopes of the G1 domain of human cartilage proteoglycan aggrecan. Clin Exp Immunol 142: 303-311; Misják P, Bősze S, Horváti K, Pásztói M, Pálóczi K, et al. (2013) The role of citrullination of an immunodominant proteoglycan (PG) aggrecan T cell epitope in BALB/c mice with PG-induced arthritis. Immunol Lett 152: 25-31) or untreated (media alone). MFI: mean fluorescence intensity (arbitrary units). In summary, single cell suspensions of bone marrow cells were prepared from femurs and tibias of both legs from separate naive (normal) retired breeder female BALB/c mice (n=2).

To generate DCs, the bone marrow cells were cultured for 9 days in the presence of recombinant mouse granulocyte-macrophage colony stimulating factor (GM-CSF, Sigma-Aldrich, St Louis, MO) at a dose of 200 U/ml (Egelston C., Kurko J., Besenyei T. et al. (2012) Suppression of dendritic cell maturation and T cell proliferation by synovial fluid myeloid cells from mice with autoimmune arthritis. Arthritis Rheum 64: 3179-3188). DCs were harvested, washed, and incubated for 24 hours with the various peptides indicated in 12 well plates at 10⁶ cells/mL. After the 24-hour culture with the indicated stimulants, the cells were washed with PBS containing 0.5% bovine serum albumin and 0.01% sodium azide (fluorescence activated cell sorter (FACS) buffer). Cells were stained with different fluorochrome-conjugated antibodies against CDllc (DC-specific marker), MHC class II (BALB/c haplotype-matched I-A^{d}/I-E^{d} molecules), and CD86 (co-stimulatory molecule) (all antibodies were from eBioscience, San Diego, CA). The labeled DCs were subjected to flow cytometric analysis using a FACS Canto II flow cytometer and FACS Diva software (BD Biosciences Immunocytometry Systems, San Jose, CA). Based on published findings (Taylor, P. R., Paustian, C. C., Koski, G. K., Zimmerman, D. H., and Rosenthal, K. S. (2010) Maturation of dendritic cell precursors into IL12-producing DCs by J-L.E.A.P.S. immunogens., Cellular immunology 262, 1-5; Taylor, P. R., Koski, G. K., Paustian, C. C., Bailey, E., Cohen, P. a, Moore, F. B.-G., Zimmerman, D. H., and Rosenthal, K. S. (2010) J-L.E.A.P.S. vaccines initiate murine Th1 responses by activating dendritic cells., Vaccine. Elsevier Ltd 28, 5533-42; Boonnak *et al., supra*) it was elected to focus on DC maturation markers CD86 and MHC class II, which are found at low densities on immature DC before differentiation induced by exposure to J L.E.A.P.S. conjugates. These results were reproduced independently for J-PG70 and PG70; the average for two animals is shown. The experiment demonstrates the ability of the J-L.E.A.P.S. conjugate to induce DC maturation, while the derG-L.E.A.P.S. conjugate does not induce this effect to the same degree.

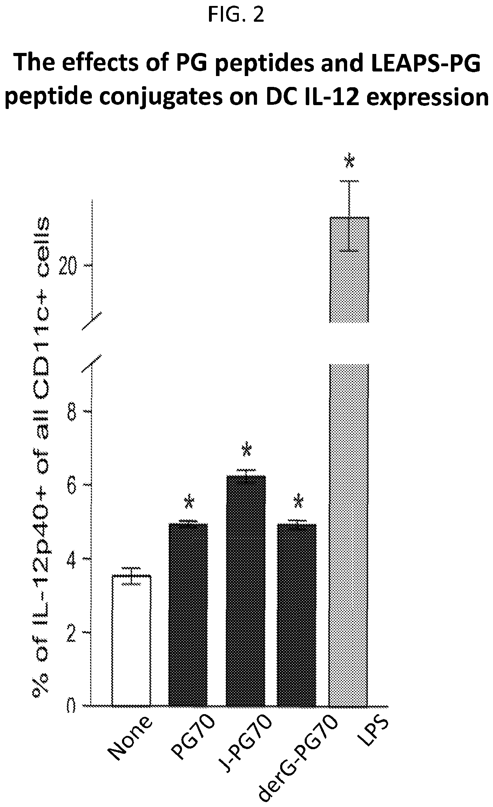

FIG. 2 illustrates the effects of L.E.A.P.S.-PG conjugates on intracellular expression of IL-12p40 by DCs. DCs from BALB/c female retired breeder normal mice (n=3), having no PGIA disease (generated from bone marrow as described under FIG. 1) were incubated for 24 hours with the indicated peptides (5 µM each) at a cell density of 1 x 10⁶ DCs/mL. DCs not receiving treatment (None) or treated with LPS (1 µg/ml) served as negative and positive controls, respectively. At the end of treatment, DCs were harvested and incubated with 1 µg/mL monensin (GolgiStop, BD Biosciences) for 4 hours to prevent cytokine secretion. Surface staining of DCs was done with fluoresecein isothiocyanate- (FITC) conjugated anti-CDllc antibody. The cells were then fixed and permeabilized using BD Biosciences' Cytofix/Cytoperm Kit. Intracellular IL-12 was detected with a phycoerythrin- (PE) labeled antibody to mouse IL-12p40 (eBioscience). Flow cytometry was carried out as described above. The number of IL-12p40+ DCs was determined by gating on CD11c+ cells, and the results expressed as the % of IL-12p40+ of all CD11c+ DCs. 20.7% of LPS treated cells showed intracellular IL-12p40, with J-PG70Cit being the second strongest inducer (7.3% positive cells vs. 3.8% in the untreated group). Significant differences (p<0.05) between the untreated and the treated DC cultures are denoted by asterisks. The error bars represent ±SEM.

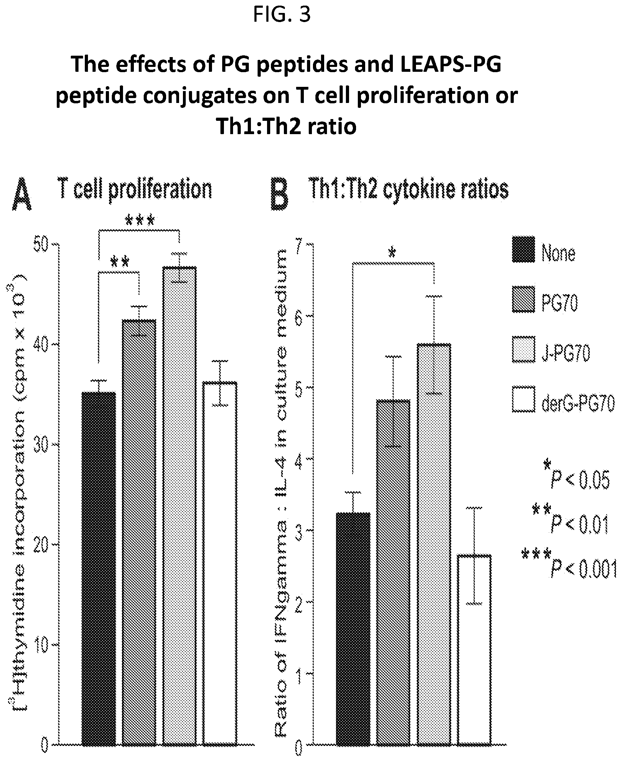

FIG. 3 illustrates T cell proliferation and T cell polarization (TH1:TH2) induced by P70, DerG-PG70 and J-PG70 peptides (Panel A). T cell proliferation rate (stimulation index, SI = T-cell proliferation induced by PG70 peptides divided by proliferation of unstimulated cultures) shows significant differences (Fishers LSD test; p<0.05, indicated by an asterisk) between untreated and treated PG70, PG70Cit, J-PG70Cit and J-PG70Cit (all SI similar levels) but no significant difference between untreated and DerG-PG70 or DerG-PG70Cit. Panel B) T cell polarization by Th1:Th2 cytokine (IFNγ:IL-4) ratios. Polarization of T cells indicates that they differentiate towards either a Th1 profile (accompanied by increased IFNγ secretion) or a Th2 response (accompanied by increased IL-4 secretion). It is noted that these two responses are mutually exclusive. Amounts of IFNγ and IL-4 in culture fluid samples were determined by ELISA (enzyme-linked immunosorbent assay, a common test that uses antibodies and color change to measure protein amounts). The "baseline" value for this ratio, *i.e.,* the value of this ratio in untreated (unpolarized) T cells, is around 3.2 (the black bar, "None," in FIG. 3B). An increased ratio indicates relatively more IFNγ and/or less IL-4, which in turn indicates a shift towards a Th1 response and away from a Th2 response. A decreased value (below 3.2) indicates relatively more IL-4 and/or less IFNγ □ and thus a shift towards a Th2 response and away from a Th1 response. Cells were isolated from spleens by gently pushing the organ through a wire cell sieve mesh screen with a sterile 5 cc syringe plunger. The spleens were from BALB/c retired female breeder mice with PGIA (n=3), cultured individually for 5 days in the absence (None) or presence of the different designated peptides (5 µM each) on PG protein (10µg). As has been previously shown, the only cell type incorporating tritiated [³H]thymidine into DNA are T cells. Therefore, proliferation of splenic T cells was measured in 96 well plates by use of [³H]thymidine (0.5 Ci/well; specific activity: 2 Ci/mmol, Amersham, Arlington Heights, IL) incorporation from day 4 to 5 and expressed as a stimulation index based on the counts per minute normalized to the untreated control (panel A). Treatments were done in triplicate wells for the proliferation assay, and identical triplicate wells were used to harvest culture fluid at 48 hours for cytokine assays. The amounts of IFNγ (a Th1 cytokine) and IL-4 (a Th2 cytokine) in the media of the same cultures were determined by ELISA and expressed as ratios.

J-PG70Cit induced a significant shift towards Th1, while cells treated with DerG-PG70 showed a significant shift towards Th2, versus untreated splenocytes (spleen cells) control. J-PG70 consistently induces higher T cell proliferation as compared to PG70 and even more so when compared to derG-PG70 (either uncitrullinated or citrullinated). There is a suggestion for citrullination to increase activity for both PG70 and J-PG70 treatment. Data shown are the mean ± SEM (n=3). (Boldizsar F Kis-Toth K Tarjanyi Olasz K Hegyi A Mikecz K Giant T 2010 Impaired Activation-Induced Cell Death Promotes Spontaneous Arthritis in Antigen (Cartilage Proteoglycan)-Specific T Cell Receptor-Transgenic Mice ARTHRITIS & RHEUMATISM 62:2984-2994).

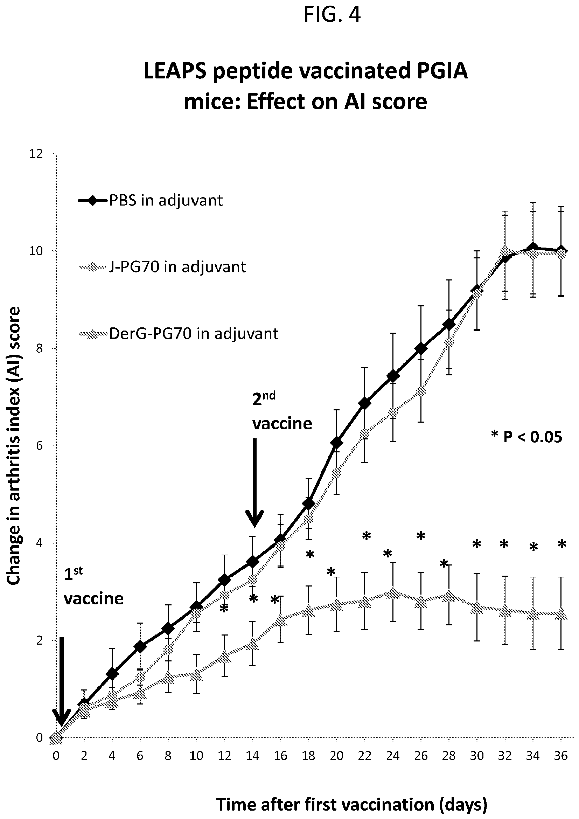

FIG. 4 shows the differences between the DerG-PG70 treated group from the other groups became statistically significant around day 12 and continued thereafter (p<0.05, repeated measures ANOVA; n=8 mice/group).

As demonstrated by the changes in AI scores, disease severity was dramatically suppressed in mice vaccinated with the L.E.A.P.S. conjugate DerG-PG70 as compared with controls or animals receiving J-PG70 vaccine.

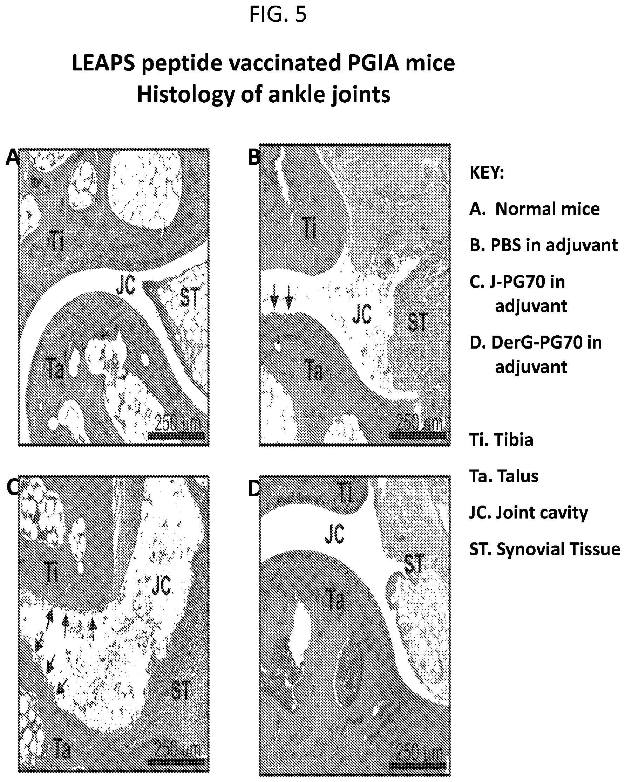

FIG. 5 shows representative sections of the same groups as in FIG. 4. Histology of ankle (tibio-talar) joints are shown in (A) normal mice and in (B-D) PGIA mice 36 days after undergoing treatment with L.E.A.P.S. conjugates. The normal joint is characterized by smooth cartilage surfaces on the tibia (*Ti*) and talus (*Ta*)*,* inflammatory cell-free joint cavity (*JC*) and synovial tissue (*ST*). The ST is lined by a thin layer of cells and contains mainly fat tissue. Ankle joints from mice with PGIA shown in (B), treated with PBS in adjuvant, show evidence of synovial thickening (hyperplasia), inflammatory cell accumulation in the JC and ST, as well as partial erosion of cartilage surface (arrows). Infiltration of both the ST and JC by inflammatory cells and cartilage erosions (arrows) as shown in (C) are even more severe in PGIA mice treated with the J-PG70 conjugate in adjuvant. In contrast, only mild ST hyperplasia as shown in (D), but no cell infiltration or cartilage damage is seen in mice treated with the DerG-PG70 conjugate in adjuvant.

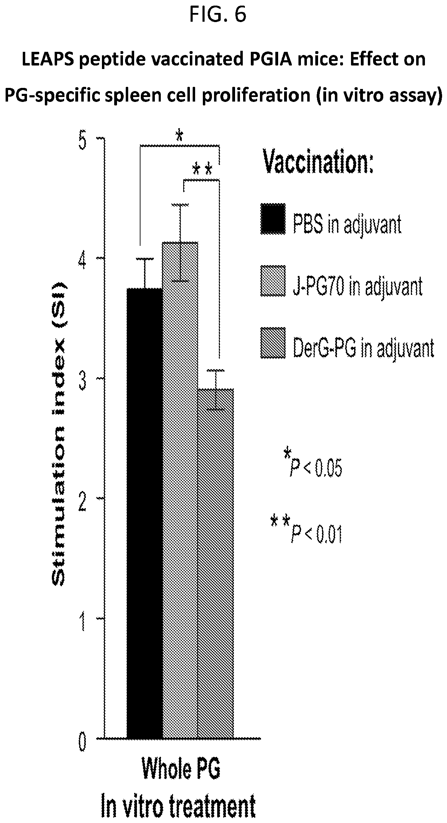

The joint sections were stained with hematoxylin and eosin. One representative section per group is shown. FIG. 6 shows L.E.A.P.S. peptide vaccinated PGIA mice and its effect on PG-specific spleen cell proliferation (*in vitro* assay) for the same groups as in FIG. 4. Protection was associated with reduced PG-specific T cell proliferation. Cells from the DerG-PG70 treatment group were much less responsive to either stimuli compared to other groups and DerG-PG70 by this assay was the least active.

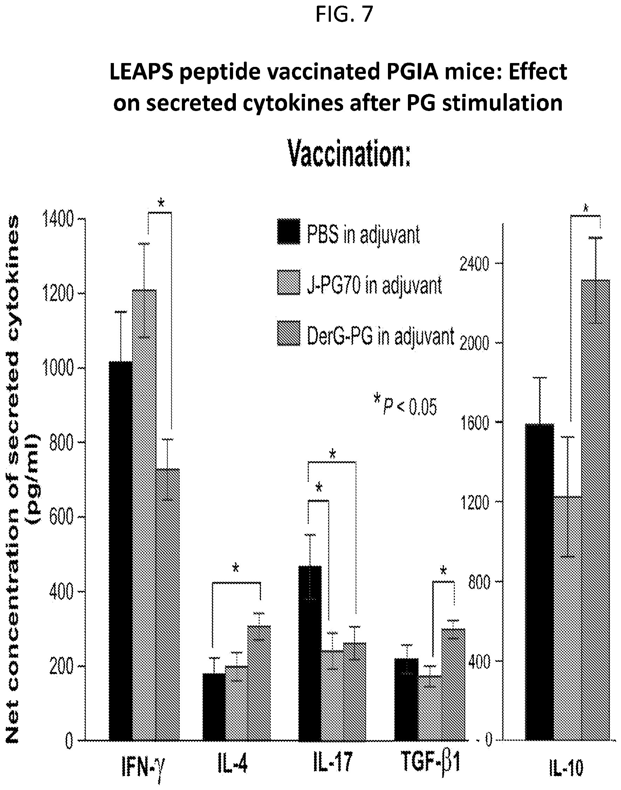

FIG. 7 shows L.E.A.P.S. peptide vaccinated PGIA mice and its effect on secreted cytokines after PG stimulation for the same groups as in FIG. 4. Protection was statistically significantly associated with increased IL-4 and IL-10 and reduced IL-17 production, as well as with increased TGFb and reduced IFN-g release (*p<0.05; Fisher's LSD test). IL-10 concentrations are shown on a separate scale as the magnitude of IL-10 production was much greater, perhaps due to secretion by non -T cells.

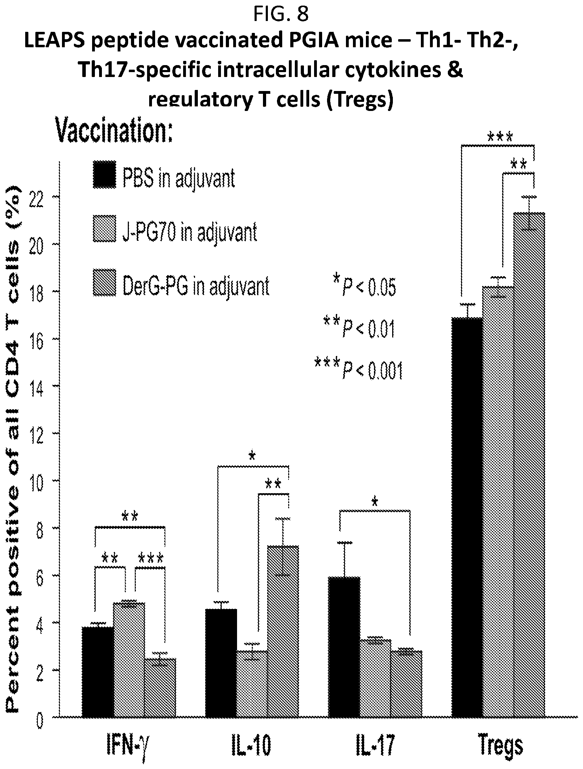

FIG. 8 shows the L.E.A.P.S. peptide vaccinated PGIA mice - Th1-, Th2-, Th17-specific intracellular cytokines & regulatory T cells (Tregs) for the same groups as in FIG. 4. The proportions of Th1, Th2, Th17, and regulatory T cells (Tregs) in spleen cell cultures from the vaccinated mice were determined by flow cytometry. Protection was associated with decreased proportions of Th1 and Th17 cells, and increases in Th2 cells and Tregs (p<0.05-0.001, Fisher's LSD test; n=4 mice/group). The DerG-PG70 vaccine is therapeutically effective in PGIA (a Th1 dominated disease) by inducing immune deviation in favor of Th2 and Treg cells. Protected animals showed a statistically significant increase in FoxP3⁺ and IL-10⁺ cells, with a decrease in IFN-γ⁺ and IL-17⁺ cells. This is similar to the pattern of cytokines secreted by the cells.

### Examples

The invention will now be described in further detail by way of representative examples; however, the present invention is not limited to the examples and should be construed to cover the subject matter as defined in appended claims.

### Prophetic Example 1

The major histocompatibility complex (MHC) can present a peptide (antigen) on the surface of an antigen-presenting cell (APC). This MHC-peptide complex can be recognized by the relevant T cell receptor (TCR) on the surface of a T cell, upon which the T cell and the APC associate with one another.

For this close association, additional factors are required, including T cell co-receptors CD4 or CD8, and receptor-ligand pairs such as LFA-1-ICAM-1. LFA-1 is also known as CD11a/CD18 and resides on the T cell surface while ICAM01, also known as CD54, resides on the APC surface. Within 30 seconds after initial contact between the APC and T cell, the MHC-peptide can complex and ICAM-1 molecules rearrange into a specific spatial organization. This spatial rearrangement on the APC can lead to a similar rearrangement of TCR and CD4 or CD8 on the T cell, and is a necessary step in its activation (Malissen B, 1999 *supra;* Grakoui A *et al.,* 1999 *supra*). According to one embodiment, such rearrangement is prevented by the close association in a peptide construct using a TCBL from ICAM-1, LFA-3 (aa26-42), VLWKKQKDKVAELENSE (SEQ ID NO. 31) (Osborn L et al., Amino acid residues required for binding of lymphocyte function-associated antigen 3 (CD58) to its counter- receptor CD2 1995, JEM, 181:429), by either the disparity in the temporal binding or higher strength of binding activity, thereby preventing the rearrangements and other interactions necessary for activation. Normally, rearrangements on the T cell surface occur during the activation process, so by preventing this shift activation should not occur. A TCBL from CD4 that binds to the TCR and CD3 may be used as the TCBL in this peptide construct. Its binding to the T cell recognition site will inhibit subsequent events from occurring (e.g., association of MHC II with CD4 or β-2 microglobulin with CD8).

### Prophetic Example 2

Another construct that can redirect the immune response initiated by the natural autoimmune inducing event from a Th1 to a Th2 response (*e.g*.: Lowrie DB et al., Therapy of tuberculosis in mice by DNA vaccination 1999, Nature, 400:269; Tisch R et al., Induction of glutamic acid decarboxylase 65-specific Th2 cells and suppression of autoimmune diabetes at late stages of disease is epitope dependent 1999, J. Immunol. 163:1178) is disclosed herein. As used herein, a Th2-directed response is one which directs the immune response toward the Th2 direction, thus favoring production of Th2-associated cytokines IL-4, IL-5, IL-10, IL-13, TNFα and antibody isotypes IgG1 in mice (or comparable in humans) as opposed to Th1, where the immune response favors production of cytokines IFNγ, IL-2, IL-6, IL-12 cytokines and antibody isotypes IgG2a and IgG2b in mice and cytotoxic T cell activity. Of note, a "Th2-directed response" implies a mixed (both Th1 and Th2) immune response which is skewed towards a Th2 profile, rather than a response that is exclusively of the Th2 profile.

According to this example of a TCBL associated with TH2 responses, the peptide conjugate can consist of peptides known to stimulate IL-4 or IL-5 synthesis, e.g., peptide G from MHC class II, along with the autoimmune inducing peptide (*e.g.* Hammer J *et al.,* 1997 *supra;* Krco CJ et al., Identification of T Cell Determinants on Human Type II Collagen Recognized by HLA-DQ8 and HLA-DQ6 Transgenic Mice 1999, J. Immunol. 163:1661; Araga S *et al.,* 1999 *supra;* Ota K *et al.,* 1990 *supra;* Ruiz PJ et al., Suppressive Immunization with DNA Encoding a Self-Peptide Prevents Autoimmune Disease: Modulation of T Cell Costimulation 1999, J. Immunol. 162:3336; Yoon JW et al., Control of Autoimmune Diabetes in NOD Mice by GAD Expression or Suppression in .beta. Cells 1999, Science 284:1183; Dittel BN et al., Presentation of the Self Antigen Myelin Basic Protein by Dendritic Cells Leads to Experimental Autoimmune Encephalomyelitis 1999, J. Immunol. 163:32; Gautam AM et al., A Viral Peptide with Limited Homology to a Self Peptide Can Induce Clinical Signs of Experimental Autoimmune Encephalomyelitis 1998, J. Immunol. 161:60; Zimmerman DH et al., A new approach to T cell activation: natural and synthetic conjugates capable of activating T cells 1996, Vacc. Res. 5:91, 5:102; Rosenthal K *et al.,* 1999 *supra*).

The described peptide constructs can be used, for example, to treat type I diabetes. In particular, an animal model the mechanism of diabetes prevention in the RIP-NP model was shown to be mediated by the insulin β-chain, and IL-4-producing, regulatory cells acting as bystander suppressors (Homann D *et al.,* 1999 *supra*). Such redirection of immune responses has previously been reported for a DNA vaccine for tuberculosis, which redirected the immune response from an (in the context of tuberculosis) inefficient Th2 response to a very effective Th1 response (Lowrie DB *et al.,* 1999 *supra*). Thus, redirecting an existing immune response from Th1 to Th2 would be effective for treating autoimmune diseases. A TCBL involved in CD28 co-stimulation (Kubo M *et al.,* 1999 *supra*) could also be effective for this purpose. If, on the other hand, the need is to redirect from a Th2 to a Th1 response, a TCBL such as peptide J, DLLKNGERIEKVE (SEQ ID NO. 32) (Zimmerman D *et al.,* 2005 *supra*) or peptides known to stimulate IL-2 or IL-12 synthesis would be used with the autoimmune inducing peptide.

### Prophetic Example 3

This example contemplates a peptide construct that does not activate the normal immune process, but activates the process leading to apoptosis of the T cell by using as the TCBL a ligand that binds to a site on the T cell whose normal binding and activation leads to apoptosis of the T cell. A specific example can be the TNFα -receptor of the T cell, in which the TCBL would be the TNFα ligand portion. Examples of such TNF peptides known to activate macrophages are amino acids 70-80 PSTHVLITHTI (SEQ ID NO. 33) (Britton WJ et al., A tumor necrosis factor mimetic peptide activates a murine macrophage cell line to inhibit mycobacterial growth in a nitric oxide-dependent fashion 1998, Infect. Immun. 66:2122) and the antagonist peptide represented by DFLPHYKNTSLGHRP (SEQ ID NO. 34) of another region (Chirinos-Rojas CL et al., A Peptidomimetic Antagonist of TNFα Mediated Cytotoxicity Identified from a Phage- Displayed Random Peptide Library 1998, J. Immunol. 161:5621). WO 99/36903A1 suggestes that the H4-1-BB ligand is useful as a treatment for autoimmune disease in a similar way to flt3-L and CD40L; therefore, H4-1BB may also be used as TCBL for inclusion with autoimmune antigens to form the inventive peptide construct. Other such TCBL examples are recited in applications involving Fas and Fas-ligand, including the noncleavable Fas-ligand (WO 99/36079A1). Representatives from another pair, IFNγ and the IFNγ ligand, can also be used as TCBLs in the peptide constructs.

In the present example, the antigenic peptide and the peptide for T cell binding (TCBL) can also be directly linked together in any order (*i.e.,* N-terminal of one to C-terminal of other or vice versa) or the peptide may be covalently bonded by a spacer or linker molecule. With regard to linkers between the two domains, suitable examples include: a thioether bond between an amino terminus bromoacetylated peptide and a carboxyl terminus cysteine, often preceded by a diglycine sequence (Zimmerman D *et al.,* 2005 *supra*); carbodiimide linkages; a multiple glycine, *e.g.,* from 3 to 6 glycines, such as triglycine, with or without one or two serines; and other conventional linkages such as the direct linkages EDS, SPDP and MBS, as disclosed in U.S. 5,652,342.

It should be understood that any of the examples may contain alternatively the above mentioned spacer/linker sequence listings SEQ ID NO.'s 40, 41, 42, 43, 44, and 45. However, for purposes of exemplification, the spacer GGG is shown.

It will be understood that any of the examples may alternatively contain the above-mentioned TCBL sequence listings SEQ ID NO.'s 31, 32, 33, 34, 35, 36, 37, 38, 39, and 49. However, for purposes of exemplification, only the peptide J TCBL peptide (SEQ ID NO. 32) is provided herein.

It is further understood that any of the examples may alternatively contain the above-mentioned L.E.A.P.S. sequence listings to activate the DC and the L.E.A.P.S.-activated DC labeled with dye or radionuclide or drug for detection or delivery of therapeutic agent (drug or radionuclide). For purposes of exemplification, the dye CFSE can be shown.

### Example 1

The following peptide construct is an example of a variant of SEQ ID NO. 32 (peptide J) as the TCBL and SEQ ID NO. 13 (PG70) as the antigenic epitope yielding-J-PG70.
DLLKNGERIEKVEGGGATEGRVRVNSAYQDK (SEQ ID NO. 14)

### Example 2

The improved variants of J-PG70, which can be generated using the substitutions indicated in Table 2 can be made and within the skill of ordinary skill. The following peptide construct used in preliminary testing employs a single X16 variation indicated in Table 2 of the first arginine in the P₁ segment of the J-PG70 peptide (SEQ ID NO. 14) to yield the J-PG70Cit peptide (SEQ ID NO. 51)
DLLKNGERIEKVEGGGATEG(Cit)VRVNSAYQDK (SEQ ID NO. 51)

### Example 3

The following peptide construct is an example of a variant of SEQ ID NO. 6 for derG as the TCBL and SEQ ID NO. 13 as the antigenic epitope.
DGQEEKAGVVSTGLIGGGATEGRVRVNSAYQDK (SEQ ID NO. 46)

### Example 4

The effect of L.E.A.P.S. conjugates on DCs following maturation is provided in this example. Bone marrow cells (BM) from naive BALB/c mice co-cultured *ex vivo* for 24 hours in the presence of L.E.A.P.S. conjugates led to matured dendritic cells (DCs) when applying J-PG70 but not derG-PG70, as indicated by the cell marker profiles (MHC class II and CD86 surface markers expression in flow cytometry) as shown in FIG. 1. Likewise L.E.A.P.S. conjugates in the form of J-PG70 but not derG-PG70 modulated the IL-12 intracellular expression in matured DCs following 24 hours of incubation (see FIG. 2).

### Example 5

The effect of L.E.A.P.S. conjugates on T cell responses is shown in this example. J-PG70 conjugates modulated the proliferation of T cells isolated from spleen or lymph-node cells from wild type BALB/c mice with PGIA or naive, PG-TCR-transgenic BALB/c mice. *Ex vivo* T cell proliferation of PGIA BALB/c splenocytes significantly increased following the 24 hour incubation with PG70, J-PG70, PG70Cit and J-PG70Cit, but not with DerG-PG70 or DerG-PG70Cit (FIG. 3 left-hand side). Similarly, T cell polarization significantly skewed towards a Th1 response, as indicated by an increased Th1:Th2 cytokine ratio (IFNγ:IL-4), when treated with PG70, J-PG70, PG70Cit and J-PG70Cit (FIG. 3 right-hand side), and significantly skewed towards a Th2 response, indicated by a decreased Th1:Th2 ratio, when treated with DerG-PG70.

### Example 6

In this example, *in vivo* experiments of efficacy and protection are shown in FIG. 4, which indicates groups from a first study of efficacy of vaccine candidates, and related controls.

### Example 7

In this example, the effects of L.E.A.P.S.-PG peptide conjugate vaccination on the proportions of splenic Th1, Th2, Th17, and regulatory T cells (Tregs) are shown in FIG. 8. The proportions of Th1, Th2, Th17, and regulatory T cells (Tregs) in spleen cell cultures from the vaccinated mice were determined by flow cytometry. Protection was associated with decreased proportions of Th1 and Th17 cells, and increases in Th2 cells and Tregs (p<0.05-0.001, Fisher's LSD test; n=4 mice/group). The DerG-PG70 vaccine is therapeutically effective in PGIA (a Th1 dominated disease) by inducing immune deviation in favor of Th2 and Treg cells. Protected animals showed a statistically significant increase in FoxP3⁺ and IL-10⁺ cells, with a decrease in IFNγ⁺ and IL-17⁺ cells.

## Claims

1. A peptide for use in modulating an autoimmune response in a subject or for maturing dendritic cells, and or activating T cells, comprising a peptide construct selected from the group consisting of SEQ ID No.'s 2, 4, 6, 8, 10, 12, 14, 16, 18, 20, 22, 24, 26, 28, 30 and 46.

2. The peptide for use of claim 1 for use in treating or preventing inappropriate autoimmune response in an individual at risk for autoimmune disease, wherein said treatment or prevention comprises administering to the individual a pharmacologically effective amount of the peptide to effectively eliminate the set or subset of T cells involved in the autoimmune response.

3. The peptide for use of claim 1 for use in modulating an inappropriate autoimmune response in an individual at risk for autoimmune disease, wherein said modulation comprises administering to the individual a pharmacologically effective amount of the peptide to redirect the autoimmune response from a Th1 to a Th2 immune response, or from a Th2 to a Th1 response, whereby the inappropriate autoimmune response is modulated to decrease or eliminate the adverse effects associated with the inappropriate autoimmune response.

4. A composition comprising
i) a population of matured dendritic cells, the population of matured dendritic cells formed by
(a) contacting immature dendritic cells or monocytes with an effective amount of a peptide construct selected from the group consisting of SEQ ID No.'s 2, 4, 6, 8, 10, 12, 14, 16, 18, 20, 22, 24, 26, 28, 30 and 46 under conditions suitable for maturation of the dendritic cells or monocytes; or
(b) contacting immature dendritic cells or monocytes with an effective amount of a peptide mixture of two or more peptide constructs selected from the group consisting of SEQ ID No.'s 2, 4, 6, 8, 10, 12, 14, 16, 18, 20, 22, 24, 26, 28, 30 and 46 under conditions suitable for maturation of the dendritic cells or monocytes; or
ii) a population of activated T cells, the population of activated T cells formed by:
(c) contacting T cells with an effective amount of a peptide construct selected from the group consisting of SEQ ID No.'s 2, 4, 6, 8, 10, 12, 14, 16, 18, 20, 22, 24, 26, 28, 30 and 46 under conditions suitable for activation of T cells; or
(d) contacting T cells with an effective amount of a peptide mixture of two or more peptide constructs selected from the group consisting of SEQ ID No.'s 2, 4, 6, 8, 10, 12, 14, 16, 18, 20, 22, 24, 26, 28, 30 and 46 under conditions suitable for activation of T cells.

5. The composition of claim 4, wherein said matured dendritic cells or activated T cells are conjugated to a radioisotope, a dye or a therapeutic agent.

6. An ex vivo method for producing
i) a matured dendritic cell population, comprising contacting or treating immature dendritic cells or monocytes with an effective amount of a peptide construct selected from the group consisting of SEQ ID No.'s 2, 4, 6, 8, 10, 12, 14, 16, 18, 20, 22, 24, 26, 28, 30 and 46 under conditions suitable for maturation of dendritic cells or monocytes to form matured dendritic cells; or
ii) an activated T cell population, comprising contacting or treating T cells or monocytes with an effective amount of a peptide construct selected from the group consisting of SEQ ID No.'s 2, 4, 6, 8, 10, 12, 14, 16, 18, 20, 22, 24, 26, 28, 30 and 46 under conditions suitable for activation of T cells to form activated T cells.

7. A matured dendritic cell population or an activated T cell population produced by the method of claim 6 for use in modulating an autoimmune condition in a subject, wherein said modulation comprises administering an effective amount of the matured dendritic cell population or the activated T cell population to the subject.

8. The matured dendritic cell population or an activated T cell population produced by the method of claim 6 for use in delivering a therapeutic or a radiation source to the site of an autoimmune condition in a subject, wherein the matured dendritic cells or the activated T cells are conjugated to the therapeutic agent or the radiation source, and wherein said delivery comprises administering the matured dendritic cells or the activated T cells to the subject.

9. The matured dendritic cell population or an activated T cell population produced by the method of claim 6 for use in detecting the presence or location of an autoimmune condition in a subject, wherein the matured dendritic cells or activated T cells are labeled with a tracking marker allowing for detection of the matured dendritic cells or activated T cells, and wherein said detection comprsises administering the matured dendritic cells or activated T cells to the subject and observing the location of the tracking marker in the body of the subject, wherein the tracking marker is concentrated in a location, tissue type or organ structure of the subject's body after an elapse of a period of time from administration of the matured dendritic cells or activated T cells to the subject.

## Patentansprüche

1. Peptid zur Verwendung beim Modulieren einer Autoimmunreaktion eines Patienten oder beim Reifen dendritischer Zellen und/oder beim Aktivieren von T-Zellen, umfassend ein Peptidkonstrukt, das aus der Gruppe bestehend aus SEQ ID Nr. 2, 4, 6, 8, 10, 12, 14, 16, 18, 20, 22, 24, 26, 28, 30 und 46 ausgewählt wurde.

2. Peptid zur Verwendung nach Anspruch 1 zur Verwendung beim Behandeln oder Vorbeugen einer unangemessenen Autoimmunreaktion bei einer Person, bei der das Risiko einer Autoimmunerkrankung besteht, wobei die Behandlung oder Vorbeugung das Verabreichen einer Menge des Peptids an die Person umfasst, die pharmakologisch wirksam ist, um die Gruppe oder Untergruppe von an der Autoimmunreaktion beteiligten T-Zellen wirkungsvoll zu eliminieren.

3. Peptid zur Verwendung nach Anspruch 1 zur Verwendung beim Modulieren einer unangemessenen Autoimmunreaktion bei einer Person, bei der das Risiko einer Autoimmunerkrankung besteht, wobei die Modulation das Verabreichen einer Menge des Peptids an die Person umfasst, die pharmakologisch wirksam ist, um die Autoimmunreaktion von einer Th1- nach einer Th2-Immunreaktion oder von einer Th2- zu einer Th1-Reaktion umzulenken, wobei die unangemessene Autoimmunreaktion so moduliert wird, dass die mit der unangemessenen Autoimmunreaktion verbundenen nachteiligen Wirkungen verringert oder beseitigt werden.

4. Zusammensetzung, umfassend
i) eine Population reifer dendritischer Zellen, wobei die Population reifer dendritischer Zellen folgendermaßen gebildet wird:
(a) Inkontaktbringen unreifer dendritischer Zellen oder von Monozyten mit einer wirksamen Menge eines Peptidkonstrukts, das aus der Gruppe bestehend aus SEQ ID Nr. 2, 4, 6, 8 10, 12, 14, 16, 18, 20, 22, 24, 26, 28, 30 und 46 ausgewählt wurde, unter Bedingungen, die für das Reifen der dendritischen Zellen oder von Monozyten geeignet sind; oder
(b) Inkontaktbringen unreifer dendritischer Zellen oder von Monozyten mit einer wirksamen Menge eines Peptidgemisches aus zwei oder mehr Peptidkonstrukten, die aus der Gruppe bestehend aus SEQ ID Nr. 2, 4, 6, 8 10, 12, 14, 16, 18, 20, 22, 24, 26, 28, 30 und 46 ausgewählt wurden, unter Bedingungen, die für das Reifen der dendritischen Zellen oder von Monozyten geeignet sind; oder
ii) eine Population aktivierter T-Zellen, wobei die Population aktivierter T-Zellen folgendermaßen gebildet wird:
(c) Inkontaktbringen von T-Zellen mit einer wirksamen Menge eines Peptidkonstrukts, das aus der Gruppe bestehend aus SEQ ID Nr. 2, 4, 6, 8, 10, 12, 14, 16, 18, 20, 22, 24, 26, 28, 30 und 46 ausgewählt wurde, unter Bedingungen, die für die Aktivierung von T-Zellen geeignet sind; oder
(d) Inkontaktbringen von T-Zellen mit einer wirksamen Menge eines Peptidgemischs aus zwei oder mehr Peptidkonstrukten, die aus der Gruppe bestehend aus SEQ ID Nr. 2, 4, 6, 8, 10, 12, 14, 16, 18, 20, 22, 24, 26, 28, 30 und 46 ausgewählt wurden, unter Bedingungen, die für die Aktivierung von T-Zellen geeignet sind.

5. Zusammensetzung nach Anspruch 4, wobei die reifen dendritischen Zellen oder aktivierten T-Zellen an ein Radioisotop, einen Farbstoff oder einen therapeutischen Agens konjugiert sind.

6. Ex-Vivo-Verfahren zum Herstellen
i) einer Population reifer dendritischer Zellen, umfassend das Inkontaktbringen oder Behandeln unreifer dendritischer Zellen oder von Monozyten mit einer wirksamen Menge eines Peptidkonstrukts, das aus der Gruppe bestehend aus SEQ ID Nr. 2, 4, 6, 8, 10, 12, 14, 16, 18, 20, 22, 24, 26, 28, 30 und 46 ausgewählt wurde, unter Bedingungen, die für das Reifen dendritischer Zellen oder von Monozyten geeignet sind, so dass reife dendritische Zellen gebildet werden; oder
ii) eine Population aktivierter T-Zellen, umfassend das Inkontaktbringen oder Behandeln von T-Zellen oder Monozyten mit einer wirksamen Menge eines Peptidkonstrukts, das aus der Gruppe bestehend aus SEQ ID Nr. 2, 4, 6, 8, 10, 12, 14, 16, 18, 20, 22, 24, 26, 28, 30 und 46 ausgewählt wurde, unter Bedingungen, die für die Aktivierung von T-Zellen geeignet sind, so dass aktivierte T-Zellen gebildet werden.

7. Population reifer dendritischer Zellen oder Population aktivierter T-Zellen, die gemäß dem Verfahren nach Anspruch 6 hergestellt wurde, zur Verwendung beim Modulieren einer Autoimmunerkrankung bei einem Patienten, wobei die Modulation das Verabreichen einer wirksamen Menge der Population reifer dendritischer Zellen oder der Population aktivierter T-Zellen an den Patienten umfasst.

8. Population reifer dendritischer Zellen oder Population aktivierter T-Zellen, die gemäß dem Verfahren nach Anspruch 6 hergestellt wurde, zur Verwendung bei der Bereitstellung eines therapeutischen Agens oder einer Strahlungsquelle an der Stelle einer Autoimmunerkrankung bei einem Patienten, wobei die reifen dendritischen Zellen oder die aktivierten T-Zellen an das therapeutische Agens oder die Strahlungsquelle konjugiert sind und wobei die Bereitstellung das Verabreichen der reifen dendritischen Zellen oder der aktivierten T-Zellen an den Patienten umfasst.

9. Population reifer dendritischer Zellen oder Population aktivierter T-Zellen, die gemäß dem Verfahren nach Anspruch 6 hergestellt wurde, zur Verwendung bei der Feststellung des Vorliegens oder des Orts einer Autoimmunerkrankung bei einem Patienten, wobei die reifen dendritischen Zellen oder aktivierten T-Zellen mit einem Marker zur Verfolgung versehen sind, der den Nachweis der reifen, dendritischen Zellen oder aktivierten T-Zellen ermöglicht, und wobei der Nachweis das Verabreichen der reifen dendritischen Zellen oder aktivierten T-Zellen an den Patienten und das Beobachten des Aufenthaltsorts des Markers zur Verfolgung im Körper des Patienten umfasst, wobei der Marker zur Verfolgung nach Ablauf eines Zeitraums seit der Verabreichung der reifen dendritischen Zellen oder aktivierten T-Zellen an den Patienten an einem Ort, in einem Gewebetyp oder einer Organstruktur des Körpers des Patienten konzentriert ist.

## Revendications

1. Peptide destiné à être utilisé dans la modulation d'une réponse auto-immune chez un sujet ou pour la maturation de cellules dendritiques et/ou l'activation de lymphocytes T, comprenant une construction peptidique choisie dans le groupe constitué de SEQ ID No. 2, 4, 6, 8, 10, 12, 14, 16, 18, 20, 22, 24, 26, 28, 30 et 46.

2. Peptide destiné à être utilisé selon la revendication 1, pour une utilisation dans le traitement ou la prévention d'une réponse auto-immune inappropriée chez un individu à risque de maladie auto-immune, dans lequel ledit traitement ou prévention comprend l'administration à l'individu d'une quantité pharmacologiquement efficace du peptide pour éliminer efficacement l'ensemble ou sous-ensemble de lymphocytes T impliqués dans la réponse auto-immune.

3. Peptide destiné à être utilisé selon la revendication 1 pour une utilisation dans la modulation d'une réponse auto-immune inappropriée chez un individu à risque de maladie auto-immune, dans lequel ladite modulation comprend l'administration à l'individu d'une quantité pharmacologiquement efficace du peptide pour rediriger la réponse auto-immune d'un Th1 à une réponse immune Th2, ou d'une réponse Th2 à une réponse Th1, moyennant quoi la réponse auto-immune inappropriée est modulée pour diminuer ou éliminer des effets indésirables associés à la réponse auto-immune inappropriée.

4. Composition comprenant
i) une population de cellules dendritiques matures, la population de cellules dendritiques matures étant formée par
(a) la mise en contact de cellules dendritiques immatures ou de monocytes avec une quantité efficace d'une construction peptidique choisie dans le groupe constitué de SEQ ID No. 2, 4, 6, 8, 10, 12, 14, 16, 18, 20, 22, 24, 26, 28, 30 et 46 dans des conditions appropriées pour la maturation des cellules dendritiques ou des monocytes ; ou
(b) la mise en contact de cellules dendritiques immatures ou de monocytes avec une quantité efficace d'un mélange peptidique de deux ou plusieurs constructions peptidiques choisies dans le groupe constitué de SEQ ID No. 2, 4, 6, 8, 10, 12, 14, 16, 18, 20, 22, 24, 26, 28, 30 et 46 dans des conditions appropriées pour la maturation des cellules dendritiques ou des monocytes ; ou
ii) une population de lymphocytes T activés, la population de lymphocytes T activés étant formée par :
(c) la mise en contact de lymphocytes T avec une quantité efficace d'une construction peptidique choisie dans le groupe constitué de SEQ ID No. 2, 4, 6, 8, 10, 12, 14, 16, 18, 20, 22, 24, 26, 28, 30 et 46 dans des conditions appropriées pour l'activation de lymphocytes T ; ou
(d) la mise en contact de lymphocytes T avec une quantité efficace d'un mélange peptidique de deux ou plusieurs constructions peptidiques choisies dans le groupe constitué de SEQ ID No. 2, 4, 6, 8, 10, 12, 14, 16, 18, 20, 22, 24, 26, 28, 30 et 46 dans des conditions appropriées pour l'activation de lymphocytes T.

5. Composition selon la revendication 4, dans laquelle lesdites cellules dendritiques matures ou lymphocytes T activés sont conjugués à un radio-isotope, un colorant ou un agent thérapeutique.

6. Procédé ex vivo destiné à produire
i) une population de cellules dendritiques matures, comprenant la mise en contact ou le traitement de cellules dendritiques immatures ou de monocytes avec une quantité efficace d'une construction peptidique choisie dans le groupe constitué de SEQ ID No. 2, 4, 6, 8, 10, 12, 14, 16, 18, 20, 22, 24, 26, 28, 30 et 46 dans des conditions appropriées pour la maturation de cellules dendritiques ou de monocytes pour former des cellules dendritiques matures ; ou
ii) une population de lymphocytes T activés, comprenant la mise en contact ou le traitement de lymphocytes T ou de monocytes avec une quantité efficace d'une construction peptidique choisie dans le groupe constitué de SEQ ID No. 2, 4, 6, 8, 10, 12, 14, 16, 18, 20, 22, 24, 26, 28, 30 et 46 dans des conditions appropriées pour l'activation de lymphocytes T pour former des lymphocytes T activés.

7. Population de cellules dendritiques matures ou population de lymphocytes T activés produite par le procédé de la revendication 6, destinée à être utilisée dans la modulation d'un problème de santé auto-immune chez un sujet, ladite modulation comprenant l'administration d'une quantité efficace de la population de cellules dendritiques matures ou de la population de lymphocytes T activés au sujet.

8. Population de cellules dendritiques matures ou population de lymphocytes T activés produite par le procédé de la revendication 6, destinée à être utilisée dans la délivrance d'une source thérapeutique ou d'une source de rayonnement au site d'un problème de santé auto-immune chez un sujet, les cellules dendritiques matures ou les lymphocytes T activés étant conjugués à l'agent thérapeutique ou à la source de rayonnement, et ladite délivrance comprenant l'administration des cellules dendritiques matures ou des lymphocytes T activés au sujet.

9. Population de cellules dendritiques matures ou population de lymphocytes T activés produite par le procédé de la revendication 6, destinée à être utilisée dans la détection de la présence ou de l'emplacement d'un problème de santé auto-immune chez un sujet, les cellules dendritiques matures ou les lymphocytes T activés étant marqués par un marqueur de poursuite permettant la détection des cellules dendritiques matures ou des lymphocytes T activés, et ladite détection comprenant l'administration des cellules dendritiques matures ou des lymphocytes T activés au sujet et l'observation de l'emplacement du marqueur de poursuite dans le corps du sujet, le marqueur de poursuite étant concentré dans un emplacement, un type de tissu ou une structure d'organe du corps du sujet après un laps de temps écoulé depuis l'administration des cellules dendritiques matures ou des lymphocytes T activés au sujet.
